(19) Europäisches Patentamt
European Patent Office
Office européen des brevets

(11) Veröffentlichungsnummer: **0 400 290 A1**

(12) **EUROPÄISCHE PATENTANMELDUNG**

(21) Anmeldenummer: **90105494.0**

(22) Anmeldetag: **19.11.84**

(51) Int. Cl.5: **C07K 5/02, C07K 5/06, C07C 229/22, C07C 237/06**

Diese Anmeldung is am 23 - 03 - 1990 als Teilanmeldung zu der unter INID-Kode 60 erwähnten Anmeldung eingereicht worden.

Die Bezeichnung der Erfindung wurde geändert (Richtlinien für die Prüfung im EPA, A-III, 7.3).

(30) Priorität: **23.11.83 CH 6285/83**

(43) Veröffentlichungstag der Anmeldung: **05.12.90 Patentblatt 90/49**

(60) Veröffentlichungsnummer der früheren Anmeldung nach Art. 76 EPÜ: **0 143 746**

(84) Benannte Vertragsstaaten: **AT BE CH DE FR GB IT LI LU NL SE**

(71) Anmelder: **CIBA-GEIGY AG**
**Klybeckstrasse 141**
**CH-4002 Basel(CH)**

(72) Erfinder: **Fuhrer, Walter, Dr.**
**Im Budler 3**
**CH-4419 Lupsingen(CH)**
Erfinder: **Bühlmayer, Peter, Dr.**
**Hangstrasse 18**
**CH-4144 Arlesheim(CH)**
Erfinder: **Rasetti, Vittorio, Dr.**
**Passwangstrasse 6**
**CH-4059 Basel(CH)**
Erfinder: **Riniker, Bernhard, Dr.**
**Adlergasse 14**
**CH-4402 Frenkendorf(CH)**

(54) **5-Amino-4-Hydroxyvalerylderivate, als Zwischenprodukte in der Herstellung von Renin-Hemmern benutzbar.**

(57) Verbindungen der Formel

$$H_2N-\underset{R_3}{CH}-\overset{R_4}{CH}-CH_2-\overset{R_5}{CH}-\overset{O}{\overset{\|}{C}}-R_6 \qquad (I),$$

worin $R_3$ Wasserstoff, Alkyl, Cycloalkyl, Arylniederalkyl oder Aryl, $R_4$ Hydroxy oder veräthertes oder verestertes Hydroxy, $R_5$ Alkyl, Cycloalkyl, Arylniederalkyl oder Aryl und $R_6$ substituiertes Amino oder substituiertes Hydroxy darstellen, und Salze von solchen Verbindungen sind Zwischenprodukte zur Herstellung von Reninhibitoren.

EP 0 400 290 A1

## 5-Amino-4-hydroxyvalerylderivate

Die Erfindung betrifft substituierte 5-Amino-4-hydroxyvalerylderivate der Formel

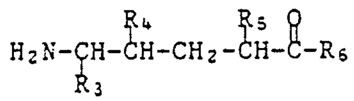

(I)

worin $R_3$ Wasserstoff, Alkyl, Cycloalkyl, Arylniederalkyl oder Aryl, $R_4$ Hydroxy oder veräthertes oder verestertes Hydroxy, $R_5$ Alkyl, Cycloalkyl, Arylniederalkyl oder Aryl und $R_6$ substituiertes oder freies Amino oder substituiertes Hydroxy darstellen, Salze dieser Verbindungen und Verfahren zu ihrer Herstellung.

In der Beschreibung der vorliegenden Erfindung bedeutet der bei der Definition von Gruppen oder Resten, z.B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die durch $R_3$, RL und $R_5$ substituierten C-Atome können die R-, S- oder R,S-Konfiguration haben. Bevorzugt sind Verbindungen der Formel I, worin diese C-Atome die S-Konfiguration aufweisen.

Die in der Beschreibung der vorliegenden Erfindung verwendeten allgemeinen Ausdrücke und Bezeichnungen haben vorzugsweise die folgenden Bedeutungen:

Ein unsubstituierter oder substituierter, gesättigter oder ungesättigter, aliphatischer, cycloaliphatischer oder cycloaliphatisch-aliphatischer Kohlenwasserstoffrest ist beispielsweise Alkyl, z.B. Niederalkyl, Niederalkenyl, Niederalkinyl, Mono-, Bi- oder Tricycloalkyl, Monocycloalkenyl, Bicycloalkenyl, Cycloalkylniederalkyl, Cycloalkylniederalkenyl oder Cycloalkenylniederalkyl.

Niederalkyl hat vorzugsweise 1-7 C-Atome und ist z.B. Methyl, Aethyl, n-Propyl, Isopropyl, n-Butyl oder tert-Butyl, welche durch eine oder mehrere funktionelle Gruppen, beispielsweise Hydroxy, veräthertes Hydroxy, z.B. Niederalkoxy, wie Methoxy oder Aethoxy, Phenyloxy oder durch einen gewünschtenfalls acetylierten oder als Acetal geschützten natürlichen Zucker veräthertes Hydroxy, z.B. Glucofuranosyl, verestertes Hydroxy, z.B. Niederalkanoyloxy, wie Acetoxy, oder Halogen, z.B. Chlor oder Brom, Hydroxysulfonyloxy, Carboxy, verestertes Carboxy, z.B. Niederalkoxycarbonyl, wie Methoxy- oder Aethoxycarbonyl, amidiertes Carboxy, z.B. Carbamoyl oder Mono- oder Diniederalkylcarbamoyl, wie Methyl- oder Dimethylcarbamoyl, Amino, Niederalkylamino, z.B. Methylamino, Hydroxyniederalkylamino, z.B. 2-Hydroxyäthylamino, Diniederalkylamino, z.B. Dimethylamino, Acylamino oder N-Acyl-N-niederalkylamino, Guanidino, Mercapto, Niederalkylthio, z.B. Methylthio, oder durch Oxo substituiert sein können.

Alkyl hat vorzugsweise 1-10 C-Atome und ist z.B. Niederalkyl mit den oben genannten Bedeutungen, z.B. Methyl, Aethyl, n-Pentyl, Neopentyl, n-Hexyl oder n-Heptyl, oder n-Octyl, n-Nonyl oder N-Decyl, und kann durch eine oder mehrere der obengenannten funktionellen Gruppen substituiert sein.

Niederalkenyl enthält z.B. 2-7, insbesondere 2-4, C-Atome und ist z.B. Vinyl, Allyl oder 2- oder 3-Butenyl. Niederalkenyl kann durch die gleichen Substituenten substituiert sein wie Niederalkyl.

Niederalkinyl enthält z.B. 2-7, insbesondere 2-4, C-Atome und ist beispielsweise Aethinyl, 1-Propinyl oder 2-Propinyl.

Cycloalkyl enthält z.B. 3-8, insbesondere 3-6, C-Atome und ist z.B. Cyclopropyl, Cyclobutyl, Cyclopentyl oder Cyclohexyl.

Bicycloalkyl enthält z.B. 5-10, insbesondere 6-9, C-Atome und ist z.B. Bicyclohexyl, -heptyl, -octyl, -nonyl oder -decyl, z.B. Bicyclo[3.1.0]hex-1-, -2- oder -3-yl, Bicyclo[4.1.0]hept-1- oder -4-yl, Bicyclo[2.2.1]-hept-2-yl, z.B. endo- oder exo-Norbornyl, Bicyclo[3.2.1]oct-2-yl, Bicyclo[3.3.0]oct-3-yl oder Bicyclo[3.3.1]-non-9-yl.

Tricycloalkyl enthält z.B. 8-10 C-Atome und ist z.B. Tricyclo[5.2.1.0$^{2,6}$]dec-8-yl oder Adamantyl.

Monocycloalkenyl enthält z.B. 3-8, insbesondere 3-6, C-Atome und ist beispielsweise Cyclohexenyl, z.B. 1-Cyclohexenyl, oder Cyclohexadienyl, z.B. 1,4-Cyclohexadienyl.

Bicycloalkenyl enthält z.B. 5-10, insbesondere 7-10, C-Atome und ist z.B. Bicyclo[2.2.1]hept-5-en-yl, z.B. 5-Norbornen-2-yl, Bicyclo[2.2.2]octen-2-yl oder Hexahydro-4,7-methanoind-1-en-6-yl.

Cycloalkylniederalkyl enthält z.B. 4-10, insbesondere 4-7, C-Atome und ist beispielsweise Cyclopropylmethyl, Cyclobutylmethyl, Cyclopentylmethyl oder Cyclohexylmethyl.

Cycloalkylniederalkenyl enthält z.B. 5-10, insbesondere 5-9, C-Atome und ist beispielsweise Cyclohexylvinyl oder Cyclohexylallyl.

Cycloalkenylniederalkyl enthält z.B. 4-10, insbesondere 4-7, C-Atome und ist beispielsweise 1-Cyclohexenylmethyl oder 1,4-Cyclohexadienylmethyl.

Die genannten cycloaliphatischen oder cycloaliphatisch-aliphatischen Reste können durch die gleichen Substituenten wie Niederalkyl substituiert sein.

Ein aromatischer bzw. aromatisch-aliphatischer Kohlenwasserstoffrest ist beispielsweise unsubstituiertes oder substituiertes Phenyl bzw. Phenylniederalkyl, z.B. Halophenyl, wie 4-Chlorophenyl, Niederalkoxyphenyl, wie 4-Methoxyphenyl oder Nitrophenyl, Benzyl, Niederalkylbenzyl wie 4-Methylbenzyl, Niederalkoxybenzyl, wie 4-Methoxybenzyl, substituiertes Anilino benzyl, wie 2-(o,o-Dichloroanilino)-benzyl oder 2-(o,o-Dichloro-N-benzylanilino)-benzyl, 2-Phenyläthyl, 2-(p-Hydroxyphenyl)-äthyl, Diphenylmethyl, Di-(4-methoxyphenyl)methyl oder Trityl, oder Phenylniederalkenyl, z.B. 3-Phenylallyl.

In einem heteroaromatischen bzw. heteroaromatisch-aliphatischen Kohlenwasserstoffrest ist der Heterocyclus fünf- oder sechsgliedrig und enthält ein bis zwei Stickstoffatome und/oder ein Sauerstoff- oder Schwefelatom. Dieser Heterocyclus kann an einem Stickstoffatom durch Niederalkyl, z.B. Methyl oder Aethyl, Phenyl, oder Phenylniederalkyl, z.B. Benzyl, substituiert und/oder benzannelliert sein und ist beispielsweise 2- oder 3-Pyrrolyl, 2-Furanyl, 2-Thiophenyl, 4-Imidazolyl, 2-, 3- oder 4-Pyridyl, 3-Indolyl, 2-, 3-oder 4-Chinolinyl, 1-, 3- oder 4-Isochinolinyl, 2-Benzoxazolyl oder 2-Benzthiazolyl.

Die aromatisch-aliphatischen oder heteroaromatisch-aliphatischen Reste können im aliphatischen Teil durch die gleichen Substituenten wie Niederalkyl substituiert sein.

Substituiertes Phenylniederalkyl ist beispielsweise Phenyl-hydroxyniederalkyl, z.B. α-Hydroxybenzyl, substituiertes oder unsubstituiertes Phenyl-amino-niederalkyl, z.B. 2-Phenyl-1-aminoäthyl oder 2-(p-Hydroxyphenyl)-1-amino-äthyl oder substituiertes oder unsubstituiertes Phenyl-acylamino-niederalkyl, z.B. 2-Phenyl-1-benzyloxycarbonylamino-äthyl oder 2-(p-Acetoxyphenyl)-1-benzyloxycarbonylamino-äthyl.

Ein fünf- oder sechsgliedriger Heterocyclus hat mindestens ein C-Atom, 1-3 Stickstoffatome und gegebenenfalls ein Sauerstoff- oder Schwefelatom als Ringglieder. Dieser Heterocyclus kann an einem seiner C-Atome oder an einem Ringstickstoffatom durch Niederalkyl, z.B. Methyl oder Aethyl, Phenyl oder Phenylniederalkyl, z.B. Benzyl, an einem seiner C-Atome durch Hydroxy oder Oxo oder an einem Ringstickstoffatom durch Acyl substituiert sein.

Ein solcher Heterocyclus ist beispielsweise Pyrrolidin-3-yl, Hydroxypyrrolidin-2- oder -3-yl, z.B. 4-Hydroxypyrrolidin-2-yl, Hydroxy-1-acylpyrrolidin-2- oder -3-yl, z.B. 1-Benzyloxycarbonyl-4-hydroxypyrrolidin- 2-yl, Oxopyrrolidin-2-yl, z.B. 5-Oxopyrrolidin-2-yl, Piperidin-2- oder -3-yl-, 1-Niederalkylpiperidin-2-, -3- oder -4-yl, z.B. 1-Methylpiperidin-2-, -3- oder -4-yl, 1-Acylpiperidin-2-, -3- oder -4-yl, z.B. 1-Benzyloxycarbonylpiperidin-2-, -3- oder -4-yl, Morpholin-2- oder -3-yl, Thiomorpholin-2- oder -3-yl oder 1-und/oder 4-Niederalkylpiperazin-2-oder -3-yl, z.B. 1,4-Dimethylpiperazin-2-oder -3-yl.

Alkyl $R_3$ oder $R_5$ ist vorzugsweise Niederalkyl, z.B. Isopropyl oder Isobutyl, oder n-Octyl.

Cycloalkyl $R_3$ oder $R_5$ hat die weiter vorn für Cycloalkyl genannten Bedeutungen und ist vorzugsweise Cyclohexyl.

Arylniederalkyl $R_3$ oder $R_5$ hat die weiter vorn für Arylniederalkyl genannten Bedeutungen und ist vorzugsweise Benzyl.

Aryl $R_3$ oder $R_5$ ist vorzugsweise Phenyl.

Eine verätherte Hydroxygruppe $R_4$ ist vorzugsweise durch solche organischen Reste veräthert, die unter physiologischen Bedingungen abspaltbar sind und nach der Abspaltung in der betreffenden Konzentration pharmakologisch unbedenkliche Spaltprodukte liefern.

Veräthertes Hydroxy $R_4$ ist z.B. Acyloxyniederalkoxy, worin Acyl die Acylgruppe einer gegebenenfalls verzweigten Niederalkancarbonsäure oder der durch gegebenenfalls verzweigtes Niederalkyl monoveresterten Kohlensäure darstellt, z.B. Niederalkanoyloxyniederalkoxy, wie Acetoxymethoxy, 1-Acetoxyäthoxy, Pivaloyloxymethoxy oder 1-Pivaloyloxyäthoxy, oder Niederalkoxycarbonyloxyniederalkoxy, wie Aethoxycarbonyloxymethoxy, 1-Aethoxycarbonyloxyäthoxy, tert-Butoxycarbonyloxymethoxy oder 1-tert-Butoxycarbonyloxyäthoxy.

Veräthertes Hydroxy $R_4$ ist ferner Niederalkoxy, z.B. Methoxy oder Aethoxy, Aryloxy, z.B. Phenoxy, oder Arylniederalkoxy, z.B. Benzyloxy.

Verestertes Hydroxy $R_4$ ist z.B. aliphatisches Acyloxy, z.B. Niederalkanoyloxy, wie Acetoxy oder Pivaloyloxy, cycloaliphatisches Acyloxy, z.B. Cycloalkanoyloxy, wie Cyclohexanoyloxy, oder aromatisches Acyloxy, z.B. Benzoyloxy.

$R_6$ mit der Bedeutung "substituiertes Amino" oder "substituiertes Hydroxy" ist beispielsweise eine Amino- oder Hydroxygruppe, welche durch einen oder gegebenenfalls zwei unsubstituierte oder substituierte, gesättigte oder ungesättigte, aliphatische Kohlenwasserstoffreste bis einschliesslich 18, bevorzugt bis einschliesslich 10, C-Atomen oder einen unsubstituierten oder substituierten, aromatischen, heteroaromatischen, aromatisch-aliphatischen oder heteroaromatisch-aliphatischen Kohlenwasserstoffrest mit bis zu 18, bevorzugt bis einschliesslich 10, C-Atomen substituiert ist.

$R_6$ mit der Bedeutung "substituiertes Amino" oder "substituiertes Hydroxy" kann ausserdem einen N-terminal mit der Gruppe -CO-verbundenen und C-terminal gegebenenfalls amidierten oder veresterten Rest einer natürlichen α-Aminosäure oder einen N-terminal mit der Gruppe -CO-verbundenen und C-terminal gegebenenfalls amidierten oder veresterten Di- oder Tripeptidrest, der aus natürlichen α-Aminosäuren und gewünschtenfalls aus Statin (abgekürzt H-Sta-OH, 4-Amino-3-hydroxy-6-methylheptansäure) besteht, darstellen.

Ein unsubstituierter oder substituierter, gesättigter oder ungesättigter, aliphatischer Kohlenwasserstoffrest, welcher die Aminogruppe oder Hydroxygruppe $R_6$ substituiert, ist beispielsweise Alkyl mit bis zu 10 C-Atomen, sowie Niederalkenyl oder Niederalkinyl bis einschliesslich 7 C-Atomen.

Diese Reste können durch eine oder mehrere der weiter vorn genannten funktionellen Gruppen, sowie durch Sulfo, Cyano oder substituiertes Amino, worin die Aminogruppe Teil eines fünf- oder sechsgliedrigen Heterocyclus enthaltend ein bis zwei Stickstoffatome und gewünschtenfalls ein Sauerstoff- oder Schwefelatom ist, substituiert sein.

Als Substituenten sind Hydroxy, substituiertes oder unsubstituiertes Phenyloxy, z.B. Carbamoylphenyloxy oder Carbamoyl-hydroxy-phenyloxy, Carboxy, verestertes Carboxy, z.B. Niederalkoxycarbonyl, wie tert-Butoxycarbonyl, oder ein physiologisch spaltbares verestertes Carboxy, z.B. 1-(Niederalkanoyloxy)-niederalkoxycarbonyl, wie Acetoxymethoxycarbonyl, Pivaloyloxymethoxycarbonyl oder 1-Propionyloxyäthoxycarbonyl, 1-(Niederalkoxycarbonyloxy)-niederalkoxycarbonyl, wie 1-(Aethoxycarbonyloxy)-äthoxycarbonyl, oder α-Aminoniederalkanoyloxymethoxycarbonyl, wie α-Aminoacetoxymethoxycarbonyl oder (S)-α-Amino-β-methylbutyryloxymethoxycarbonyl, Carbamoyl, substituiertes oder unsubstituiertes Niederalkylcarbamoyl, z.B. Hydroxyniederalkylcarbamoyl wie 2-Hydroxyäthylcarbamoyl, Triniederalkylsilyloxyniederalkylcarbamoyl, oder Methoxycarbonyl-hydroxyniederalkylcarbamoyl, Amino, substituiertes oder unsubstituiertes Niederalkylamino, z.B. Hydroxyniederalkylamino, Diniederalkylamino, über ein Stickstoff-Atom gebundenes fünf- oder sechsgliedriges, gewünschtenfalls durch Oxo substituiertes Heterocyclyl, z.B. 1-Piperidyl, 4-Morpholinyl oder 2-Oxo-1-pyrrolidinyl, und Acylamino, z.B. Niederalkoxycarbonylamino, wie tert-Butoxycarbonylamino, bevorzugt.

Alkyl mit 1-10 C-Atomen ist z.B. Niederalkyl mit den oben genannten Bedeutungen, z.B. Methyl oder Aethyl, sowie n-Pentyl, Neopentyl, n-Hexyl, n-Heptyl, n-Octyl, n-Nonyl oder n-Decyl.

Substituiertes Alkyl ist z.B. Hydroxyniederalkyl, z.B. 2-Hydroxyäthyl, Niederalkoxyniederalkyl, z.B. Niederalkoxymethyl oder Niederalkoxyäthyl, z.B. Methoxymethyl oder 2-Methoxyäthyl, Niederalkanoyloxyniederalkyl, z.B. Niederalkanoyloxymethyl oder Niederalkanoyloxyäthyl, z.B. Acetoxymethyl oder 2-Acetoxyäthyl, Halogenniederalkyl, z.B. Halogenmethyl oder Halogenäthyl, z.B. Trifluormethyl, Chlor- oder Brommethyl, 2-Chlor-, 2-Brom- oder 2,2,2-Trichloräthyl, Hydroxysulfonyloxyniederalkyl, z.B. Hydroxysulfonyloxymethyl oder 2-Hydroxysulfonyloxyäthyl, Phenyloxyniederalkyl, z.B. 2-Phenyloxyäthyl, substituiertes Phenyloxyniederalkyl, z.B. 2-(3-Carbamyol-4-hydroxyphenoxy)-äthyl, substituiertes Phenyloxy-hydroxyniederalkyl, z.B. 3-(3-Carbamoylphenoxy)-2-hydroxypropyl, Carboxyalkyl, z.B. 4-Carboxy-n-butyl, 5-Carboxy-n-pentyl, 6-Carboxy-n-hexyl, 7-Carboxy-n-heptyl oder 8-Carboxy-n-octyl, ferner Carboxymethyl, 2-Carboxyäthyl, 3-Carboxy-n-propyl oder 1- oder 2-Carboxyprop-2-yl, verestertes Carboxy- alkyl, z.B. Niederalkoxycarbonylalkyl, z.B. 4-tert-Butoxycarbonyl-n-butyl, 7-tert-Butoxycarbonyl-n-heptyl oder 8-tert-Butoxycarbonyl-n-octyl, ferner tert-Butoxycarbonylmethyl oder 2-tert-Butoxycarbonyläthyl, oder physiologisch spaltbares verestertes Carboxyalkyl, z.B 7-Pivaloyloxymethoxycarbonyl-n-heptyl, 7-(1-Aethoxycarbonyloxyäthoxycarbonyl)-n-heptyl oder 4-Pivaloyloxymethoxycarbonyl-n-butyl, Carbamoylniederalkyl, z.B. Carbamoylmethyl, 2-Carbamoyläthyl oder Dicarbamoylmethyl, Niederalkylcarbamoylniederalkyl, z.B. Methylcarbamoylmethyl, Hydroxyniederalkylcarbamoylniederalkyl, z.B. 7-(2-Hydroxyäthyl)-carbamoyl-n-heptyl, 4-(2-Hydroxyäthyl)-carbamoyl-n-butyl, 7-(Tris-[hydroxymethyl]-methyl)-carbamoyl-n-heptyl oder 4-(Tris-[hydroxymethyl]-methyl)-carbamoyl-n-butyl, Triniederalkylsilyloxyniederalkyl-carbamoylniederalkyl, z.B. 4-(Tris-[tert-butyldimethylsilyloxymethyl]-methyl)-carbamoyl-n-butyl, Niederalkoxycarbonyl-hydroxy-niederalkylcarbamoylniederalkyl, z.B. Carbamoyl-( 2-hydroxy-1-isobutyl-3-methoxycarbonylpropyl)-carbamoylmethyl, Diniederalkylcarbamoylniederalkyl, z.B. Dimethylcarbamoylmethyl, Sulfoniederalkyl, z.B. Sulfomethyl oder 2-Sulfoäthyl, Aminoalkyl, z.B. 4-Amino-n-butyl, 5-Amino-n-pentyl, 6-Amino-n-hexyl, 7-Amino-n-heptyl oder 8-Amino-n-octyl, ferner 2-Aminoäthyl oder 3-Amino-n-propyl, Niederalkylaminoniederalkyl, z.B. 2-Methylaminoäthyl oder 3-Methylamino-n-propyl, Hydroxyniederalkylaminoniederalkyl, z.B. 2-(2-Hydroxyäthylamino)-äthyl, Diniederalkylaminoniederalkyl, z.B. 2-Dimethylaminoäthyl oder 3-Dimethylamino-n-propyl, über ein Stickstoff-Atom gebundenes fünf- oder sechsgliedriges Heterocyclylniederalkyl, z.B. 3-(1-Piperidinyl)-propyl, 2-(1-Piperidinyl)-äthyl, 3-(4-Morpholinyl)-propyl, 2-(4-Morpholinyl)-äthyl, 3-(2-Oxo-1-pyrrolidinyl)-propyl oder 2-(2-Oxo-1-pyrrolidinyl)-äthyl, oder Acylaminoalkyl, z.B. Niederalkanoylaminoalkyl, z.B. Pivaloylaminoalkyl oder Acetylaminoalkyl, wie 4-Pivaloylamino-n-butyl oder 4-Acetylamino-n-butyl, oder Niederalkoxycarbonylaminoalkyl, z.B. tert-Butoxycarbonylaminoalkyl, wie 4-tert-Butoxycarbonylamino-n-butyl, 5-tert-Butoxycarbonylamino-n-pentyl, 6-tert-Butoxycarbonylamino-n-hexyl oder 7-tert-Butoxycarbonylamino-n-heptyl, ferner 2-tert-Butoxycarbonylaminoäthyl.

Niederalkenyl und Niederalkinyl sind wie die oben genannten Reste definiert und können durch die gleichen Substituenten wie Niederalkyl substituiert sein.

Ein aromatischer oder aromatisch-aliphatischer Kohlenwasserstoffrest hat die oben genannten Bedeutungen und ist vorzugsweise Phenyl oder Phenylniederalkyl.

Diese Reste können im Aromaten z.B. durch Niederalkyl, wie Methyl oder Aethyl, Hydroxy, veräthertes Hydroxy, z.B. Niederalkoxy, wie Methoxy oder tert-Butoxy, verestertes Hydroxy, z.B. Niederalkanoyloxy, wie Acetoxy, oder Halogen, z.B. Fluor oder Chlor, Carboxy, verestertes Carboxy, z.B. Niederalkoxycarbonyl, wie tert-Butoxycarbonyl, Carbamoyl, Amino, Niederalkylamino, z.B. Methylamino, Diniederalkylamino, z.B. Dimethylamino, acyliertes Amino, z.B. Niederaloxycarbonylamino, wie tert-Butoxycarbonylamino, sowie durch Nitro substituiert sein.

Niederalkyl in einem Rest Phenylniederalkyl kann durch die gleichen Substituenten wie Alkyl in einem Rest $R_6$ substituiert sein.

Substituiertes Phenyl ist beispielsweise 2-, 3- oder 4-Niederalkylphenyl, z.B. 2-, 3- oder 4-Methylphenyl, 2-, 3- oder 4-Hydroxyphenyl, 2-, 3- oder 4-Niederalkoxyphenyl, z.B 2-, 3- oder 4-Methoxyphenyl, 2,3-, 2,4- oder 2,5-Dimethoxyphenyl oder 2-, 3- oder 4-tert-Butoxyphenyl, 2-, 3-oder 4-Halogenphenyl, z.B. 2-, 3- oder 4-Chlorphenyl, 2,3-, 3,4- oder 2,5-Dihalogenphenyl, z.B. 2,3-, 3,4-oder 2,5-Dichlorphenyl, 2-, 3- oder 4-Carboxyphenyl, 2-, 3- oder 4-Niederalkoxycarbonylphenyl, z.B. 2-, 3-oder 4-tert-Butoxycarbonylphenyl, 2-, 3- oder 4-Carbamoylphenyl, 2-, 3-oder 4-Aminophenyl, 2-, 3- oder 4-Niederalkoxycarbonylaminophenyl, z.B. 2-, 3- oder 4-tert-Butoxycarbonylaminophenyl, oder 2-, 3- oder 4-Nitrophenyl.

Substituiertes oder unsubstituiertes Phenylniederalkyl ist beispielsweise Benzyl, 2-Phenyläthyl, 1-Phenylprop-2-yl, 3-Phenylpropyl, oder 1-Hydroxymethyl-2-phenyl-äthyl, 21-Hydroxy-1- oder -2-phenyl-äthyl oder 1-(2-Cyano- oder 2-Carboxy-1-hydroxyäthyl)-2-phenyläthyl.

Ein heteroaromatischer oder heteroaromatisch-aliphatischer Kohlenwasserstoffrest hat die oben genannten Bedeutungen und ist vorzugsweise Oxazolyl, Thiazolyl, Pyridylniederalkyl, Imidazolylniederalkyl oder Indolylniederalkyl.

Diese Reste können im Heteroaromaten z.B. durch Niederalkyl, z.B. Methyl oder Aethyl, Carboxy, verestertes Carboxy, z.B. Niederalkoxycarbonyl, wie tert-Butoxycarbonyl, Carbamoyl, Carboxyniederalkyl, z.B. Carboxymethyl, verestertes Carboxyniederalkyl, z.B. Niederalkoxycarbonyl, wie Methoxycarbonylmethyl, oder Carbamoylniederalkyl, z.B. Carbamoylmethyl substituiert sein.

Substituiertes oder unsubstituiertes Heteroaryl ist beispielsweise 2-Oxazolyl, 2-Thiazolyl oder 4- oder 5-Carbamoylmethyl-2-thiazolyl.

Substituiertes oder unsubstituietes Heteroarylniederalkyl ist beispielsweise 2-, 3- oder 4-Pyridylmethyl, 2-(2-, 3- oder 4-Pyridyl)-äthyl, 3-(2-, 3- oder 4-Pyridyl)-propyl, 4-Imidazolylmethyl, 2-(4-Imidazolyl)-äthyl, 3-Indolylmethyl oder 2-(3-Indolyl)-äthyl.

Die Amino- oder Hydroxygruppe $R_6$ kann ausserdem durch einen fünf- oder sechsgliedrigen Heterocyclus substituiert sein, der die oben genannten Bedeutungen hat. Ein solcher Heterocyclus ist beispielsweise 1-Benzylpiperidin-2-, -3- oder -4-yl, oder 1-Niederalkoxycarbonylpiperidinyl, z.B. 1-Aethoxy- oder 1-n-Butoxycarbonylpiperidin-2-, -3- oder -4-yl.

Substituiertes Amino $R_6$ ist vorzugsweise Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino oder Tris(hydroxymethyl)-methylamino, Niederalkoxyniederalkylamino, z.B. 2-Methoxyäthylamino, substituiertes Phenoxyniederalkylamino, z.B. 2-(3-Carbamoyl-4-hydroxyphenoxy)-äthylamino, substituiertes Phenoxyhydroxy-niederalkylamino, z.B. 3-(3-Carbamoylphenoxy)-2-hydroxypropylamino, Carboxyalkylamino, z.B. 4-Carboxy-n-butylamino, 5-Carboxy-n-pentylamino, 6-Carboxy-n-hexylamino, 7-Carboxy-n-heptylamino, 8-Carboxy-n-octylamino oder 1-Carboxy-prop-2-ylamino, Niederalkoxycarbonylalkyl amino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 4-Pivaloyloxymethoxycarbonyl-n-butylamino, 7-(1-Aethoxy-carbonyloxyäthoxycarbonyl)-n-heptylamino oder 7-Pivaloyloxymethoxycarbonyl-n-heptylamino, Carbamoylniederalkylamino, z.B. Carbamoylmethylamino oder Dicarbamoyl-methylamino, Hydroxyniederalkylcarbamoylniederalkylamino. z.B. 4-(Tris[hydroxymethyl]-methyl)-carbamoyl-n-butylamino, Niederalkoxycarbonyl-hydroxy-niederalkylcarbamoyl-niederalkylamino. z.B. $\alpha$-Carbamoyl-$\alpha$-(2-hydroxy-1-isobutyl-3-methoxycarbonylpropyl)-carbamoyl-methylamino, Aminoalkylamino, z.B. 4-Amino-n-butylamino, 5-Amino-n-pentylamino, 6-Amino-n-hexylamino, 7-Amino-n-heptylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf-oder sechsgliedriges Heterocyclylniederalkylamino, z.B. 3-(4-Morpholinyl)-propylamino oder 3-(2-Oxo-1-pyrrolidinyl)propylamino, Acylaminoniederalkylamino, z.B. Niederalkanoylaminoniederalkylamino, wie 4-Pivaloylamino-n-butylamino, oder Niederalkoxycarbonylaminoniederalkylamino, wie 4-tert-Butoxycarbonylamino-n-butylamino, 5-tert-Butoxycarbonylamino-n-pentylamino, 6-tert-Butoxycarbonylamino-n-hexylamino oder 7-tert-Butoxycarbonylamino-n-octylamino, Benzylamino, Carboxybenzylamino, z.B. 3-Carboxybenzylamino, sowie Niederalkoxycarbonylbenzylamino, z.B. 3-tert-Butoxycarbonylbenzylamino.

Substituiertes Hydroxy $R_6'$ ist vorzugsweise Niederalkoxy, z.B. Methoxy, Aethoxy, n-Propoxy, Isopropoxy, Butoxy, Isobutoxy, tert-Butoxy oder tert-Pentoxy, oder physiologisch spaltbares Alkoxy, z.B. Pivaloyloxymethoxy.

Ein N-terminal mit der -CO-Gruppe verbundener und C-terminal gegebenenfalls amidierter oder veresterter natürlicher Aminosäurerest $R_6$ besteht vorzugsweise aus einer der 20 $\alpha$-Aminosäuren, die besonders häufig in Proteinen vorkommen, z.B. -His-, -Phe- und besonders -Ala- oder -Ile- und ist gegebenenfalls C-terminal durch Amino, substituiertes Amino oder substituiertes Hydroxy substituiert. Solches substituiertes Amino oder substituiertes Hydroxy hat die weiter vorn für $R_6$ genannten Bedeutungen und ist beispielsweise Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Niederalkoxycarbonylalkylamino, z.B. 4-tert-Butoxycarbonyl-n-butylamino oder 7-tert-Butoxycarbonyl-n-heptylamino, physiologisch spaltbares, verestertes Carboxyalkylamino, z.B. 4-Pivaloyloxymethoxycarbonyl-n-butylamino, Carbamoylniederalkylamino, z.B. Carbamoylmethylamino oder Dicarbamoyl-methylamino, Hydroxyniederalkylcarbamoyl-niederalkylamino, z.B. 2-Hydroxyäthylcarbamoyl-methylamino oder Di-(2-hydroxyäthylcarbamoyl)-methylamino, Aminoalkylamino, z.B. 4-Amino-n-butylamino, 5-Amino-n-pentylamino oder 8-Amino-n-octylamino, Hydroxyniederalkylamino-niederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf- oder sechsgliedriges Heterocyclylniederalkylamino, z.B. 3-(4-Morpholinyl)-propylamino-2-(4-Morpholinyl)-äthylamino oder 3-(2-Oxo-1-pyrrolidinyl)-propylamino, Acylaminoniederalkylamino, z.B. Niederalkoxycarbonylaminoniederalkylamino, wie 4-tert-Butoxycarbonylamino-n-butylamino, Phenylniederalkylamino, z.B. Benzylamino, Carboxybenzylamino, z.B. 3-Carboxybenzylamino, Niederalkoxycarbonylbenzylamino, z.B. 3-tert-Butoxycarbonylbenzylamino, Carbamoylbenzylamino, z.B. 3-Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1- oder 2-phenyläthylamino, 1-(2-Cyano- oder 2-Carboxy-1-hydroxyäthyl)-2-phenyl-äthylamino, Thiazolylamino, z.B. 2-Thiazolylamino oder 4-oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino, 3-(2-, 3- oder 4-Pyridyl)-propylamino, Imidazolylniederalkylamino, z.B. 4-Imidazolylmethylamino, 2-(4-Imidazolyl)-äthylamino, Indolylniederalkylamino, z.B. 3-Indolylmethylamino, 2-(3-Indolyl)-äthylamino, Piperidinylamino, z.B. 1-Benzylpiperidin-2-, -3- oder -4-ylamino, 1-Niederalkoxycarbonylpiperidinylamino, z.B. 1-Aethoxy- oder 1-n-Butoxycarbonylpiperidin-2-, -3- oder -4-ylamino, ausserdem Niederalkoxy, z.B. Methoxy, Aethoxy oder tert-Butoxy, oder physiologisch spaltbares substituiertes Alkoxy, z.B. Pivaloyloxymethoxy.

In einem N-terminal mit der -CO-Gruppe verbundenen und C-terminal gegebenenfalls amidierten oder veresterten Di- oder Tripeptidrest sind die Aminosäurebestandteile vorzugsweise von den 20 $\alpha$-Aminosäuren, die besonders häufig in Proteinen vorkommen, sowie vom Statin abgeleitet. Ein solcher Di- oder Tripeptidrest ist beispielsweise -Ile-His-, -Ile-Phe-, -Ala-His-, -Gly-Gly-, -Gly-His-, -Gly-Ala- -Gly-Ser-, -Ala-Sta-, -Ile-Sta-, -Gly-Gly-Gly-, -Gly-His-Lys-, -Gly-Ala-Gly- oder Ile-His-Lys.

Die Aminosäurereste können N-terminal durch Niederalkyl, z.B. Methyl oder Aethyl, substituiert sein. Die Carboxy-Funktion in der Seitenkette von -Glu- und -Asp- ist gewünschtenfalls mit einem Niederalkanol, z.B. mit Methanol oder tert-Butanol verestert. Die Amino-Funktion in der Seitenkette von -Lys- ist gewünschtenfalls acyliert, z.B. durch Pivaloyl, tert-Butoxycarbonyl oder Benzyloxycarbonyl.

Ein Di- oder Tripeptidrest ist C-terminal gegebenenfalls durch Amino, substituiertes Amino oder substituiertes Hydroxy substituiert. Solches substituiertes Amino oder substituiertes Hydroxy hat die weiter vorn für $R_6$ genannten Bedeutungen und ist beispielsweise Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy.

Salze werden beispielsweise von Verbindungen der Formel I mit einer sauren Gruppe, z.B. einer Carboxygruppe, gebildet und sind in erster Linie geeignete Alkalimetall-, wie Natrium- oder Kalium-, oder Erdalkalimetallsalze, z.B. Magnesium- oder Calciumsalze, ferner Zinksalze, oder Ammoniumsalze, inkl. solche Salze, welche mit organischen Aminen, wie gegebenenfalls durch Hydroxy substituierten Mono-, Di- oder Trialkylaminen gebildet werden, z.B. Diäthylamin, Di-(2-hydroxyäthyl)-amin, Triäthylamin, N,N-Dimethyl-N-(2-hydroxyäthyl)-amin, Tri-(2-hydroxyäthyl)-amin oder N-Methyl-D-glucamin. Die Verbindungen

der Formel I können Säureadditionssalze bilden, z.B. mit anorganischen Säuren, z.B. Salzsäure, Schwefelsäure der Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, wie z.B. den weiter vorn genannten α-Aminosäuren, sowie Methansulfonsäure, Aethansulfonsäure, 2-Hydroxyäthansulfonsäure, Aethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Die Verbindungen der vorliegenden Erfindung können als Zwischenprodukte zur Herstellung von Renininhibitoren verwendet werden, die als Antihypertensiva, ferner zur Behandlung von Herzinsuffizienz geeignet sind.

Die Erfindung betrifft insbesondere Verbindungen der Formel I, worin $R_3$ Wasserstoff, Niederalkyl, Cycloalkyl, Arylniederalkyl oder Aryl, $R_4$ Hydroxy, $R_5$ Alkyl, Cycloalkyl, Arylniederalkyl oder Aryl, und $R_6$ Amino oder eine Amino- oder Hydroxygruppe, welche durch einen oder gegebenenfalls zwei unsubstituierte oder substituierte, gesättigte oder ungesättigte, aliphatische Kohlenwasserstoffreste bis einschliesslich 18, bevorzugt bis einschliesslich 10, C-Atomen oder einen aromatischen, heteroaromatischen, aromatischaliphatischen oder heteroaromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer natürlichen α-Aminosäure oder einen N-terminal mit der Gruppe -CO-verbundenen und C-terminal gegebenenfalls amidierten oder veresterten Di- oder Tripeptidrest, der aus natürlichen α-Aminosäuren und gewünschtenfalls aus Statin besteht, darstellen, ferner Salze dieser Verbindungen.

Die Erfindung betrifft hauptsächlich Verbindungen der Formel I, worin $R_3$ Wasserstoff, Niederalkyl, z.B. Isopropyl oder Isobutyl, Cycloalkyl, Phenylniederalkyl oder Phenyl, $R_4$ Hydroxy, $R_5$ Alkyl mit 1-10 C-Atomen, z.B. Niederalkyl, wie Isopropyl oder Isobutyl, oder n-Octyl, Cycloalkyl, z.B. Cyclohexyl, Phenylniederalkyl, z.B. Benzyl, oder Phenyl, und $R_6$ Amino, Alkylamino mit 1 bis 10 C-Atomen, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino oder Diäthylamino, Hydroxyniederalkylamino, z.B. 2-Hydroxyäthylamino, 3-Hydroxypropylamino oder Tris-(hydroxymethyl)-methylamino, Di-(hydroxyniederalkyl)-amino, z.B. Di-(2-hydroxyäthyl)-amino, Niederalkoxyniederalkylamino, z.B. Methoxymethylamino oder 2-Methoxyäthylamino, Niederalkanoyloxyniederalkylamino, z.B. Acetoxymethylamino oder 2-Acetoxyäthylamino, Halogenniederalkylamino, z.B. Halogenmethyl- oder Halogenäthylamino, wie Trifluoromethyl-, Chlormethyl-, Brommethyl-, 2-Chloräthyl, 2-Bromäthyl- oder 2,2,2-Trichloräthylamino, Hydroxysulfonyloxyniederalkylamino, z.B. Hydroxysulfonyloxymethylamino oder 2-Hydroxysulfonyloxyäthylamino, Phenyloxyniederalkylamino oder Phenyloxy-hydroxyniederalkylamino, worin Phenyl gegebenenfalls durch Niederalkyl, Niederalkoxy, Hydroxy, Carboxy, Niederalkoxycarbonyl oder Carbamoyl substituiert ist, z.B. 2-Phenyloxyäthyl-, 2-(3-Carbamoyl-4-hydroxyphenoxy)-äthyl- oder 3-(3-Carbamoylphenoxy)-2-hydroxypropylamino, Carboxyalkylamino, z.B. 4-Carboxy-n-butyl-, 5-Carboxy-n-pentyl-, 6-Carboxy-n-hexyl, 7-Carboxy-n-heptyl-, 8-Carboxy-n-octyl-, 2-Carboxyäthyl-, 2-Carboxy-n-propyl oder 1- oder 2-Carboxyprop-2-yl-amino, Niederalkoxycarbonylalkylamino, z.B. 4-tert-Butoxycarbonyl-n-butyl-, 7-tert-Butoxycarbonyl-n-heptyl-, 8-tert-Butoxycarbonyl-n-octyl-, 2-tert-Butoxycarbonyläthyl- oder 1-tert-Butoxycarbonylprop-2-yl-amino, physiologisch spaltbares verestertes Carboxyalkylamino, z.B. 4-Pivaloyloxymethoxycarbonyl-n-butylamino, 7-(1-Aethoxycarbonyloxyäthoxycarbonyl)-n-heptylamino oder 7-Pivaloyloxymethoxycarbonyl-n-heptylamino, Carbamoylniederalkylamino, z.B. Carbamoylmethylamino, 2-Carbamoyläthylamino oder Dicarbamoyl-methylamino, Niederalkylcarbamoylniederalkylamino, z.B. Methylcarbamoylmethylamino, Hydroxyniederalkylcarbamoylniederalkylamino, z.B. 7-(2-Hydroxyäthyl)-carbamoyl-n-heptyl-, 4-(2-Hydroxyäthyl)-carbamoyl-n-butyl-, 7-(Tris-[hydroxymethyl]-methyl)-carbamoyl-n-heptyl- oder 4-(Tris-[hydroxymethyl]-methyl)-carbamoyl-n-butylamino, Triniederalkylsilyloxyniederalkylcarbamoylniederalkylamino, z.B. 4-(Tris-[tert-butyldimethylsilyloxymethyl]-methyl)-carbamoyl-n-butylamino, Niederalkoxycarbonyl-hydroxy-niederalkylcarbamoylniederalkylamino, z.B. α-Carbamoyl-α-(2-hydroxy-1-isobutyl-3-methoxycarbonylpropyl)-carbamoyl-methylamino, Diniederalkylcarbamoylniederalkylamino, z.B. Dimethylcarbamoylmethyl amino oder Bis-dimethylcarbamoyl-methylamino, Sulfoniederalkylamino, z.B. Sulfomethylamino oder 2-Sulfoäthylamino, Aminoalkylamino, z.B. 4-Amino-n-butyl-, 5-Amino-n-pentyl-, 6-Amino-n-hexyl-, 7-Amino-n-heptyl-, 8-Amino-n-octyl-, 2-Amino-äthyl- oder 1-Amino-prop-2-yl-amino, Niederalkylaminoniederalkylamino, z.B. 2-Methylaminoäthylamino oder 3-Methylamino-n-propylamino, Hydroxyniederalkylaminoniederalkylamino, z.B. 2-(2-Hydroxyäthylamino)-äthylamino oder 3-(2-Hydroxyäthylamino)-n-propylamino, Diniederalkylaminoniederalkylamino, z.B. 2-Dimethylaminoäthylamino oder 3-Dimethylamino-n-propylamino, 1-Piperidinylniederalkylamino, z.B. 2-(1-Piperidinyl)-äthlyamino oder 3-(1-Piperidinyl)-propylamino, 4-Morpholinylniederalkylamino, z.B. 2-(4-Morpholinyl)-äthylamino oder 3-[4-Morpholinyl)-propylamino, 1-Pyrrolidinylniederalkylamino, worin Pyrrolidin gegebenenfalls durch Hydroxy und/oder Oxo substituiert ist, z.B. 2-(2-Oxopyrrolidin-1-yl)-äthylamino oder 3-(2-Oxopyrrolidin-1-yl)-propylamino, Acylaminoalkylamino, z.B. Niederalkanoylaminoalkylamino, z.B. Pivaloylaminoalkylamino oder Acetylaminoalkylamino, wie 4-Pivaloylamino-n-butylamino, 2-Pivaloylaminoäthylamino oder 4-Acetylamino-n-butylamino, Niederalkoxycarbonylaminoalkylamino, wie 4-tert-Butoxycarbonylamino-n-butyl-, 5-tert-Butoxycarbonylamino-n-pentyl-, 6-tert-Butoxycarbonylamino-n-hexyl-, 7-tert-Butoxycarbonylamino-n-heptyl oder 2-tert-Butoxycarbonylaminoäthyl-amino, Niederalkenylamino, z.B. Allylamino oder 2- oder 3-Butenylamino, Niederalkinylamino, z.B. Propargylamino, Phenylamino oder Phenylniederalkylamino, worin Phenyl gegebenenfalls durch Niederalkyl, z.B. Methyl, Hydroxy, Niederalkoxy, z.B. Methoxy oder tert-Butoxy, Niederalkanoyloxy, z.B. Acetoxy, Halogen, z.B. Fluor oder Chlor, Carboxy, Niederalkoxycarbonyl, z.B. tert-Butoxycarbonyl, Carbamoyl, Amino, Niederalkylamino, z.B. Methylamino, Diniederalkylamino, z.B. Dimethylamino, Acylamino, z.B. tert-Butoxycarbonylamino und/oder durch Nitro mono- oder mehrfach substituiert ist, z.B. Phenyl-, 2-, 3- oder 4-Methylphenyl-, 4-Hydroxyphenyl-, 4-Methoxyphenyl-, 2,3-, 2,4- oder 2,5-Dimethoxyphenyl-, 4-Chlorphenyl, 2-, 3- oder 4-Carboxyphenyl-, 2-, 3- oder 4-Methoxy- oder tert-Butoxycarbonylphenyl-, 2-, 3-oder 4-Carbamoylphenyl-, 4-Aminophenyl-, 4-tert-Butoxycarbonylaminophenyl- oder 4-Nitrophenyl-amino, ferner z.B. Benzylamino, 4-Methylbenzylamino, 4-Methoxybenzylamino, 2-, 3- oder 4-Carboxybenzylamino, 2-, 3- oder 4-tert-Butoxycarbonylbenzylamino, 2-, 3- oder 4-Carbamoylbenzylamino, 2-Phenyläthylamino, 2-Phenylprop-2-ylamino oder 3-Phenylpropyl amino, Phenylniederalkylamino, worin Niederalkyl durch Hydroxy, Cyano, Carboxy, Niederalkoxy und/oder Carbamoyl substituiert ist, z.B. 1-Hydroxymethyl-2-phenyläthylamino, 2-Hydroxy-1- oder -2-phenyläthylamino, oder 1-(2-Cyano- oder -2-Carboxy-1-hydroxyäthyl)-2-phenyläthylamino, Oxazolylamino, z.B. 2-Oxazolylamino, Thiazolylamino, das gegebenenfalls durch Carboxyniederalkyl, z.B. Carboxymethyl, Niederalkoxycarbonylniederalkyl, z.B. Methoxycarbonylmethyl, oder Carbamoylniederalkyl, z.B. Carbamoylmethyl, substituiert ist, z.B. 4- oder 5-Carboxymethyl-2-thiazolylamino oder 4- oder 5-Carbamoylmethyl-2-thiazolylamino, Pyridylniederalkylamino, z.B. 2-, 3- oder 4-Pyridylmethyl-, 2-(2-, 3-oder 4-Pyridyl)-äthyl- oder 3-(2-, 3- oder 4-Pyridyl)-propylamino, Imidazolylniederalkylamino, z.B. 4-Imidazolylmethylamino oder 2-(4-Imidazolyl)-äthylamino, Indolylniederalkylamino, z.B. 3-Indolylmethylamino oder 2-(3-Indolyl)-äthylamino, 1-Benzylpiperidinylamino, z.B. 1-Benzylpiperidin-4-ylamino, 1-Niederalkoxycarbonyl piperidinylamino, z.B. 1-Aethoxy- oder 1-n-Butoxycarbonylpiperidin-2-, -3- oder -4-ylamino, ferner Niederalkoxy, z.B. Methoxy, Aethoxy, n-Propoxy, Isopropoxy, Butoxy, Isobutoxy, tert-Butoxy oder tert-Pentoxy, Phenyloxy, Phenylniederalkoxy, z.B. Benzyloxy oder Diphenylmethoxy, oder physiologisch spaltbares Alkoxy, z.B. Pivaloyloxymethoxy,

ferner -His-, -Phe-, -Ala-, -Ile-, C-terminal substituiert durch Amino, Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Niederalkoxycarbonylalkylamino, z.B. 4-tert-Butoxycarbonyl-n-butylamino oder 7-tert-Butoxycarbonyl-n-heptylamino, physiologisch spaltbares, verestertes Carboxyalkylamino, z.B. 4-Pivaloyloxymethoxycarbonyl-n-butylamino, Carbamoylniederalkylamino, z.B. Carbamoylmethylamino oder Dicarbamoyl-methylamino, Hydroxyniederalkylcarbamoyl-niederalkylamino, z.B. 2-Hydroxyäthylcarbamoyl-methylamino oder Di-(2-hydroxyäthylcarbamoyl)-methylamino, Aminoalkylamino, z.B. 4-Amino-n-butylamino, 5-Aminon-pentylamino oder 8-Amino-n-octylamino, Hydroxyniederalkylamino-niederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf oder sechsgliedriges Heterocyclylnieder alkylamino, z.B. 3-(4-Morpholinyl)-propylamino, 2-(4-Morpholinyl)-äthylamino oder 3-(2-Oxo-1-pyrrolidinyl)-propylamino, Acylaminoniederalkylamino, z.B. Niederalkoxycarbonylaminoniederalkylamino, wie 4-tert-Butoxycarbonylamino-n-butylamino, Phenylniederalkylamino, worin Phenyl durch Carboxy Niederalkoxycarbonyl oder Carbamoyl und Niederalkyl durch Hydroxy, Cyano oder Carboxy substituiert sein kann, z.B. Benzylamino, Carboxybenzylamino, z.B. 3-Carboxybenzylamino, Niederalkoxycarbonylbenzylamino, z.B. 3-tert-Butoxycarbonylbenzylamino, Carbamoylbenzylamino, z.B. 3-Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1-oder 2-phenyläthylamino, 1-(2-Cyano- oder 2-Carboxy-1-hydroxyäthyl)-2-phenyläthylamino, unsubstituiertes oder substituiertes Thiazolylamino, z.B. 2-Thiazolylamino oder 4-oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino, 3-(2-, 3- oder 4-Pyridyl)-propylamino, Imidazolylniederalkylamino, z.B. 4-Imidazolylmethylamino oder 2-(4-Imidazolyl)äthylamino, Indolylniederalkylamino, z.B. 3-Indolylmethylamino oder 2-(3-Indolyl)-äthylamino, Piperidinylamino, z.B. 1-Benzylpiperidin-2-, -3- oder -4-ylamino, 1-Niederalkoxycarbonylpiperidinylamino, z.B. 1-Aethoxy- oder 1-n-Butoxycarbonylpiperidin-2-, -3- oder -4-ylamino, ausserdem durch Niederalkoxy, z.B.

Methoxy, Aethoxy oder tert-Butoxy, oder physiologisch spaltbares substituiertes Alkoxy, z.B. Pivaloyloxymethoxy,

ferner -Ile-His-, -Ile-Phe-, -Ala-His-, -Gly-Gly-, -Gly-His-, -Gly-Ala-, -Gly-Ser-, -Ala-Sta-, -Ile-Sta-, -Gly-Gly-Gly-, -Gly-His-Lys-, -Gly-Ala-Gly- oder -Ile-His-Lys-, worin die Aminofunktion in der Seitenkette von -Lys- gegebenenfalls durch Niederalkanoyl, z.B. Pivaloyl, Niederalkoxycarbonyl, z.B. tert-Butoxycarbonyl, oder Phenylniederalkoxycarbonyl, z.B. Benzyloxycarbonyl, acyliert ist, C-terminal substituiert durch Amino, Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy, darstellt, sowie Salze dieser Verbindungen.

Die Erfindung betrifft ganz besonders Verbindungen der Formel 1, worin $R_3$ Niederalkyl, z.B. Isopropyl oder Isobutyl, $R_4$ Hydroxy, $R_5$ Alkyl, z.B. Niederalkyl wie Isopropyl oder Isobutyl, oder n-Octyl, Cyclohexyl oder Phenyl, und

$R_6$ Amino, Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino oder Tris(hydroxymethyl)-methylamino, Niederalkoxyniederalkylamino, z.B. 2-Methoxyäthylamino, substituiertes Phenoxyniederalkylamino, z.B. 2-(3carbamoyl-4-hydroxyphenoxy)-äthylamino, substituiertes Phenoxy-hydroxy-niederalkylamino, z.B. 3-(3-Carbamoylphenoxy)-2-hydroxypropylamino, Carboxyalkylamino, z.B. 4-Carboxy-n-butylamino, 5-Carboxy-n-pentylamino, 6-Carboxy-n-hexylamino, 7-Carboxy-n-heptylamino, 8-Carboxy-n-octylamino oder 1-Carboxy-prop-2-ylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 4-Pivaloyloxymethoxycarbonyl-n-butylamino, 7-(1-Aethoxy-carbonyloxyäthoxycarbonyl)-n-heptylamino oder 7-Pivaloyloxymethoxycarbonyl-n-heptylamino, Carbamoylniederalkylamino, z.B. Carbamoylmethylamino oder Dicarbamoyl-methylamino, Hydroxyniederalkylcarbamoylniederalkylamino, z.B. 4-(Tris[hydroxymethyl]-methyl)-carbamoyl-n-butylamino, Niederalkoxycarbonyl-hydroxy-niederalkylcarbamoyl-niederalkylamino, z.B. $\alpha$-Carbamoyl-$\alpha$-(2-hydroxy-1-isobutyl-3-methoxycarbonylpropyl)-carbamoyl-methylamino, Aminoalkylamino, z.B. 4-Amino-n-butylamino, 5-Amino-n-pentylamino, 6-Amino-n-hexylamino, 7-Amino-n-heptylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, über ein Stickstoffatom gebundenes fünf- oder sechsgliedriges Heterocyclylniederalkylamino, z.B. 3-(4-Morpholinyl)-propylamino oder 3-(2-Oxo-1-pyrrolidinyl)-propylamino, Acylaminoniederalkylamino, z.B. Niederalkanoylaminoniederalkylamino, wie 4-Pivaloylamino-n-butylamino, oder Niederalkoxycarbonylaminoniederalkylamino, wie 4-tert-Butoxycarbonylamino-n-butylamino, 5-tert-Butoxycarbonylamino-n-pentylamino, 6-tert-Butoxycarbonylamino-n-hexylamino oder 7-tert-Butoxycarbonylamino-n-octylamino, Benzylamino, Carboxybenzylamino, z.B. 3-Carboxybenzylamino, Niederalkoxycarbonyl benzylamino, z.B. 3-tert-Butoxycarbonylbenzylamino, sowie Niederalkoxy, z.B. Methoxy, Aethoxy, n-Propoxy, Isopropoxy, Butoxy, Isobutoxy, tert-Butoxy oder tert-Pentoxy, oder physiologisch spaltbares Alkoxy, z.B. Pivaloyloxymethoxy,

ferner -Ala- oder -Ile-, C-terminal substituiert durch Amino, Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Carbamoylniederalkylamino, z.B. Carbamoylmethylamino oder Dicarbamoyl-methylamino, Hydroxyniederalkylcarbamoylniederalkylamino, z.B. 2-Hydroxyäthylcarbamoylmethylamino oder Di-(2-hydroxyäthylcarbamoyl)-methylamino, Aminoalkylamino, z.B. 4-Amino-n-butylamino, 5-Amino-n-pentylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, 4-Morpholinylniederalkylamino, z.B. 3-(4-Morpholinyl)-propylamino oder 2-(4-Morpholinyl)-äthylamino, Phenylniederalkylamino, z.B. Benzylamino, Carboxybenzylamino, z.B. 3-Carboxybenzylamino, Niederalkoxycarbonylbenzylamino, z.B. 3-tert-Butoxycarbonylbenzylamino, Carbamoylbenzylamino, z.B. 3-Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1- oder 2-phenyläthylamino oder 1-(2-Cyano- oder 2-Carboxy-1-hydroxyäthyl)-2-phenyläthylamino, Thiazolylamino, z.B. 4- oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino oder 3-(2-, 3- oder 4-Pyridyl)-propylamino, Imidazolylniederalkylamino, z.B. 4-Imidazolylmethylamino, oder 2-(4-Imidazolyl)-äthylamino, Indolylniederalkylamino, z.B. 3-Indolylmethylamino oder 2-(3-Indolyl)-äthylamino. 1-Niederalkoxycarbonylpiperidinylamino, z.B. 1-Aethoxycarbonylpiperidin-4-ylamino, Niederalkoxy, z.B. Methoxy, Aethoxy oder tert-Butoxy, oder physiologisch spaltbares substituiertes Alkoxy, z.B. Pivaloyloxymethoxy,

ferner -Ile-His-, -Ile-Phe, -Ile-Sta-, -Ala-His-, -Ala-Sta- oder -Ile-His-Lys-, worin die Aminofunktion in der

Seitenkette von -Lys- gegebenenfalls durch Niederalkanoyl, z.B. Pivaloyl, Niederalkoxcarbonyl, z.B. tert-Butoxycarbonyl, oder Phenylniederalkoxycarbonyl, z.B. Benzyloxycarbonyl, acyliert ist, C-terminal substituiert durch Amino, Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy, darstellt, sowie Salze dieser Verbindungen.

Die Erfindung betrifft bevorzugt Verbindungen der Formel I, worin $R_3$ Isopropyl oder Isobutyl, $R_4$ Hydroxy, $R_5$ Isopropyl, n-Octyl, Cyclohexyl oder Phenyl und $R_6$ Amino, Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino oder Tris(hydroxymethyl)-methylamino, Niederalkoxyniederalkylamino, z.B. 2-Methoxyäthylamino, substituiertes Phenoxyniederalkylamino, z.B. 2-(3-Carbamoyl-4-hydroxyphenoxy)-äthylamino, substituiertes Phenoxy-hydroxy-niederalkylamino, z.B. 3-(3-Carbamoylphenoxy)-2-hydroxypropylamino, Carboxyalkylamino, z.B. 4-Carboxy-n-butylamino, 5-Carboxy-n-pentylamino, 6-Carboxy-n-hexylamino, 7-Carboxy-n-heptylamino, 8-Carboxy-n-octylamino oder 1-Carboxy-prop-2-ylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 4-Pivaloyloxymethoxycarbonyl-n-butylamino, 7-(1-Aethoxycarbonyloxyäthoxycarbonyl)-n-heptylamino oder 7-Pivaloyloxymethoxycarbonyl-n-heptylamino, Carbamoylniederalkylamino, z.B. Carbamoylmethylamino oder Dicarbamoyl-methylamino, Hydroxyniederalkylcarbamoylniederalkylamino, z.B. 4-(Tris[hydroxymethyl]-methyl)-carbamoyl-n-butylamino, Niederalkoxycarbonyl-hydroxy-niederalkoxycarbamoyl-niederalkylamino, z.B. α-Carbamoyl-α-( 2-hydroxy-1-isobutyl-3-methoxycarbonylpropyl)-carbamoylmethylamino, Aminoalkylamino, z.B. 4-Amino-n-butylamino, 5-Amino-n-pentylamino, 6-Amino-n-hexylamino, 7-Amino-n-heptylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf- oder sechsgliedriges Heterocyclylniederalkylamino, z.B. 3-(1-Morpholinyl)- propylamino oder 3-(2-Oxo-1-pyrrolidinyl)-propylamino, Acylaminoniederalkylamino, z.B. Niederalkanoylaminoniederalkylamino, wie 4-Pivaloylamino-n-butylamino, oder Niederalkoxycarbonylaminoniederalkylamino, wie 4-tert-Butoxycarbonylamino-n-butylamino, 5-tert-Butoxycarbonylamino-n-pentylamino, 6-tert-Butoxycarbonylamino-n-hexylamino oder 7-tert-Butoxycarbonylamino-n-octylamino, Benzylamino, Carboxybenzylamino, z.B. 3-Carboxybenzylamino, sowie Niederalkoxycarbonylbenzylamino, z.B. 3-tert-Butoxycarbonylbenzylamino, Niederalkoxy, z.B. Methoxy, Aethoxy, n-Propoxy, Isopropoxy, Butoxy, Isobutoxy, tert-Butoxy oder tert-Pentoxy, oder physiologisch spaltbares Alkoxy, z.B. Pivaloyloxymethoxy, ferner -Ala- oder -Ile-, C-terminal substituiert durch Amino, Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Carbamoylniederalkylamino, z.B. Carbamoylmethylamino oder Dicarbamoyl-methylamino, Hydroxyniederalkylcarbamoylniederalkylamino, z.B. 2-Hydroxyäthylcarbamoylmethylamino oder Di-(2-hydroxyäthylcarbamoyl)-methylamino, Aminoalkylamino, z.B. 4-Amino-n-butylamino, 5-Amino-n-pentylamino oder 8-Amino-n-octylamino, Hydroxyniederalkylaminoniederalkylamino, z.B. 2-(2-Hydroxyäthylamino)-äthylamino, 4-Morpholinylniederalkylamino, z.B. 2-(4-Morpholinyl)-äthylamino, Carboxybenzylamino, z.B. 3-Carboxybenzylamino, Carbamoylbenzylamino, z.B. 3-Carbamoylbenzylamino, Thiazolylamino, z.B. 4- oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino oder 3-(2-, 3- oder 4-Pyridyl)-propylamino, Imidazolylniederalkylamino, z.B. 4-Imidazolylmethylamino, oder 2-(4-Imidazolyl)-äthylamino, Indolylniederalkylamino, z.B. 3-Indolylmethylamino oder 2-(3-Indolyl)-äthylamino, Niederalkoxy, z.B. Methoxy, Aethoxy oder tert-Butoxy, oder physiologisch spaltbares substituiertes Alkoxy, z.B. Pivaloyloxymethoxy, ferner -Ile-His-, -Ile-Sta- oder -Ala-Sta-, C-terminal substituiert durch Amino, Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy, darstellt, sowie Salze dieser Verbindungen.

Die Erfindung betrifft in erster Linie Verbindungen der Formel I, worin $R_3$ Isobutyl, $R_4$ Hydroxy, $R_5$ Isopropyl, n-Octyl, Cyclohexyl oder Phenyl, und $R_6$ Amino, Niederalkylamino, z.B. Methyl-, Aethyl- oder n-Butylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino, 7-Carboxy-n-heptylamino oder 8-Carboxy-n-octylamino, Niederalkoxycarbonylalkylamino, z.B. 4-tert-Butoxycarbonyl-n-butylamino oder 7-tert-Butoxycarbonyl-n-heptylamino, physiologisch spaltbares verestertes Carboxyalkylamino, z.B. 4-Pivaloyloxymethoxycarbonyl-n-butylamino oder 7-Pivaloyloxymethoxycarbonyl-n-heptylamino, Aminoalkylamino. z.B. 4-Amino-n-butylamino oder 8-Amino-n-butylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, 4-Morpholinylniederalkylamino, z.B. 3-(4-Morpholinyl)-propylamino, 2-Oxo-1-pyrrolidinylniederalkylamino, z.B. 3-(2-Oxo-1-pyrrolidinyl)-propylamino, ferner -Ala- oder -Ile-, C-terminal substituiert durch Amino, Niederalkylamino, z.B. Methylamino, Aethylamino oder n-Butylamino, Diniederalkylamino, z.B. Dimethylamino, Hydroxyniederalkylamino, z.B. 2-Hydroxyäthylamino, Dicarbamoylmethylamino, Hydroxyniederalkylaminoniederalkylamino, z.B. 2-(2-Hydroxyäthylamino)-äthylamino, 4-Morpholinylniederalkylamino, z.B. 2-(4-Morpholinyl)-äthylamino, 3-Carbamoylbenzylamino, 4-Carbamoylmethyl-2-thiazolylamino, Pyridylniederalkylamino. z.B. 2-Pyridylmethylamino, 2-(2-Pyridyl)-äthylamino oder 3-(2-Pyridyl)-propylamino oder Imidazolylniederalkylamino, z.B. 2-(4-Imidazolyl)-äthylamino oder 2-(3-Indolyl)-äthylamino, ferner -Ile-His- oder -Ile-Sta-, C-terminal substituiert durch Amino, oder -Ala-Sta-, C-terminal substituiert durch Niederalkoxy, z.B. Methoxy, darstellt, sowie Salze dieser Verbindungen.

Die Erfindung betrifft vor allem Verbindungen der Formel I, worin $R_3$ Isobutyl, $R_4$ Hydroxy, $R_5$ Isopropyl, und $R_6$ Amino, Niederalkylamino, z.B. Methylamino, Aethylamino oder n-Butylamino. Dimethylamino, 4-Carboxy-n-butylamino, 7-Carboxy-n-heptylamino, 4-tert-Butoxycarbonyl-n-butylamino, 7-tert-Butoxycarbonyl-n-heptylamino, 7-Pivaloyloxymethoxycarbonyl-n-heptylamino, 3-(2-Oxo-1-pyrrolidinyl)-propylamino, -Ala-, C-terminal substituiert durch 2-(4-Imidazolyl)-äthylamino oder 2-(2-Pyridyl)-äthyl amino, -Ile-, C-terminal substituiert durch Dicarbamoylmethylamino, 3-Carbamoylbenzylamino, 4-Carbamoylmethyl-2-thiazolylamino, 2-(2-Pyridyl)-äthylamino, 2-Pyridylmethylamino oder 2-(3-Indolyl)-äthylamino, -Ile-His- oder -Ile-Sta-, C-terminal substituiert durch Amino, oder -Ala-Sta-, C-terminal substituiert durch Methoxy, darstellt, sowie Salze dieser Verbindungen.

Die Erfindung betrifft zu allererst die in den Beispielen erwähnten Verbindungen und deren Salze.

Die Erfindung betrifft vorallem folgende in den Beispielen beschriebenen Verbindungen: [5(S)-Amino-4(S)-hydroxy-2(S)-isopropyl-7-methyloctanoyl]-Ile-His-$NH_2$, [5(S)-Amino-4(S)-hydroxy-2(S)-isopropyl-7-methyloctanoyl]-Ala-Sta-$OCH_3$, [5(S)-Amino-4(S)-hydroxy-(2S)-isopropyl-7-methyloctanoyl]-Ile-di-carbamoylmethylamid, [5(S)-Amino-4(S)-hydroxy-2(S)-isopropyl-7-methyloctanoyl]-2-(3-carbamoyl-4-hydroxy-phenyloxy)-äthylamid, [5(S)-Amino-4(S)-hydroxy-2(S)-isopropyl-7-methyloctanoyl]-3-(2-pyrrolidinon-1-yl)-propylamid, [5(S)-Amino-4(S)-hydroxy-2(S)-isopropyl-7-methyloctanoyl]-methylamid, [5(S)-Amino-4(S)-hydroxy-2(S)-isopropyl-7-methyloctanoyl]-4-[tris-(hydroxymethyl)-methylaminocarbonyl]-butylamid und [5-(S)-Amino-4(S)-hydroxy-2(S)-isopropyl-7-methyloctanoyl]-carbamoyl-(3-methoxycarbonyl-2-hydroxy-1-isobutyl-propylaminocarbonyl)-methylamid.

Verfahren:

Die erfindungsgemässen Verbindungen der Formel I und Salze von solchen Verbindungen werden nach an sich bekannten Verfahren erhalten, z.B. indem man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, eine Verbindung der Formel

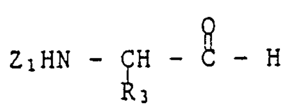

(IX),

worin $R_3$ die genannte Bedeutung hat und $Z_1$ eine Aminoschutzgruppe ist, mit einer Schwefel-Ylid-Verbindung in ein Epoxid der Formel

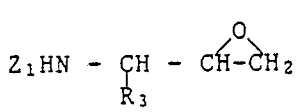

(X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

$$R_a - (C = O) - R_b \qquad (XII),$$

worin $R_3$ und $Z_1$ die genannten Bedeutungen haben und X eine nukleofuge Abgangsgruppe ist, mit einer Carbonylverbindung der Formel
$R_a - (C = O) - R_b$ (XII),
worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

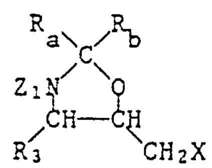

(XIII),

worin die Substituenten die genannten Bedeutungen haben, mit einem Carbonsäureestersalz der Formel

$[R_5 - \overset{\ominus}{C}H - \overset{O}{\overset{||}{C}} - OZ_2]\, Y^{\oplus}$ (XIV),
worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium-oder bevorzugt das Lithiumion, ist, umsetzt, und in einer erhältlichen Verbindung der Formel

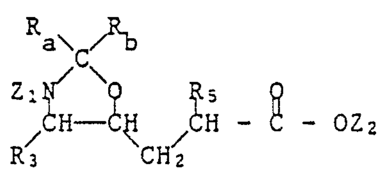

(XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2$ = H) je nach Bedeutung des einzuführenden Rests $R_6$ amidiert, verestert oder mit einem C-terminal gegebenenfalls amidierten oder veresterten Rest einer Aminosäure, eines Di- oder Tripeptidrest substituiert und in einer erhältlichen Verbindung der Formel

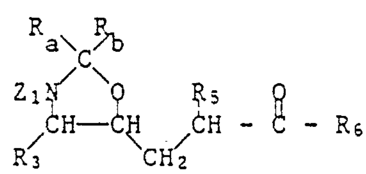

(XVI),

worin die Substituenten die genannten Bedeutungen haben, mit einem geeigneten Solvolysereagens den Ring öffnet und gegebenenfalls die Schutzgruppe $Z_1$ abspaltet, und gewünschtenfalls in einer erhältlichen Verbindung vorhandene Schutzgruppen abspaltet und/oder gewünschtenfalls nach Ausführung eines der vorstehend genannten Verfahren a) oder b) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe

Verbindung der Formel I in eine andere erfindungsgemässe Verbindung der Formel I umwandelt.

Die Erfindung betrifft auch die nach irgendeinem der oben genannten Verfahren erhältlichen anderen Verbindungen als Verbindungen der Formel I (Nebenprodukt), sowie Verbindungen der Formel I und ihre Salze, welche nach einem anderen Verfahren als einem der weiter vorn genannten hergestellt werden.

Verfahren a) (Herstellung einer Amidbindung):

Fragmente einer Verbindung der Formel I mit einer endständigen Carboxygruppe, welche mit einem zur Verbindung der Formel I komplementären Fragment unter Bildung einer Amidbindung kondensiert werden können, sind z.B. Verbindungen der Formeln:

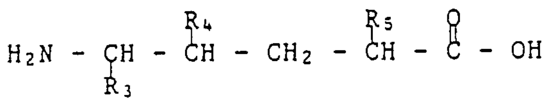 und

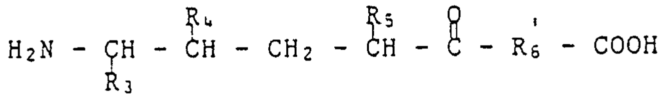,

worin die Gruppe $R_6'$-COOH den Rest einer α-Aminosäure, eines Dipeptids oder eines Tripeptids darstellt, wie er für $R_6$ oben definiert ist, oder die von diesen Verbindungen abgeleiteten aktivierten Ester oder reaktionsfähigen Anhydride, ferner reaktionsfähige cyclische Amide. Die reaktionsfähige Säurederivate können auch in situ gebildet werden.

Aktivierte Ester sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode), oder 1-Niederalkoxyvinylester (erhältlich z.B. durch Behandeln der entsprechenden Säure mit einem Niederalkoxyacetylen; Aethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode), oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenyl, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyamino-bzw. N-Hydroxyamido-Verbindung, z.B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benzotriazin-4-on, z.B. nach der Anhydrid-oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z . B. durch Behandeln der entsprechenden Säure mit Chlorameisensäureniederalkylestern, oder mit einem 1-Niederalkoxycarbonyl-2-niederalkoxy-1,2-dihydrochinolin, z.B. 1-Niederalkoxycarbonyl-2-äthoxy-1,2-dihydrochinolin; Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid;

Phosphoroxychloridmethode), Anhydride mit anderen Phosphor säurederivaten (z.B. solchen, die man mit Phenyl-N-phenylphosphoramidochloridat erhalten kann) oder mit Phosphorsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diäthylaminopropin; Methode der symmetrischen Anhyride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N′-Carbonyldiimidazol; Imidazol-Methode), oder Pyrazol, z.B. 3,5-Dimethylpyrazol (erhältlich z.B. über das Säurehydrazid durch Behandeln mit Acetylaceton; Pyrazolid-Methode).

Zur Verbindung der Formel I komplementäre Fragmente mit einer freien Aminogruppe sind z.B. je nach Bedeutung von $R_5$ Ammoniak, ein primäres oder sekundäres Amin, eine $\alpha$-Aminosäure, ein Dipeptid oder ein Tripeptid mit einer freien $\alpha$-Aminogruppe.

Die an der Reaktion teilnehmende Aminogruppe in einem zu einer Verbindung der Formel I komplementären Fragment liegt bevorzugt in freier Form vor, insbesondere wenn die damit reagierende Carboxygruppe in reaktionsfähiger Form vorliegt, sie kann aber auch selbst derivatisiert sein, z.B. durch Reaktion mit einem Phosphit, wie Diäthylchlorphosphit, 1,2-Phenylenchlorphosphit, Aethyldichlorphosphit, Aethylenchlorphosphit oder Tetraäthylpyrophosphit. Ein Derivat eines solchen komplementierenden Bruchstücks mit einer Aminogruppe ist z.B. auch ein Carbaminsäurehalogenid oder ein Isocyanat, wobei die an der Reaktion teilnehmende Amino gruppe durch Halogencarbonyl, z.B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Ist das komplementäre Fragment mit einer Aminogruppe Ammoniak selbst oder ein durch Niederalkyl oder Arylniederalkyl mono- oder disubstituiertes Amin, so stellt auch eine entsprechende Harnstoff-Verbindung ein reaktionsfähiges Derivat dar. Beispielsweise erhält man beim Erhitzen äquimolarer Mengen dieser Harnstoffverbindung und der Komponente mit freier Carboxygruppe entsprechende Verbindungen der Formel I mit zum Teil guter Ausbeute.

Ist das komplementäre Fragment Dimethylamin, so ist auch Dimethylformamid ein reaktionsfähiges Derivat.

Funktionelle Gruppen in Ausgangsmaterialien, deren Umsetzung vermieden werden soll, insbesondere Carboxy-, Amino-, Hydroxy- und Mercaptogruppen, können durch geeignete Schutzgruppen (conventional protecting groups) geschützt sein, die üblicherweise bei der Synthese von Peptid-Verbindungen, aber auch von Cephalosporinen und Penicillinen verwendet werden. Diese Schutzgruppen können bereits in den Vorstufen vorhanden sein und sollen die betreffenden funktionellen Gruppen gegen unerwünschte Nebenreaktionen wie Acylierungen, Verätherungen, Veresterungen, Oxydationen, Solvolyse etc. schützen.

Der Schutz von funktionellen Gruppen durch solche Schutzgruppen, die Schutzgruppen selbst, sowie ihre Abspaltungsreaktionen, sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy, Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxyäthoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, sowie 2-Triniederalkylsilylniederalkoxycarbonyl, z.B. 2-Trimethylsilyläthoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und die Aminogruppe oder die Carboxygruppe eines zweiten Moleküls der Formel I substituiert sein. Verbindungen mit solchen Schutzgruppen lassen sich z.B. mit Dimethylchlorsilan als Silylierungsmittel herstellen.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl und Diphenylmethoxycarbonyl.

Eine Aminogruppe kann z.B. in Form einer Acylamino-, Arylmethylamino-, verätherten Mercaptoamino- oder Silylaminogruppe oder als Azidogruppe geschützt sein.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure oder gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, 2-Triniederalkylsilylniederalkoxycarbonyl, z.B. 2-Trimethylsilyläthoxycarbonyl, oder 2-Triarylsilylniederalkoxycarbonyl, z.B. 2-Triphenylsilyläthoxycarbonyl.

Eine Arylmethylaminogruppe ist z.B. Mono-, Di- oder insbesondere Triphenylmethylamino, z.B. Benzyl-, Diphenylmethyl- oder Tritylamino.

In einer verätherten Mercaptoaminogruppe ist die verätherte Mercaptogruppe in erster Linie substituiertes Arylthio, z.B. 4-Nitrophenylthio.

Eine Silylaminogruppe ist beispielsweise eine Triniederalkylsilyl-aminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. mit Dimethylchlorsilan als Silylierungsmittel herstellen.

Bevorzugte Aminoschutzgruppen sind Acylreste von Kohlensäurehalbestern, insbesondere tert-Butoxycarbonyl, gegebenenfalls substituiertes Benzyloxycarbonyl, z.B. 4-Nitrobenzyloxycarbonyl, Diphenylmethoxycarbonyl, 2-Halogen-niederalkoxycarbonyl, z.B. 2,2,2-Trichloräthoxycarbonyl, ferner Trityl oder Formyl.

Eine Hydroxygruppe kann beispielsweise durch eine Acylgruppe, z.B. durch Halogen, z.B. Chlor, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichloräthoxycarbonyl, 4-Nitrobenzyloxycarbonyl, Diphenylmethoxycarbonyl oder Trityl. Eine Hydroxygruppe kann ferner durch Triniederalkylsilyl, z.B. Trimethylsilyl oder Dimethyl-tert-butylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl, oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Zwei benachbarte Hydroxylgruppen können beispielsweise durch eine vorzugsweise substituierte Methylengruppe geschützt sein, z.B. durch Niederalkyliden, z.B. Isopropyliden, Cycloalkyliden, z.B. Cyclohex-

yliden, oder Benzyliden.

Eine Mercaptogruppe, wie z.B. in Cystein, kann insbesondere durch 5-Alkylierung mit gegebenenfalls substituierten Alkylresten, Thioacetalbildung, S-Acylierung oder durch die Bildung asymmetrischer Disulfid-Gruppierungen geschützt sein. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls am Phenylrest, z.B. durch Methoxy, substituiertes Diphenylmethyl, wie 4,4′-Dimethoxydiphenylmethyl, Triphenylmethyl, Trimethylsilyl, Benzylthiomethyl, Tetrahydropyranyl, Acylaminomethyl, Benzoyl, Benzyloxycarbonyl oder Niederalkylaminocarbonyl, wie Aethylaminocarbonyl.

Die Kondensation zur Herstellung der Amidbindung kann in an sich bekannter Weise durchgeführt werden, beispielsweise wie in Standardwerken, wie "Houben-Weyl, Methoden der organischen Chemie", 4. Auflage, Band 15/II, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Priciples of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diäthyl-, Dipropyl- N-Aethyl-N′-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Aethyl-5-phenyl-1,2-oxazolium-3′-sulfonat und 2-tert-Butyl-5-methylisoxazoliumperchlorat, oder eine geeignete Acylaminoverbindung, z.B. 2-Aethoxy-1-äthoxycarbonyl-1,2-dihydrochinolin, ferner aktivierte Phosphate, z.B. Diphenylphosphorylazid, Diäthylphosphorylcyanid oder Phenyl-N-phenylphosphoramidochloridat.

Gewünschtenfalls wird eine organische Base zugegeben, z.B. ein Triniederalkylamin mit voluminösen Resten, z.B. Aethyldiisopropylamin, oder eine heterocyclischen Base, z.B. Pyridin, 4-Dimethylaminopyridin oder bevorzugt N-Methylmorpholin.

Die Kondensation von Säureanhydriden mit Aminen kann z.B. in Gegenwart von Alkalimetallcarbonaten, z.B. Alkalimetallcarbonaten .oder -hydrogencarbonaten, wie Natrium- oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, gegebenenfalls bei erniedrigter oder erhöhter Temperatur, z.B. in einem Temperaturbereich von etwa -40 °C bis etwa +100 °C, bevorzugt von etwa -10 °C bis etwa +50 °C, und gegebenenfalls unter Inertgas-, z.B. Stickstoffatmosphäre.

Reaktionsfähige Säurederivate können auch in situ gebildet werden. So kann man z.B. N,N′-disubstituierte Amidinoester in situ bilden, indem man das Gemisch des Fragments mit freier Carboxygruppe und des komplementierenden Fragments mit einer Aminogruppe in Gegenwart eines geeigneten disubstituierten Carbodiimids, z.B. Dicyclohexylcarbodiimid, umsetzt. Ferner kann man Amino- oder Amidoreste von solchen Säuren in Gegenwart der zu acylierenden Aminokomponente bilden, indem man das Gemisch der entsprechenden Säure- und Amino-Ausgangsstoffe in Gegenwart eines disubstituierten Carbodiimids, z.B. Dicyclohexylcarbodiimid, und eines N-Hydroxylamins oder N-Hydroxyamids, z.B. N-Hydroxybenztriazol, N-Hydroxysuccinimid oder N-Hydroxy-5-norbornen-2,3-dicarbonsäureimid, gegebenenfalls in Anwesenheit einer geeigneten Base, z.B. 4-Dimethylaminopyridin, N-Methylmorpholin oder Aethyldiisopropylamin, umsetzt.

<u>Verfahren b:</u>

Die Aminoschutzgruppe Z· ist eine übliche Schutzgruppe, wie sie weiter vorn genannt sind, z.B. tert-Butoxycarbonyl oder Benzyloxycarbonyl.

Eine geeignete Schwefel-Ylid-Verbindung ist beispielsweise ein Dialkylsulfoniummethylid, z.B. Dimethylsulfoniummethylid, ein Alkyl- oder Phenyl-dialkylaminosulfoxoniummethylid, z.B. Methyl- oder Phenyldimethylaminosulfoxoniummethylid, oder ein Dialkylsulfoxoniummethylid, z.B. Dimethyl- oder Diäthylsulfoxononiummethylid.

Die betreffende Schwefel-Ylid-Verbindung wird zweckmässigerweise in situ aus dem entsprechenden Sulfonium- bzw. Sulfoxoniumsalz und einer Base, z.B. Natriumhydrid, in einem dipolar aprotischen Lösungsmittel, z.B. Dimethylsulfoxid, oder einem Aether, z.B. Tetrahydrofuran oder 1,2-Dimethoxyäthan, hergestellt und anschliessend mit der Verbindung der Formel IX umgesetzt. Die Umsetzung erfolgt normalerweise bei Raumtemperatur, unter Kühlen z.B. bis auf -20 °, oder unter leichtem Erwärmen, z.B. bis

auf 40°. Das gleichzeitig gebildete Sulfid, Sulfinamid bzw. Sulfoxid wird bei der anschliessenden wässrigen Aufarbeitung entfernt.

Eine nukleofuge Abgangsgruppe X ist insbesondere mit einer starken anorganischen oder organischen Säure verestertes Hydroxy, wie mit einer Mineralsäure, z.B. Halogenwasserstoffsäure, wie Chlorwasserstoff-, Bromwasserstoff- oder Jodwasserstoffsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon-oder p-Toluolsulfonsäure verestertes Hydroxy oder mit Stickstoffwasserstoffsäure verestertes Hydroxy.

Ein die nukleofuge Abgangsgruppe X einführendes Reagens ist je nach der Bedeutung von X die freie Säure HX, z.B. Jod- oder Bromwasserstoffsäure oder ein Salz dieser Säure, z.B. Natrium- oder Kaliumjodid.

Vorzugsweise werden die Reaktionsbedingungen so gewählt, dass die Reaktion im wesentlichen als nukleophile Substitution zweiter Ordnung abläuft. Z.B. kann man zur Herstellung einer Verbindung der Formel XI, worin X für eine Abgangsgruppe mit hoher Polarisierbarkeit, z.B. für Jod, steht, eine Verbindung der Formel X in einem dipolaren aprotischen Lösungsmittel, z.B. Aceton, Acetonitril, Nitromethan oder Dimethylformamid, mit einem Alkalimetallsalz von HX, z.B. Natriumjodid, umsetzen.

Die Reaktion kann auch zweistufig durchgeführt werden, wobei eine Verbindung der Formel X in Wasser, das gegebenenfalls ein organisches Lösungsmittel, z.B. einen Alkohol, wie Aethanol, einen Aether, z.B. Tetrahydrofuran oder Dioxan, oder Aceton, als Lösungsvermittler enthält, mit einem Alkalimetallhydroxid, z.B. Natrium- oder Kaliumhydroxid, oder mit einer verdünnten Mineralsäure, z.B. Salzsäure oder Schwefelsäure, oder einer starken organischen Sulfonsäure, z.B. eine der oben im Zusammenhang mit der Abgangsgruppe X genannten Sulfonsäure, umsetzt und das dabei gebildete Diol der Formel XI, worin X OH bedeutet, mit einem den Rest X, wie er oben definiert ist, einführenden Reagens umsetzt, beispielsweise mit einem Halogenid einer organischen Sulfonsäure, z.B. Methansulfonsäurechlorid, Benzolsulfonsäurechlorid oder p-Toluolsulfonsäurechlorid, mit einem Thionylhalogenid, z.B. Thionylchlorid, oder einem Phosphortrihalogenid, z.B. Phosphortrichlorid oder Phosphortribromid. Dieses den Rest X einführende Reagens wird bevorzugt in äquimolarer Menge oder in einem geringen Ueberschuss in einem inerten Lösungsmittel, z.B. einem Halogenkohlenwasserstoff, wie Methylenchlorid, oder einem Aether, z.B. Diäthyläther, Tetrahydrofuran oder Dioxan, oder auch in Acetonitril oder Dimethylformamid bei Temperaturen zwischen -20° und +50°, gegebenenfalls in Gegenwart einer organischen oder anorganischen Base, z.B. Triäthylamin, Aethyldiisopropylamin, Pyridin oder 4-Dimethylaminopyridin, oder Natriumbicarbonat, Calciumcarbonat oder Magnesiumcarbonat, umgesetzt.

Carbonylverbindungen der Formel XII sind Aldehyde oder vorzugsweise Ketone, worin jeder der Reste $R_a$ und $R_b$ Niederalkyl, vorzugsweise mit bis zu 4 Kohlenstoffatomen, z.B. Methyl oder Aethyl, Aryl oder Arylniederalkyl mit bis zu 10 Kohlenstoffatomen, z.B. Phenyl oder Benzyl darstellt, insbesondere Niederalkanone, z.B. Aceton, oder, wenn $R_a$ und $R_b$ zusammen gegebenenfalls überbrücktes Alkyliden bedeuten, zyklische Ketone, insbesondere Cycloalkanone, z.B. Cyclohexanon, oder Bicycloalkanone, z.B. Campher, ebenfalls optisch aktive Ketone oder Aldehyde.

Derivate der Carboxylverbindung der Formel XII sind z.B. Acetale oder Enoläther, vorallem Acetondimethylketal (2,2-Dimethoxypropan) oder 2-Methoxypropen.

Saure Reagenzien sind starke und schwache Brönstedt-Säuren, beispielsweise Mineralsäuren, z.B. Schwefelsäure oder Salzsäure, oder organische Säuren, z.B. p-Toluolsulfonsäure, oder Lewis-Säuren, z.B. Bortrifluoridätherat, Kupfersulfat oder Eisen(III)chlorid.

Als Lösungsmittel eignen sich unpolare, aprotische Lösungsmittel, z.B. Diäthyläther oder Kohlenwasserstoffe, z.B. Benzol oder Toluol.

Die Reaktion wird vorzugsweise bei erhöhten Temperaturen von etwa 40° bis etwa 150°, vor allem bei der Siedetemperatur des jeweiligen Reaktionsgemisches, durchgeführt.

Eine Carboxyschutzgruppe $Z_2$ ist eine der weiter vorn unter Verfahren a) genannten Carboxyschutzgruppen, z.B. tert-Butyl, Benzyl oder 4-Nitrobenzyl, sowie unverzweigtes Niederalkyl, z.B. Methyl oder Aethyl.

Das Salz eines Carbonsäureesters der Formel XIV wird durch Umsetzung des Carbonsäureesters der Formel

$$R_5-CH_2-\overset{O}{\overset{\|}{C}}-OZ_2 \qquad \text{(XIVa)}$$

mit einem geeigneten Metallisierungsmittel hergestellt.

Geeignete Metallisierungsreagenzien sind substituierte und unsubstituierte Alkalimetallamide, Alkalimetallhydride oder Alkalimetallniederalkylverbindungen, worin das Alkalimetall Natrium oder insbesondere Lithium ist, z.B. Natrium- oder Lithiumamid, Lithium-bis-trimethylsilylamid, Natriumhydrid, Lithiumhydrid

oder bevorzugt Lithiumdiisopropylamid oder Butyllithium.

Für die Metallierungsreaktion geeignete Lösungsmittel dürfen keinen aktiven Wasserstoff enthalten und sind z.B. Kohlenwasserstoffe, z.B. Hexan, Benzol, Toluol oder Xylol, schwach polare Aether, z.B. Diäthyläther, Tetrahydrofuran oder Dioxan, oder Säureamide, z.B. Hexamethylphosphorsäuretriamid. Das metallierte Zwischenprodukt der Formel XIV braucht nicht isoliert, sondern kann im Anschluss an die Metallierungsreaktion mit der Verbindung der Formel XIII umgesetzt werden. Die Metallierungsreaktion erfolgt bei Temperaturen von etwa -100° bis ca. 0°, vorzugsweise unterhalb -30°. Die weitere Umsetzung unter Bildung von XV kann bei der gleichen Temperatur und gegebenenfalls langsamem Erwärmen bis zum Siedepunkt des Reaktionsgemisches erfolgen.

Die Abspaltung der Carboxyschutzgruppe $Z_2$ erfolgt unter den weiter vorn für die Abspaltung von Carboxyschutzgruppen genannten Reaktionsbedingungen. Wenn $Z_2$ beispielsweise unverzweigtes Niederalkyl ist, kann der Niederalkylester der Formel XV nach den üblichen Verseifungsmethoden, z.B. basisch, z.B. in Gegenwart von Kalium-tert-butylat, in Aether oder in einem anderen organischen Lösungsmittel, gegebenenfalls in Gegenwart von Wasser, gespalten werden. Bei basischer Verseifung des Niederalkylesters der Formel XV erhält man das Salz der freien Carbonsäure, welches man in die freie Carbonsäure umwandeln kann, z.B. in wässrig-alkoholischer Lösung. Die freie Carbonsäure der Formel XV oder das Salz dieser Carbonsäure kann nach den weiter vorn beschriebenen Verfahren amidiert, verestert oder mit einer am terminalen C-Atom gegebenenfalls amidierten oder veresterten Aminosäure bzw. Di- oder Tripeptidrest kondensiert werden.

Die Umsetzung einer erhältlichen Verbindung der Formel XVI mit einem geeigneten Solvolysereagens erfolgt unter Ringöffnung und Abspaltung von $R_a(C=O)R_b$ und unter Abspaltung der Schutzgruppe Z·, wenn diese eine solvolytisch abspaltbare Schutzgruppe ist. Ist Z· beispielsweise eine hydrogenolytisch abspaltbare Schutzgruppe, z.B. Benzyloxycarbonyl, kann die Ringöffnung und die Abspaltung dieser Schutzgruppe ebenfalls gleichzeitig erfolgen, indem die Hydrogenolyse in Gegenwart eines Katalysators, z.B. Palladium-Kohle, und eines Solvolysemittels, z.B. Methanol, durchgeführt wird.

Geeignete Solvolysereagentien sind beispielsweise organische Säuren, z.B. Niederalkancarbonsäuren, z.B. Eisessig oder Ameisensäure, Anhydride von Niederalkancarbonsäuren, z.B. Essigsäureanhydrid, oder Sulfonsäuren, z.B. p-Toluolsulfonsäure, Mineralsäuren, z.B. Schwefel- oder Salzsäure, Niederalkanole, z.B. Methanol oder Aethanol, oder Niederalkandiole, z.B. Aethylenglykol.

Die genannten Solvolysereagenzien werden unverdünnt oder mit Wasser verdünnt zugegeben. Die Solvolyse kann auch mit reinem Wasser durchgeführt werden. Die Solvolyse mit einem sauren Reagens findet bevorzugt in wässriger Lösung dieses Reagens und bei Temperaturen von etwa -20° bis etwa zum Siedepunkt des Reaktionsgemisches, bevorzugt bei Zimmertemperatur bis zum Siedepunkt des Reaktionsgemisches, statt.

Die gesamte Reaktionsfolge des Verfahrens b) kann in situ oder nach Isolierung von einzelnen oder sämtlichen verfahrensgemäss erhältlichen Vorprodukten erfolgen.

Nachoperationen:

In einer erhältlichen Verbindung der Formel I, worin $R_3$, $R_4$ und $R_5$ die genannten Bedeutungen haben und $R_6$ den Rest einer Aminosäure, eines Dipeptids oder eines Tripeptids mit einer endständigen Carboxamid-, Carboxy- oder Carbonsäureestergruppe darstellt, kann man die endständige Carboxamidgruppe substituieren, die in freier oder in reaktionsfähiger Form vorliegende Carboxygruppe verestern bzw. eine veresterte Carboxygruppe in eine Carboxamidgruppe überführen.

Die Substitution der terminalen Carboxamidgruppe oder einer anderen Aminogruppe erfolgt z.B. durch Alkylierung.

Geeignete Mittel zur Alkylierung der terminalen Carboxamidgruppe in einer Verbindung der Formel I sind z.B. Diazoverbindungen, z.B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z.B. in Gegenwart eines Edelmetalls in fein verteilter Form, z.B. Kupfer, oder eines Edelmetallsalzes, z.B. Kupfer(I)-chlorid oder Kupfer(II)-sulfat.

Weitere Alkylierungsmittel sind die in der Deutschen Offenlegungsschrift 2 331 133 genannte Alkylierungsmittel, z.B. Alkylhalogenide, Sulfosäureester, Meerweinsalze oder 1-substituierte 3-Aryltriazene, welche man unter den dort genannten Reaktionsbedingungen mit einer Verbindung der Formel I mit endständiger Carboxamidgruppe umsetzen kann.

Zur Veresterung einer endständigen Carboxygruppe in einer Verbindung der Formel I kann man die

freie Säure verwenden oder die freie Säure in eines der unter Verfahren a) genannten reaktionsfähigen Derivate überführen und mit einem Alkohol umsetzen, oder man kann die freie Säure oder ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit dem Halogenid eines Alkohols umsetzen.

Die Veresterung einer endständigen Carboxygruppe kann mit den für die Substitution der Carboxamidgruppe oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

In einer erhältlichen Verbindung der Formel I kann man eine endständige veresterte Carboxygruppe durch Aminolyse mit Ammoniak oder einem primären oder sekundären Amin in eine gegebenenfalls substituierte Carboxamidgruppe überführen.

Die Aminolyse kann nach den im Organikum, letzte Auflage, VEB Deutscher Verlag der Wissenschaften, Berlin (Ost) für solche Umsetzungen genannten Reaktionsbedingungen erfolgen.

In einer erhältlichen Verbindung der Formel I, worin die Substituenten die genannten Bedeutungen haben und mindestens eine freie Hydroxygruppe vorhanden ist und die übrigen funktionellen Gruppen gegebenenfalls in geschützter Form vorliegen, kann man die freie Hydroxygruppe, z.B. die Hydroxygruppe $R_4$, veräthern oder verestern.

Die Verätherung dieser Hydroxygruppe kann mit den oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Die Veresterung der freien Hydroxygruppe kann mit den üblichen Acylierungsmitteln und den üblichen im Organikum angegebenen Reaktionsbedingungen erfolgen, z.B. mit Essigsäureanhydrid.

Die genannten Alkylierungsreaktionen, Verätherungen, Veresterungen etc. können statt im Endstoff auch in einem Ausgangsmaterial entsprechend durchgeführt werden.

In einer erhältlichen Verbindung der Formel I, worin eine oder mehrere funktionelle Gruppen geschützt sind, können diese Gruppen, z.B. Carboxy-, Amino-, Hydroxy- und/oder Mercaptogruppen, in an sich bekannter Weise, mittels Solvolyse, insbesondere Hydrolyse, gegebenenfalls enzymatische Hydrolyse, Alkoholyse oder Acidolyse, oder mittels Reduktion, insbesondere Hydrogenolyse, oder chemische Reduktion, gegebenenfalls stufenweise oder gleichzeitig, freigesetzt werden. Die Abspaltung der Schutzgruppen ist in den weiter vorn im Abschnitt "Schutzgruppen" genannten Standardwerken beschrieben.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch durch Reduktion, z.B. durch Behandeln mit einem Alkalimetall-, z.B. Natrium-dithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumjodid, gespalten werden. 2-Triniederalkylsilylniederalkoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie oben beschrieben, freigesetzt werden. Verestertes Carboxy kann auch enzymatisch gespalten werden, z.B.

verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nukleophilen, vorzugsweise salzbildenden Reagenz, wie Natriumthiophenolat, und 4-Nitrobenzyloxycarbonylamino auch durch Behandeln mit einem Alkalimetall-, z.B. Natrium-dithionit, gespalten werden. Gegebenenfalls substituiertes Diphenylmethoxycarbonylamino, tert-Niederalkoxycarbonylamino oder 2-Triniederalkylsilylniederalkoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kolenwasserstoff, z.B. Methylenchlorid, oder auch in Wasser oder einem Gemisch von Wasser und einem organischen Lösungsmittel, wie einem Alkohol oder Dioxan, bei etwa 20° C bis 25° C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert-Niederalkyl oder durch einen 2-oxa- oder 2-thiaaliphatischen oder -cycloaliphatischen Kohlenwasserstoffrest, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Aethylhexansäure, oder mit organischen Alkali- oder Erdalkalimetallsalzen, z.B. Natriumhydrogencarbonat, oder mit Ammoniak oder einem geeigneten organische Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscher reagenz. Innere Salze von Verbindungen der Formel I, welche z.B. eine freie Carboxygruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen übergeführt werden: Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in

Diastereomere, beispielsweise durch Umsetzung mit optisch aktiven Säuren oder Basen, gespalten werden.

An einzelnen Chiralitätszentren in einer Verbindung der Formel I, z.B. dem CH-$R_4$-C-Atom, kann die Konfiguration gezielt umgekehrt werden. Beispielsweise kann man die Konfiguration am CH-$R_4$-Atom durch nukleophile Substitution zweiter Ordnung nach Ueberführung der Hydroxygruppe in eine Abgangsgruppe X und Reaktion mit einem den Substituenten $R_4$ einführendes Reagens umkehren.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Die folgenden Beispiele dienen zur Illustration der Erfindung, Temperaturen werden in Celsiusgraden angegeben.

Die $R_f$-Werte werden, wenn nicht anders angegeben, auf Kieselgeldünnschichtplatten in folgenden Lösungsmittelsystemen ermittelt:

| | | |
|---|---|---|
| N1 | Chloroform-Methanol | 9:1 |
| N2 | Chloroform-Methanol | 95:5 |
| N3 | Essigester-Hexan | 1:1 |
| N4 | Essigester-Hexan | 1:4 |
| N5 | Essigester-Hexan | 1:9 |
| N6 | Methylenchlorid-Aether | 4:1 |
| N7 | Methylenchlorid-Aether | 1:1 |
| N8 | Methylenchlorid-Methanol | 9:1 |
| N9 | Methylenchlorid-Methanol | 6:1 |
| N10 | Methylenchlorid-Methanol | 20:1 |
| N11 | Methylenchlorid-Methanol | 10:1 |
| N12 | Methylenchlorid-Methanol | 5:1 |
| B1 | Chloroform-Methanol-Ammoniak konz. | 40:10:1 |
| B2 | Chloroform-Methanol-Ammoniak konz. | 350:50:1 |
| B3 | Methylenchlorid-Methanol-Ammoniak konz. | 1000:50:1 |
| B4 | Methylenchlorid-Methanol-Ammoniak konz. | 350:50:1 |
| B5 | Methylenchlorid-Methanol-Ammoniak konz. | 140:10:1 |
| B6 | Methylenchlorid-Methanol-Ammoniak konz. | 105:50:1 |
| B7 | Methylenchlorid-Methanol-Ammoniak konz. | 90:10:1 |
| B8 | Methylenchlorid-Methanol-Ammoniak konz. | 80:10:1 |
| B9 | Methylenchlorid-Methanol-Ammoniak konz. | 65:10:1 |
| B10 | Methylenchlorid-Methanol-Ammoniak konz. | 50:10:1 |
| B11 | Methylenchlorid-Methanol-Ammoniak konz. | 40:10:1 |
| B12 | Methylenchlorid-Methanol-Ammoniak konz. | 40:25:1 |
| B13 | Methylenchlorid-Methanol-Ammoniak konz. | 30:10:1 |
| B14 | Methylenchlorid-Methanol-Ammoniak konz. | 25:10:1 |
| B15 | Methylenchlorid-Methanol-Ammoniak konz. | 20:5:1 |
| B16 | Methylenchlorid-Methanol-Ammoniak konz. | 10:10:1 |
| B17 | Essigester-Pyridin-Eisessig-Wasser | 62:21:6:11 |
| B18 | n-Butanol-Pyridin-Eisessig-Wasser | 38:24:8:30 |
| B19 | Pyridin-n-Butanol-n-Amylalkohol-Methyläthylketon-Eisessig-Ameisensäure-Wasser | 25:20:15:10:3:3:25 |
| B20 | n-Butanol-Pyridin-Ameisensäure-Wasser | 42:24:4:20 |
| B21 | n-Butanol-Pyridin-Eisessig-Wasser | 42:24:4:30 |

| | | |
|---|---|---|
| B22 | n-Butanol-Pyridin-Ameisensäure-Wasser | 44:24:2:20 |
| S1 | Essigester-Pyridin-Eisessig-Wasser | 62:21:6:11 |
| S2 | Essigester-Methanol-Eisessig-Wasser | 67:10:12:23 |
| S3 | Chloroform-Methanol-Wasser-Eisessig | 300:108:20:2 |
| S4 | Chloroform-Methanol-Wasser-Eisessig | 75:27:5:0,5 |
| S5 | Chloroform-Methanol-Wasser-Eisessig | 70:40:10:0,5 |
| S6 | Chloroform-Methanol-Wasser-Eisessig | 90:10:1:0,5 |
| S7 | Chloroform-Methanol-Wasser-Eisessig | 55:47:13:0,5 |
| S8 | Chloroform-Methanol-Wasser-Eisessig | 80:20:3:3 |
| S9 | Chloroform-Methanol-Wasser-Eisessig | 70:30:3:3 |
| S10 | Methylenchlorid-Methanol-Wasser-Eisessig | 750:270:50:5 |
| S11 | n-Butanol-Eisessig-Wasser | 67:10:23 |

Beispielsweise bedeutet die Abkürzung "$R_f$(N1)", dass der $R_f$-Wert im System N1 ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

| Abkürzungen für Aminosäuren und Aminosäurederivate: | |
|---|---|
| H-Ala-OH | L-Alanin |
| H-Arg-OH | L-Arginin |
| H-Glu-OH | L-Glutaminsäre |
| H-Glu(OR)-OH | L-Glutaminsäure, worin die Carbonsäure-Funktion der Seitenkette mit dem Rest R verestert ist |
| H-Gly-OH | Glycin |
| H-His-OH | L-Histidin |
| H-Ile-OH | L-Isoleucin |
| H-Leu-OH | L-Leucin |
| H-Lys-OH | L-Lysin |
| H-Lys(R)-OH | L-Lysin, worin die Amino-Funktion der Seitenkette mit dem Rest R substituiert ist |
| H-Phe-OH | L-Phenylalanin |
| H-(N-methyl-Phe)-OH | N-Methyl-L-phenylalanin |
| H-Sta-OH | Statin, (3S,4S)4-Amino-3-hydroxy-6-methylheptansäure |
| H-Val-OH | L-Valin |
| $-NH_2$ statt -OH: C-terminales (Carbonsäure)Amid | |
| -OMe statt -OH: C-terminaler (Carbonsäure)Methylester | |

Das Fragment mit der Bezeichnung -Leu $\underline{^{C}}$ Val- bedeutet das zweiwertige Radikal von (2S,4S,5S)-2-Isopropyl-4-hydroxy-5-amino-7-methyloctansäure und hat die Formel

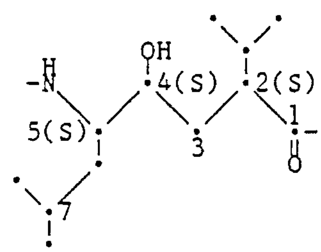

Das Fragment mit der Bezeichnung -Leu $\stackrel{cx}{-}$ Val- leitet sich vom Fragment -Leu $\stackrel{c}{-}$ Val- durch Ueberbrückung von NH und OH durch eine Isopropyliden-Gruppe ab und hat die Formel

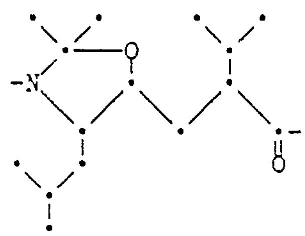

Das Fragment mit der Bezeichnung -Leu $\stackrel{c}{-}$ Gly(R)- bedeutet das zweiwertige Radikal von (4S.5S)-2-R-4-hydroxy-5-amino-7-methyloctansäure mit (R)-oder (S)-Konfiguration am C-Atom 2 und hat die Formel

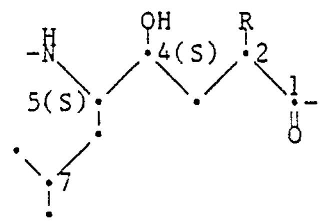

Das Fragment mit der Bezeichnung -Leu $\stackrel{cx}{-}$ Gly(R)- leitet sich vom Fragment -Leu $\stackrel{c}{-}$ Gly(R)- durch Ueberbrückung von NH und OH durch eine Isopropyliden-Gruppe ab.

Weitere Abkürzungen:

Ac = Acetyl
BOC = tert-Butoxycarbonyl
DC = Dünnschichtchromatographie, wenn nicht anders angegeben auf Kieselgel
DCCI = Dicyclohexylcarbodiimid
DCH = Dicyclohexylharnstoff
DMF = Dimethylformamid
DMSO = Dimethylsulfoxid
HOBt = 1-Hydroxybenzotriazol
Min. = Minute(n)
Sdp. = Siedepunkt
Smp. = Schmelzpunkt
Std. = Stunde(n)
THF = Tetrahydrofuran
Vol. = Volumen(anteile)
Z = Benzyloxycarbonyl

Beispiel 1: H-Leu $\stackrel{c}{-}$ Val-Ile-His-$NH_2$ und Kondensation mit Z-Phe-His-OH

1,7 g Z-Phe-His-OH (hergestellt nach der Vorschrift in Chem. Ber. 94, 2768 (1961)), 1,71 g H-Leu $\stackrel{c}{-}$ Val-Ile-His-$NH_2$ und 544 mg HOBt (Fluka purum, enthält 11-13% Wasser) werden in 60 ml DMF abs. auf 0° abgekühlt. Nach Zugabe von 1,03 g DCCI wird während 15 Std. bei Eisbadkühlung und anschliessend während 2 Tagen bei Raumtemperatur gerührt. Die Suspension wird im Hochvakuum eingeengt und der Rückstand während 60 Min. in einem Gemisch von Methanol-Wasser-Eisessig (94:3:3) in einem Oelbad von 60° gerührt. Nach Entfernen des Lösungsmittels im Hochvakuum wird der Rückstand mittels Flash-Chromatographie aufgetrennt (1000 g Kieselgel 60, 40-63 µm, Fliessmittel-System B8). Die produkthaltigen Fraktionen werden eingedampft. Nach dem Trocknen im Hochvakuum erhält man Z-Phe-His-Leu $\stackrel{c}{-}$ Val-Ile-His-$NH_2$ als farbloses Pulver. $R_f$(B4) = 0,07; $R_f$(S3) = 0,29. Das Produkt kann durch Aufrühren in Methanol und Versetzen mit 2 Aequivalenten wässriger 1N HCl in das Dihydrochlorid überführt werden.

H-Leu $\stackrel{c}{-}$ Val-Ile-His-$NH_2$ kann nach folgendem Reaktionsschema hergestellt werden:

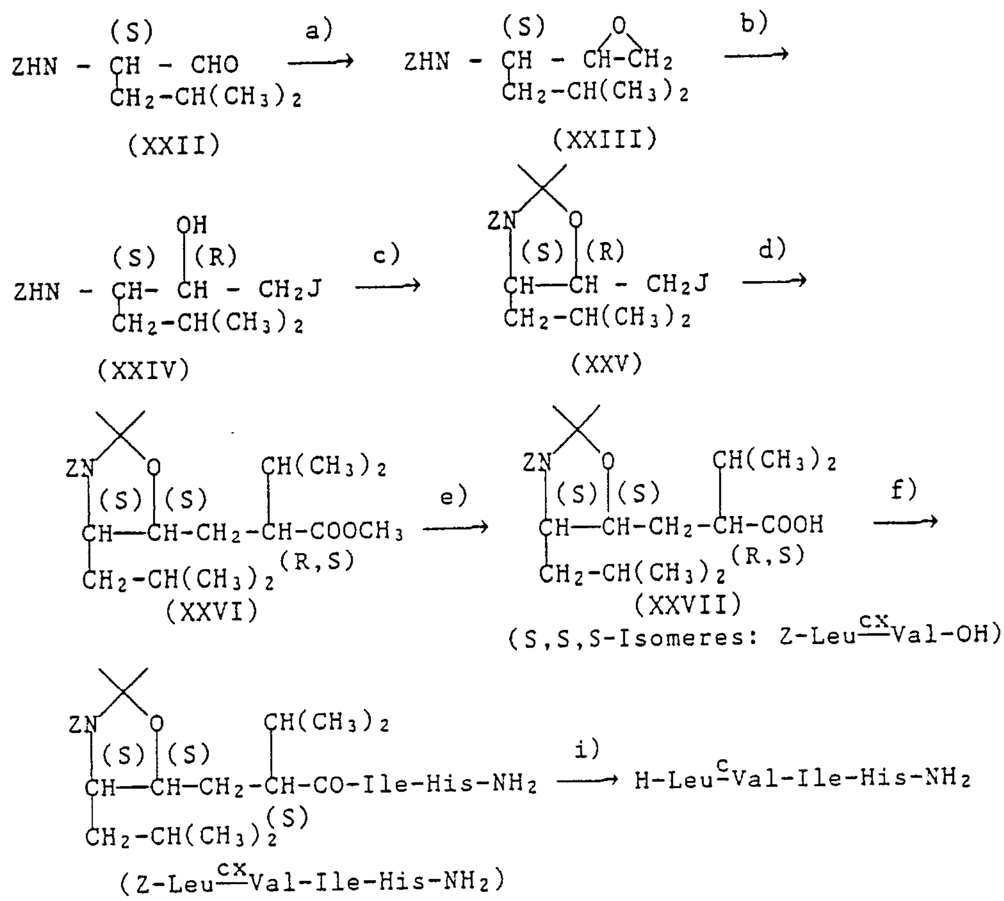

(XXII) (XXIII) (XXIV) (XXV) (XXVI) (XXVII)
(S,S,S-Isomeres: Z-Leu $\stackrel{cx}{-}$ Val-OH)
H-Leu $\stackrel{c}{-}$ Val-Ile-His-$NH_2$
(Z-Leu $\stackrel{cx}{-}$ Val-Ile-His-$NH_2$)

a) 8,75 g Natriumhydriddispersion (55% in Oel) werden in einem trockenen Sulfierkolben unter Argon durch dreimaliges Aufrühren in 85 ml Petroläther (Sdp. 40-60°) und anschliessendem Abdekantieren des Lösungsmittels vom Oel befreit. Nach Trocknen im Hochvakuum wird ein graues Pulver erhalten, das tropfenweise mit einer Lösung von 44,1 g Trimethylsulfoxoniumiodid in 180 ml Dimethylsulfoxid versetzt wird, wobei die Temperatur auf ca. 40° steigt. Die graue Suspension wird bei Raumtemperatur 1 Std. gerührt und anschliessend innerhalb von 50 Min. bei 10° mit einer Lösung von 43,4 g (S)-2-Benzyloxycarbonylamino-4-methylpentanal (XXII) (Herstellung: Helvetica Chimica Acta 66, 450 (1983)) in 180 ml DMSO versetzt. Die gelbe Suspension wird 15 Min. bei 10° und anschliessend 1,5 Std. bei Raumtemperatur gerührt. Die gelblich-trübe Lösung wird auf 500 g Eis gegossen. Die wässrige Lösung wird mit Aether extrahiert, die organische Phase mit Wasser gewaschen und nach Trocknen über Natriumsulfat eingedampft. Der ölige Rückstand wird mittels Flash- Chromatographie an 2 kg Kieselgel (40-63 µm) mit einem Gemisch von Methylenchlorid/n-Hexan/ Essigsäureäthylester (5:10:3) aufgetrennt Die produkthaltigen Fraktionen werden vereinigt, eingedampft und im Hochvakuum getrocknet. Man erhält das Epoxid (XXIII) (Diastereomerengemisch, ca. 3:1) als leicht gelbliches Oel. $R_f$(B1) = 0,57; $R_f$(N4) = 0,16.

b) 33,8 g der Verbindung (XXIII) werden in 255 ml Acetonitril aufgenommen und die erhaltene

Lösung auf 0° abgekühlt. Nach Zugabe von 19,24 g Natriumjodid werden während 30 Min. tropfenweise bei 0° 16,27 ml Trimethylchlorsilan zugegeben. Das Gemisch wird 40 Min. bei 0-3° gerührt und anschliessend auf 250 ml eiskaltes Wasser gegossen. Das wässrige Gemisch wird mit Aether extrahiert und die organische Phase mit 10%-iger wässriger Natriumthiosulfatlösung und gesättigter, wässriger Natriumchloridlösung gewaschen. Nach Trocknen über Natriumsulfat und Eindampfen erhält man ein öliges Gemisch des gewünschten Alkohols (XXIV) und des entsprechenden Trimethylsilyläthers (beides Diastereomerengemische). Der freie Alkohol wird mittels Flash-Chromatographie abgetrennt (2 kg Kieselgel 60, 40-63 µm, Laufmittel Essigsäureäthylester Hexan 1:4). Nach Eindampfen der produkthaltigen, vereinigten Fraktionen erhält man den Alkohol (XXIV) als Oel. Nach Vereinigen und Eindampfen der Fraktionen, welche den Trimethylsilyläther enthalten, nimmt man den Rückstand in THF auf, versetzt mit einer Lösung von 2,67 g Tetrabutylammoniumfluorid in Wasser und rührt das so erhaltene Gemisch 3 Tage lang bei Raumtemperatur. Die Lösung wird anschliessend zwischen Wasser und Aether ausgeschüttelt. Die organische Phase wird mit Wasser gewaschen, über Natriumsulfat getrocknet und eingedampft. Nach Trocknen im Hochvakuum erhält man eine zusätzliche Menge des Diastereomerengemisches der Verbindung (XXIV) als Oel. Die gesamte Menge des Diastereomerengemisches (XXIV) wird zwecks weiterer Reinigung in zwei Teilen durch Mitteldruckchromatographie aufgetrennt (1,4 kg Lichroprep® Si 60, 25-40 µm, Laufmittel: Essigsäureäthylester Hexan (1:8), Fraktionen von ca. 220 ml). Durch Kristallisation aus Petroläther (Sdp. 40-60°) lässt sich die stärker polare Komponente aus dem Rohmaterial und den Mischfraktionen teilweise anreichern (Smp. 104-106°). Die beiden Diastereomeren fallen in einem Verhältnnis von ca. 3:1 an. Bei der weniger polaren, öligen Komponente handelt es sich um die gewünschte Verbindung (XXIV). $R_f$(N4) = 0,16; $R_f$(B1) = 0,54.

c) 66,6 g der Verbindung (XXIV) und 1,62 g p-Toluolsulfonsäuremonohydrat werden in 1500 ml 2,2-Dimethoxypropan 3 Std. lang unter Rückfluss erhitzt. Nach Abkühlen auf Raumtemperatur wird das Gemisch zwischen 1000 ml Aether und 500 ml gesättigter, wässriger Natriumhydrogencarbonatlösung ausgeschüttelt. Die organische Phase wird mit Wasser gewaschen, über Natriumsulfat getrocknet und eingedampft. Das halbkristalline Rohprodukt wird mittels Flash-Chromatographie gereinigt (2 kg Kieselgel 60, 40-63 µm, Laufmittel: Essigsäureäthylester Hexan (1:6), Fraktionen zu ca. 400 ml). Durch Eindampfen der vereinigten, produkthaltigen Fraktionen erhält man die Verbindung (XXV) als leicht gelbliche Kristalle. $R_f$(N4) = 0,48; $R_f$(N5) = 0,26.

d) 10,82 ml Diisopropylamin werden in 100 ml absolutem Tetrahydrofuran unter Argon gelöst und auf 0° abgekühlt. Anschliessend wird bei 0-5° das Gemisch 20 Min. lang tropfenweise mit einer 1,6 M Lösung von n-Butyllithium in Hexan versetzt und 15 Min. gerührt. Dann werden bei -70° bis -75° 10,1 ml Isovaleriansäuremethylester zugetropft und die Mischung 1,5 Std. bei -75° gerührt. Bei -60° bis -75° werden unter Rühren 190 ml Hexamethylphosphorsäuretriamid zugetropft. Die entstandene Suspension wird 10 Min. lang gerührt und schliesslich bei -70° bis -75° in 5 Min. tropfenweise mit einer Lösung von 30,0 g der Verbindung (XXV) in 50 ml Tetrahydrofuran versetzt. Das Reaktionsgemisch wird bei Raumtemperatur während 2,5 Std. gerührt und schliesslich auf ein Gemisch von 450 ml gesättigter, wässriger Ammoniumchloridlösung und 500 g Eis gegossen. Die wässrige Phase wird mit Aether extrahiert und die organische Phase mit Wasser gewaschen und über Natriumsulfat getrocknet. Nach Eindampfen erhält man das Diastereomerengemisch der Verbindung (XXVI) als gelbes Oel. $R_f$(N5) = 0,20; $R_f$(N4) = 0,40 (Werte für die weniger polare Komponente).

e) 10,7 g Kalium-tert-butylat werden in 80 ml Aether bei ca. 5° mit 1,07 ml Wasser versetzt. Die weisse Suspension wird noch 10 Min. im Eisbad gerührt und darauf mit 10,0 g der Verbindung (XXVI) (Diastereomerengemisch) in 80 ml Aether versetzt, wobei die Temperatur unterhalb 10° gehalten wird. Das Reaktionsgemisch wird nun während 18 Stunden bei Raumtemperatur gerührt und schliesslich auf 1600 ml gesättigte, wässrige Ammoniumchloridlösung gegossen. Die wässrige Phase wird mit Aether extrahiert und die organische Phase mit gesättigter, wässriger Natriumchloridlösung gewaschen, über Natriumsulfat getrocknet und eingedampft. Das ölige Rohprodukt wird durch Mitteldruckchromatographie aufgetrennt (740 g Lichroprep® Si 60, 25-40 µm, Laufmittel Essigsäureäthylester Hexan 1:5). Z-Leu$^{c}$ $^{x}$Val-OH, die weniger polare Komponente der Verbindung (XXVII) mit der gewünschten Konfiguration des an die Isopropylgruppe gebundenen C-Atoms (S-Konfiguration) wird als gelbes Oel erhalten. $R_f$ (Methylenchlorid Methanol Wasser 500:10:1) = 0,13; $R_f$ (N6) = 0,58.

f) Z-Leu $\xrightarrow{cx}$ Val-Ile-His-$NH_2$: 1,96 g Z-Leu $\xleftarrow{cx}$ Val-OH, 1,42 g H-Ile-His-$NH_2$ (Herstellung siehe g) und 0,74 g HOBt werden in 15 ml DMF auf 0° abgekühlt. Nach Zugabe von 1,3 g DCCI wird 2 Std. lang bei 0° und dann 18 Std. lang bei Raumtemperatur gerührt. Der entstandene DCH wird abfiltriert und das Lösungsmittel des Filtrats im Hochvakuum eingedampft. Der Rückstand wird in einem Gemisch von 25 ml Methanol/Wasser Essigsäure (94:3:3) aufgenommen und 1 Std. lang bei 60° gerührt. Dann wird bis zur Trockne eingedampft und der Rückstand in 40 ml Methanol im Eisbad während 30 Min. verrührt. Nach

Filtrieren und Abdampfen des Lösungsmittels wird der Rückstand in n-Butanol aufgenommen und mit gesättigter Natriumhydrogencarbonatlösung und gesättigter Natriumchloridlösung gewaschen. Die organische Phase wird über Natriumsulfat getrocknet und eingedampft. Das Rohprodukt wird durch Mitteldruckchromatographie aufgetrennt (240 g Lichroprep® Si 60, 25-40 µm, Fraktionen zu ca. 40 ml, Druck ca. 8 bar, Laufmittel: Methylenchlorid/Methanol/Wasser 80:10:1, Fliessgeschwindigkeit 40 ml/Min.). Nach ca. 20 Min. wird das Produkt eluiert. Durch Eindampfen der produkthaltigen Fraktionen erhält man die Titelverbindung. $R_f$(S3) = 0,19; $R_f$(B4) = 0,48.

g) H-Ile-His-$NH_2$: 2,5 g Z-Ile-His-$NH_2$ werden in 25 ml Methanol-Wasser (9:1) gelöst und in Gegenwart von 400 mg Palladium-Kohle (10 %) unter $CO_2$-Absorption bis zur Sättigung hydriert. Der Katalysator wird abfiltriert, das Filtrat zur Trockne eingeengt und der Rückstand durch Flash-Chromatographie an Kieselgel im System S9 gereinigt. Die produkthaltigen Fraktionen ergeben nach Einengen im Rotationsverdampfer und Trocknen im Hochvakuum H-Ile-His-$NH_2$.

h) Z-Ile-His-$NH_2$: 3,0 g Z-Ile-His-$OCH_3$ werden in 120 ml einer 5 N methanolischen Ammoniak-Lösung gelöst und 16 Std. lang bei Raumtemperatur gerührt. Der gebildete, farblose Niederschlag wird abfiltriert und im Hochvakuum getrocknet. $R_f$(S5) = 0,58, Smp. 204-205°.

i) H-Leu $\overset{c}{-}$ Val-Ile-His-$NH_2$: 1,352 g Z-Leu $\overset{cx}{-}$ Val-Ile-His-$NH_2$ werden 1 Std. lang in 25 ml Methanol/Wasser (95:5) bei Raumtemperatur in Gegenwart von 150 mg Palladium-Kohle (5%) unter Normaldruck hydriert. Nach Abfiltrieren des Katalysators wird das Lösungsmittel abgedampft und der Rückstand in 30 ml Methanol/Wasser (1:1) während 30 Min. bei Raumtemperatur gerührt. Nach Abdampfen des Lösungsmittels und Trocknen im Hochvakuum erhält man H-Leu $\overset{c}{-}$ Val-Ile-His-$NH_2$ als farbloses Pulver. $R_f$(B4) = 0,1; $R_f$(B11) = 0,42.

Beispiel 2: H-Leu $\overset{c}{-}$ Val-7-tert-butoxycarbonyl-heptylamid

a) H-Leu $\overset{c}{-}$ Val-7-tert-butoxycarbonyl-heptylamid wird durch Hydrierung von 213 mg Z-Leu $\overset{cx}{-}$ Val-7-tert-butoxycarbonyl-heptylamid in Gegenwart von 40 mg Palladium-Kohle (10 %) analog Beispiel 1i erhalten. $R_f$ (B2) = 0,13.

b) Z-Leu $\overset{cx}{-}$ Val-7-tert-butoxycarbonyl-heptylamid wird analog Beispiel 1 ausgehend von 162 mg Z-Leu $\overset{cx}{-}$ Val-OH und 103 mg 8-Amino-octansäure-tert-butylester nach Zugabe von 61 mg HOBt, 53 µl 4-Methylmorpholin und 107 mg DCCI erhalten. $R_f$(N4) = 0,20.

c) 8-Amino-octansäure-tert-butylester wird durch Hydrierung von 1,5 g 8-Benzyloxycarbonylamino-octansäure-tert-butylester in 15 ml Methanol nach Zugabe von 0,10 g Palladium-Kohle (10 %) während 4 Std. bei Normaldruck, Abfiltrieren des Katalysators, Abdampfen des Lösungsmittels und Trocknen im Hochvakuum als farbloses Oel erhalten. $R_f$(B3) = 0,08.

d) 8-Benzyloxycarbonylamino-octansäure-tert-butylester: In einem Autoklaven werden 2 g 8-Benzyloxycarbonylamino-octansäure in 12 ml Dioxan in Gegenwart von 1,2 ml Schwefelsäure mit 7,5 g Isobutylen umgesetzt und 48 Stunden bei Raumtemperatur stehengelassen. Die Reaktionsmischung wird mit Eis und 40 ml 1,8 N wässriger Ammoniaklösung versetzt. Das Reaktionsgemisch wird mit Aether extrahiert. Die organische Phase wird mit Wasser und gesättigter, wässriger Kochsalzlösung gewaschen, über Natriumsulfat getrocknet und im Vakuum eingeengt. Man erhält die Titelverbindung als gelbes, klares Oel. $R_f$(N8) = 0,44.

e) 8-Benzyloxycarbonylamino-octansäure: Zu 7 g 8-Amino-octansäure werden 25 ml 2N NaOH-Lösung gegeben. Anschliessend gibt man gleichzeitig bei ca. 0°-5° 30 Min. lang 17,1 g Chlorameisensäurebenzylester (50 % in Toluol) und 12,5 ml 4N NaOH-Lösung zu. Man beobachtet einen voluminösen weissen Niederschlag. Das Reaktionsgemisch wird mit 150 ml Aether und 60 ml Wasser versetzt. Die wässrige Phase wird mit Aether extrahiert, mit Eis versetzt und unter Rühren langsam mit verdünnter wässriger HCl-Lösung auf pH 2 gebracht. Die entstandene Suspension wird mit Essigester zweimal extrahiert. Man vereinigt die organischen Phasen, wäscht diese mit Wasser und gesättigter, wässriger Kochsalzlösung, trocknet über Natriumsulfat und dampft im Vakuum ein. Man erhält die Titelverbindung mit einem Schmelzpunkt von 63°-64°, $R_f$(N1) = 0,53.

Beispiel 3: H-Leu $\overset{c}{-}$ Val-Ala-Sta-$OCH_3$

a) H-Leu $\overset{c}{-}$ Val-Ala-Sta-$OCH_3$•HCl wird als farbloses Pulver erhalten durch Hydrierung von 602 mg Z-Leu $\overset{cx}{-}$ Val-Ala-Sta-$OCH_3$ mit 65 mg Palladium-Kohle 5 % bei pH 5,0 analog Beispiel 2c. $R_f$(B11) = 0,5.

b) Z-Leu $\overset{cx}{-}$ Val-Ala-Sta-$OCH_3$ wird als farbloses Pulver erhalten analog Beispiel 1 ausgehend von

677 mg Z-Leu $\overset{cx}{-}$ Val-OH (Beispiel 1e), 546 mg H-Ala-Sta-$OCH_3$•HCl, 0,31 ml Aethyldiisopropylamin, 256 mg HOBt und 483 mg DCCI nach Mitteldruckchromatographie (245 g Lichroprep® Si60, 25-40 µm, System Methylenchlorid/Aether 2:1). $R_f$ (Essigester/Hexan 3:1) = 0,32.

c) H-Ala-Sta-$OCH_3$•HCl wird als farbloses Pulver erhalten durch Hydrierung von 750 mg Z-Ala-Sta-$OCH_3$ mit 80 mg Palladium-Kohle (10 %) bei pH 5,0 analog Beispiel 2c. $R_f$(B4) = 0,25.

d) Z-Ala-Sta-$OCH_3$ wird als farbloses Pulver erhalten analog Beispiel 1 ausgehend von 1,09 g Z-Ala-OH, 1,00 g Statin-methylester-hydrochlorid (hergestellt wie beschrieben in der Europäischen Patentanmeldung 111'266), 678 mg HOBt, 0,49 ml Aethyldiisopropylamin und 1,28 g DCCI nach Flash-Chromatographie (330 g Kieselgel 60, 40-63 µm, System Essigester/Hexan 3:2). $R_f$ (Essigester/Hexan 2:1) = 0,24.

Beispiel 4: H-Leu $\overset{c}{-}$ Val-4-[tris-(tert-butyldimethylsilyloxymethyl)-methylaminocarbonyl]-butylamid.

a) H-Leu $\overset{c}{-}$ Val-4-[tris-(tert-butyldimethylsilyloxymethyl)-methylaminocarbonyl]-butylamid wird erhalten durch Hydrierung von 900 mg Z-Leu $\overset{cx}{-}$ Val-4-[tris-(tert-butyldimethylsilyloxymethyl)-methylaminocarbonyl]-butylamid mit 90 mg Palladium-Kohle 5 % analog Beispiel 1i und anschliessender Mitteldruckchromatographie (60 g Lichroprep® Si60, 40-60 µm, System B7). Farbloses Oel. $R_f$(B7) = 0.24.

b) Z-Leu $\overset{cx}{-}$ Val-4-[tris-(tert-butyldimethylsilyloxymethyl)-methylaminocarbonyl]-butylamid wird erhalten analog Beispiel 1 ausgehend von 500 mg Z-Leu $\overset{cx}{-}$ Val-OH, 694 mg 5-Aminovaleriansäure-tris-(tert-butyldimethylsilyloxymethyl)-methylamid, 0,21 ml Diisopropyläthylamin, 189 mg HOBt und 331 mg DCCI nach Mitteldruckchromatographie (60 g Lichroprep® Si60, 40-60 µm, System N4) als farbloses Oel. $R_f$(N3) = 0,57.

c) 5-Aminovaleriansäure-tris-(tert-butyldimethylsilyloxymethyl)methylamid wird erhalten durch Hydrierung von 4,03 g der entsprechenden 5-Benzyloxycarbonylamino-Verbindung mit 0.4 g Palladium-Kohle 5 % analog Beispiel 2c und Mitteldruckchromatographie (60 g Lichroprep® Si60, 40-60 µm, System N12). Farbloses Oel, $R_f$(B7) = 0,28.

d) 5-Benzyloxycarbonylaminovaleriansäure-tris-(tert-butyldimethylsilyloxymethyl)-methylamid wird erhalten analog Beispiel 1 ausgehend von 291 mg 5-Benzyloxycarbonylaminovaleriansäure, 500 mg Tris-(tert-butyldimethylsilyloxymethyl)-methylamin, 182 mg HOBt, 0,2 ml Aethyldiisopropylamin und 189 mg DCCI nach Mitteldruckchromatographie (60 g Lichroprep® Si60, 40-60 µm, System N4) als farbloses Oel. $R_f$(N4) = 0,56.

e) Tris-(tert-butyldimethylsilyloxymethyl)-methylamin wird erhalten durch Hydrierung von 399 mg des entsprechenden Benzyloxycarbonyl-Derivates mit 50 mg Palladium-Kohle 5 % als farbloses Oel. $R_f$(N4) = 0,41.

f) Benzyloxycarbonyl-tris-(tert-butyldimethylsilyloxymethyl)-methylamid wird hergestellt aus 250 mg Benzyloxycarbonyl-tris-(hydroxymethyl)-methylamid, 234 mg Imidazol und 472 mg tert-Butyldimethylsilylchlorid in 10 ml DMF während 15 Stunden bei Raumtemperatur.

g) Benzyloxycarbonyl-tris-(hydroxymethyl)-methylamid wird hergestellt aus 20 g Tris-(hydroxymethyl)-methylamin, 31.3 g Chlorameisensäurebenzylester und 42,4 ml Triäthylamin in 200 ml DMF bei 0°. Nach Abdampfen des Lösungsmittels wird in Essigester aufgenommen und mit 2N HCl und gesättigter Natriumchlorid-Lösung gewaschen, die organische Phase getrocknet und das Lösungsmittel abgedampft. $R_f$(N8) = 0.27.

Beispiel 5: H-Leu $\overset{c}{-}$ Val-2-methoxycarbonyl-1-methyl-äthylamid

a) H-Leu $\overset{cx}{-}$ Val-2-methoxycarbonyl-1-methyl-äthylamid wird durch Hydrierung von 187 mg Z-Leu $\overset{cx}{-}$ Val-2-methoxycarbonyl-1-methyl-äthylamid in Gegenwart von 30 mg Palladium-Kohle 10 % analog Beispiel 1i erhalten und als Rohprodukt weiterverarbeitet. $R_f$(B2) = 0,22.

b) Z-Leu $\overset{cx}{-}$ Val-2-methoxycarbonyl-1-methyl-äthylamid wird analog Beispiel 1 ausgehend von 162 mg Z-Leu $\overset{cx}{-}$ Val-OH (Beispiel 1e), 59 mg DL-3-Aminobuttersäuremethylester, 61 mg HOBt, 53 µl 4-Methylmorpholin und 107 mg DCCI nach Flash-Chromatographie mit System N10 erhalten. $R_f$(N10) = 0.64.

Beispiel 6: H-Leu $\overset{c}{-}$ Val-4-tert-butoxycarbonyl-butylamid

a) H-Leu $\overset{c}{-}$ Val-4-tert-butoxycarbonyl-butylamid wird durch Hydrierung von 185 mg Z-Leu $\overset{cx}{-}$ Val-4-tert-butoxycarbonyl-butylamid in Gegenwart von 30 mg Palladium-Kohle (10 %) analog Beispiel 2c erhalten und als Rohprodukt weiterverarbeitet. $R_f$(B2) = 0,17.

b) Z-Leu $\overset{cx}{-}$ Val-4-tert-butoxycarbonyl-butylamid wird analog Beispiel 1 ausgehend von 101 mg Z-Leu $\overset{cx}{-}$ Val-OH, 52 mg 5-Amino-valeriansäure-tert-butylester, 38 mg HOBt, 33 µl N-Methylmorpholin und

68 mg DCCI nach Flash-Chromatographie mit System N4 erhalten. $R_f$(N4) = 0,20.

c) 5-Amino-valeriansäure-tert-butylester wird aus 5-Aminovaleriansäure über 5-Benzyloxycarbonylamino-valeriansäure und 5-Benzyloxycarbonylamino-valeriansäure-tert-butylester analog der in den Beispielen 2c, 2d und 2e beschriebenen Art und Weise hergestellt.

### Beispiel 7: H-Leu $\underline{c}$ Val-$NH_2$

a) H-Leu $\underline{c}$ Val-$NH_2$ wird durch Hydrierung von 73 mg Z-Leu $\underline{cx}$ Val-$NH_2$ in Gegenwart von 15 mg Palladium-Kohle (10 %) analog Beispiel 2c erhalten und als Rohprodukt weiterverarbeitet. $R_f$(B2) = 0,04; $R_f$(S10) = 0,18.

b) Z-Leu $\underline{cx}$ Val-$NH_2$: Eine Mischung aus 93 mg Z-Leu $\underline{cx}$ Val-OH (Beispiel 1e), 2 ml Methylenchlorid, 39 µl Aethyldiisopropylamin und 62 mg Phosphorsäure-phenylester-anilid-chlorid wird 30 Min. gerührt und bei Raumtemperatur mit 0,2 ml Ammoniaklösung 8N (in Methanol) versetzt. Nach weiteren 30 Min. filtriert man den Niederschlag ab. Das Filtrat wird eingeengt und der Rückstand mittels Flash-Chromatographie (30 g Kieselgel 60, 40-63 µm, ca. 0,4 bar, Fliessmittel N3) gereinigt. Die produkthaltigen Fraktionen werden vereinigt und eingeengt und die Titelverbindung als farbloses Oel isoliert. $R_f$(N3) = 0,20.

### Beispiel 8: H-Leu $\underline{c}$ Val-methylamid

a) H-Leu $\underline{c}$ Val-methylamid wird durch Hydrierung von 48 mg Z-Leu $\underline{cx}$ Val-methylamid in Gegenwart von 10 mg Palladium-Kohle (10%) analog Beispiel 2c erhalten und als Rohprodukt weiterverarbeitet. $R_f$(B2) = 0,08; $R_f$(S10) = 0,27.

b) Z-Leu $\underline{cx}$ Val-methylamid: Eine Mischung aus 100 mg Z-Leu $\underline{cx}$ Val-OH (Beispiel 1e), 2 ml DMF, 49 mg HOBt und 66 mg DCCI wird 24 Std. bei 0° stehen gelassen. Die Mischung wird mit einem Ueberschuss an Methylamin vesetzt und 2 Std. bei 0° und 2 Std. bei Raumtemperatur gerührt. Die Titelverbindung wird nach Flash-Chromatographie mit System N3 als farbloses Oel erhalten. $R_f$(N7) = 0,55.

### Beispiel 9: H-Leu $\underline{c}$ Val-benzylamid

a) H-Leu $\underline{c}$ Val-benzylamid wird durch Hydrierung von 100 mg Z-Leu $\underline{cx}$ Val-benzylamid in Gegenwart von 20 mg Palladium-Kohle (10 %) analog Beispiel 2c erhalten und als Rohprodukt weiterverarbeitet. $R_f$(B2) = 0,20; $R_f$(S10) = 0,37.

b) Z-Leu $\underline{cx}$ Val-benzylamid: Eine Mischung aus 150 mg Z-Leu $\underline{cx}$ Val-OH (Beispiel 1e), 3 ml DMF, 74 mg HOBt und 99 mg DCCI wird 24 Std. bei 0° stehengelassen. Die Mischung wird mit 55 µl Benzylamin versetzt und 6 Std. bei 0° und 24 Std. bei Raumtemperatur gerührt. Die Titelverbindung wird nach Flash-Chromatographie mit Essigester-Hexan (1:2) als farbloses Oel erhalten. $R_f$(N7) = 0,56.

### Beispiel 10: H-Leu $\underline{c}$ Val-dimethylamid

a) H-Leu $\underline{c}$ Val-dimethylamid wird durch Hydrierung von 160 mg Z-Leu $\underline{cx}$ Val-dimethylamid in Gegenwart von 30 mg Palladium-Kohle (10 %) analog Beispiel 2c erhalten und als Rohprodukt weiterverarbeitet. $R_f$(B2) = 0,15; $R_f$(S10) = 0,37.

b) Z-Leu $\underline{cx}$ Val-dimethylamid wird analog Beispiel 9b aus 100 mg Z-Leu $\underline{cx}$ Val-OH, 2 ml DMF, 49 mg HOBt und 66 mg DCCI gefolgt von einem Ueberschuss an Dimethylamin nach Flash-Chromatographie mit Essigester-Hexan (1:2) als farbloses Oel erhalten. $R_f$(N7) = 0,35.

### Beispiel 11: H-Leu $\underline{c}$ Val-3-(m-carbamoylphenyloxy)-2-hydroxy-propylamid

a) H-Leu $\underline{c}$ Val-3-(m-carbamoylphenyloxy)-2-hydroxy-propylamid: 80 mg Z-Leu $\underline{cx}$ Val-3-(m-carbamoylphenyloxy)-2-hydroxy-propylamid werden in 8 ml Methanol-Wasser 9:1 gelöst und nach Zugabe von 20 mg Palladium-Kohle (10 %) unter $CO_2$- Absorption bis zur Sättigung hydriert. Nach 24 Std. bei Raumtemperatur wird der Katalysator abfiltriert, das Filtrat eingeengt und am Hochvakuum getrocknet. Die Titelverbindung wird durch Flash-Chromatographie (65 g Kieselgel 60, 40-63 µm, System B11) gereinigt. $R_f$(B11) = 0,28.

b) Z-Leu $\underline{cx}$ Val-3-(m-carbamoylphenyloxy)-2-hydroxy-propylamid wird analog Beispiel 1 ausgehend von 156 mg Z-Leu $\underline{cx}$ Val-OH, 89 mg m-(3-Amino-2-hydroxy-propyloxy)-benzoesäureamid, 71 mg HOBt und 111 mg DCCI nach Flash-Chromatographie im System N11 erhalten. $R_f$(N11) = 0,34; $R_f$(N12) = 0,59.

c) m-(3-Amino-2-hydroxy-propyloxy)-benzoesäureamid: 15 g m-(2,3-Epoxy-propyloxy)-benzoesäureamid werden gelöst in 150 ml Methanol langsam zu 155 ml Ammoniak conc. zugegeben. Anschliessend wird noch während 2 Std. auf 40° erwärmt, am Rotationsverdampfer zur Trockene eingeengt und das Produkt mittels Flash-Chromatographie im System B11 gereinigt. $R_f$(B11) = 0,14.

d) m-(2,3-Epoxy-propyloxy)-benzoesäureamid: 16,2 g m-Hydroxy-benzoesäureamid und 32,4 g Pottasche werden in 120 ml Epichlorhydrin während 3 Std. am Rückfluss gekocht. Das Reaktionsgemisch wird anschliessend auf Eis gegossen, das Produkt mit Essigester extrahiert und mittels Flash-Chromatographie im System N10 gereinigt. $R_f$(N10) = 0,28.

Beispiel 12: H-Leu $\overset{c}{-}$ Val-3-(2-pyrrolidinon-1-yl)-propylamid

a) H-Leu $\overset{c}{-}$ Val-3-(2-pyrrolidinon-1-yl)-propylamid wird durch Hydrierung von 210 mg Z-Leu $\overset{cx}{-}$ Val-3-(2-pyrrolidinon-1-yl)-propylamid in Gegenwart von 30 mg Palladium-Kohle (10 %) analog Beispiel 11a erhalten und durch Flash-Chromatographie mit System B7 gereinigt. $R_f$(B7) = 0,20; $R_f$(B11) = 0,47.

b) Z-Leu $\overset{cx}{-}$ Val-3-(2-pyrrolidinon-1-yl)-propylamid wird analog Beispiel 1 ausgehend von 150 mg Z-Leu $\overset{cx}{-}$ Val-OH, 64 mg N-(3-Aminopropyl)-2-pyrrolidinon, 68 mg HOBt und 107 mg DCCI erhalten und durch Flash-Chromatographie mit System N10 gereinigt. $R_f$(N10) = 0,20; $R_f$(N11) = 0,55.

Beispiel 13: H-Leu $\overset{c}{-}$ Val-3-(N-morpholino)-propylamid

a) H-Leu $\overset{c}{-}$ Val-3-(N-morpholino)-propylamid wird durch Hydrierung von 210 mg Z-Leu $\overset{cx}{-}$ Val-3-(N-morpholino)-propylamid in Gegenwart von 30 mg Palladium-Kohle (10 %) analog Beispiel 11a erhalten und durch Flash-Chromatographie mit System B11 gereinigt. $R_f$(B11) = 0,35; $R_f$ (B7) = 0,11.

b) Z-Leu $\overset{cx}{-}$ Val-3-(N-morpholino)-propylamid wird analog Beispiel 1 ausgehend von 150 mg Z-Leu $\overset{cx}{-}$ Val-OH, 80 mg 4-(3-Aminopropyl)-morpholin, 68 mg HOBt und 115 mg DCCI erhalten und durch Flash-Chromatographie mit System N11 gereinigt. $R_f$(N11) = 0,35; $R_f$(B7) = 0,53.

Beispiel 14: H-Leu $\overset{c}{-}$ Val-2-(2-hydroxyäthylamino)-äthylamid

a) H-Leu $\overset{c}{-}$ Val-2-(2-hydroxyäthylamino)-äthylamid wird durch Hydrierung von 110 mg Z-Leu Val-2-(2-hydroxyäthylamino)-äthylamid in Gegenwart von 20 mg Palladium-Kohle (10 %) analog Beispiel 11a erhalten und durch Flash-Chromatographie mit System S10 gereinigt. $R_f$(S10) = 0,25; $R_f$(B11) = 0,12.

b) Z-Leu $\overset{cx}{-}$ Val-2-(2-hydroxyäthylamino)-äthylamid wird analog Beispiel 1 ausgehend von 150 mg Z-Leu $\overset{cx}{-}$ Val-OH, 130 mg N-(2-Hydroxyäthyl)-äthylendiamin, 68 mg HOBt und 114 mg DCCI erhalten und durch Flash-Chromatographie mit System B7 gereinigt. $R_f$(B7) = 0,24; $R_f$(B11) = 0,67

Beispiel 15: H-Leu $\overset{c}{-}$ Val-2-(3-carbamoyl-4-hydroxy-phenyloxy)-äthylamid

a) H-Leu $\overset{c}{-}$ Val-2-(3-carbamoyl-4-hydroxy-phenyloxy)-äthylamid wird durch Hydrierung von 240 mg Z-Leu $\overset{cx}{-}$ Val-2-(3-carbamoyl-4-hydroxy-phenyloxy)-äthylamid in Gegenwart von 40 mg Palladium-Kohle (10 %) analog Beispiel 11a erhalten und durch Flash-Chromatographie mit System B9 gereinigt. $R_f$(B9) = 0,30; $R_f$(B11) = 0,40.

b) Z-Leu $\overset{cx}{-}$ Val-2-(3-carbamoyl-4-hydroxy-phenyloxy)-äthylamid wird analog Beispiel 1 ausgehend von 147 mg Z-Leu $\overset{cx}{-}$ Val-OH, 107 mg 5-(1-Aminoäthyloxy)-2-hydroxy-benzoesäureamid (hergestellt nach J. Med. Chem. 27, 831 (1984)), 61 mg HOBt und 97 mg DCCI erhalten und durch Flash-Chromatographie mit System B7 gereinigt. $R_f$(B7) = 0,41; $R_f$(N7) = 0,20.

Beispiel 16: H-Leu $\overset{c}{-}$ Val-Ala-2-(4-imidazolyl)-äthylamid

a) H-Leu $\overset{c}{-}$ Val-Ala-2-(4-imidazolyl)-äthylamid wird durch Hydrierung von 240 mg Z-Leu $\overset{cx}{-}$ Val-Ala-2-(4-imidazolyl)-äthylamid in Gegenwart von 40 mg Palladium-Kohle (10 %) analog Beispiel 11a erhalten und durch Flash-Chromatographie mit System B11 gereinigt. $R_f$(B11) = 0,20; $R_f$(B9) = 0,09.

b) Z-Leu $\overset{cx}{-}$ Val-Ala-2-(4-imidazolyl)-äthylamid wird analog Beispiel 1 ausgehend von 160 mg Z-Leu $\overset{cx}{-}$ Val-OH, 100 mg L-Alanin-2-(4-imidazolyl)-äthylamid, 67 mg HOBt und 106 mg DCCI erhalten und durch Flash-Chromatographie mit System B7 gereinigt. $R_f$(B7) = 0,22; $R_f$(N11) = 0,17.

c) L-Alanin-2-(4-imidazolyl)-äthylamid wird durch Hydrierung von 300 mg Z-Ala-2-(4-imidazolyl)-äthylamid in Gegenwart von 50 mg Palladium-Kohle (10 %) analog Beispiel 11a erhalten und durch Flash-Chromatographie mit System B11 gereinigt. $R_f$(B11) = 0,17.

d) Z-Ala-2-(4-imidazolyl)-äthylamid wird analog Beispiel 1 ausgehend von 940 mg Z-Ala-OH, 853 mg Histamin-dihydrochlorid, 1,6 ml Aethyldiisopropylamin, 709 mg HOBt und 1,13 g DCCI erhalten und durch Flash-Chromatographie (200 g Kieselgel 60, 40-63 µm, Fliessmitel-System B7) gereinigt. $R_f$(B7) = 0,26; $R_f$-(N11) = 0,15.

Beispiel 17: H-Leu $\underline{c}$ Val-Alá-2-(2-pyridyl)-äthylamid

a) H-Leu $\underline{c}$ Val-Ala-2-(2-pyridyl)-äthylamid wird durch Hydrierung von 320 mg Z-Leu $\underline{cx}$ Val-Ala-2-(pyridyl)-äthylamid in Gegenwart von 50 mg Palladium-Kohle (10 %) analog Beispiel 11a erhalten und durch Flash-Chromatographie mit System B9 gereinigt. $R_f$(B9) = 0,26.

b) Z-Leu $\underline{cx}$ Val-Ala-2-(2-pyridyl)-äthylamid wird analog Beispiel 1 ausgehend von 200 mg Z-Leu $\underline{cx}$ Val-OH, 143 mg L-Alanin-2-(2-pyridyl)-äthylamid, 91 mg HOBt und 132 mg DCCI erhalten und durch Flash-Chromatographie mit System N10 gereinigt. $R_f$(N10) = 0,32; $R_f$(N11) = 0,46.

c) L-Alanin-2-(2-pyridyl)-äthylamid wird durch Hydrierung von 900 mg Z-Ala-2-(2-pyridyl)-äthylamid in Gegenwart von 100 mg Palladium-Kohle (10 %) analog Beispiel 11a erhalten und durch Flash-Chromatographie mit System B7 gereinigt. $R_f$(B7) = 0,20.

d) Z-Ala-2-(2-pyridyl)-äthylamid wird analog Beispiel 1 ausgehend von 635 mg Z-Ala-OH, 400 mg 2-(2-Aminoäthyl)-pyridin, 436 mg HOBt und 762 mg DCCI erhalten und durch Flash-Chromatographie (130 g Kieselgel, System N11) gereinigt. $R_f$(N11) = 0,33.

Beispiel 18: H-Leu $\underline{c}$ Val-Ala-2-(2-hydroxyäthylamino)-äthylamid

a) H-Leu $\underline{c}$ Val-Ala-2-(2-hydroxyäthylamino)-äthylamid wird durch Hydrierung von 89 mg Z-Leu $\underline{cx}$ Val-Ala-2-(2-hydroxyäthylamino)-äthylamid in Gegenwart von 20 mg Palladium-Kohle (10 %) analog Beispiel 11a erhalten und ohne Reinigung weiterverarbeitet. $R_f$(B11) = 0,08; $R_f$(S10) = 0,10.

b) Z-Leu $\underline{cx}$ Val-Ala-2-(2-hydroxyäthylamino)-äthylamid wird analog Beispiel 1 ausgehend von 100 mg Z-Leu $\underline{cx}$ Val-OH, 150 mg L-Alanin-2-(2-hydroxyäthylamino)-äthylamid, 45 mg HOBt und 76 mg DCCI erhalten und durch Flash-Chromatographie mit System B9 gereinigt. $R_f$(B9) = 0,33; $R_f$(B11) = 0,53.

c) L-Alanin-2-(2-hydroxyäthylamino)-äthylamid wird durch Hydrierung von 540 mg Z-Ala-2-(2-hydroxyäthylamino)-äthylamid in Gegenwart von 70 mg Palladium-Kohle (10 %) analog Beispiel 11a erhalten und ohne Reinigung direkt weiterverwendet. $R_f$(B14) = 0,17.

d) Z-Ala-2-(2-hydroxyäthylamino)-äthylamid wird analog Beispiel 1 ausgehend von 571 mg Z-Ala-OH, 400 mg N-(2-Hydroxyäthyl)-äthylendiamin, 470 mg HOBt und 788 mg DCCI erhalten und durch Flash-Chromatographie (160 g Kieselgel, System B11) gereinigt. $R_f$(B11) = 0,23.

Beispiel 19: H-Leu $\underline{c}$ Val-Ala-1(S)-benzyl-2-hydroxy-äthylamid

a) H-Leu $\underline{c}$ Val-Ala-1(S)-benzyl-2-hydroxy äthylamid wird durch Hydrierung von 210 mg Z-Leu $\underline{cx}$ Val-Ala-1(S)-benzyl-2-hydroxy-äthylamid in Gegenwart von 20 mg Palladium-Kohle (10 %) analog Beispiel 11a erhalten und durch Flash-Chromatographie mit System B7 gereinigt. $R_f$(B7) = 0,16.

b) Z-Leu $\underline{cx}$ Val-Ala-1(S)-benzyl-2-hydroxy-äthylamid wird analog Beispiel 1 ausgehend von 200 mg Z-Leu $\underline{cx}$ Val-OH, 160 mg L-Alanin-1(S)-benzyl-2-hydroxy-äthylamid, 110 mg HOBt und 160 mg DCCI erhalten und durch Flash-Chromatographie mit System N10 gereinigt. $R_f$(N10) = 0,27.

c) L-Alanin-1(S)-benzyl-2-hydroxy-äthylamid wird durch Hydrierung von 1,3 g Z-Ala-1(S)-benzyl-2-hydroxy-äthylamid in Gegenwart von 130 mg Palladium-Kohle (10 %) analog Beispiel 11a erhalten und durch Flash-Chromatographie mit System B7 gereinigt. $R_f$(B7) = 0,28.

d) Z-Ala-1(S)-benzyl-2-hydroxy-äthylamid wird analog Beispiel 1 ausgehend von 1,0 g Z-Ala-OH, 680 mg 2(S)-Amino-3-phenylpropanol, 750 mg HOBt und 1,2 g DCCI erhalten und durch Flash-Chromatographie (220 g Kieselgel, System N11) gereinigt. $R_f$(N11) = 0,44.

Beispiel 20: H-Leu $\underline{c}$ Val-Ala-1(S)-benzyl-3-cyano-2(R,S)-hydroxy-propylamid

a) H-Leu $\underline{c}$ Val-Ala-1(S)-benzyl-3-cyano-2(R,S)hydroxy-propylamid wird durch Hydrierung von 350 mg Z-Leu $\underline{cx}$ Val-Ala-1(S)-benzyl-3-cyano-2(R,S)-hydroxy-propylamid in Gegenwart von 50 mg Palladium-Kohle (10 %) analog Beispiel 11a erhalten und durch Flash-Chromatographie mit System B11 gereinigt. $R_f$(B11) = 0,46; $R_f$(B7) = 0,16.

b) Z-Leu $\underline{cx}$ Val-Ala-1(S)-benzyl-3-cyano-2(R,S)-hydroxy-propylamid wird analog Beispiel 1 ausgehend von 240 mg Z-Leu $\underline{cx}$ Val-OH, 232 mg L-Alanin-1(S)-benzyl-3-cyano-2(R,S)-hydroxy-propylamid,

100 mg HOBt und 159 mg DCCI erhalten und durch Flash-Chromatographie mit System N7 gereinigt. $R_f$-(N7) = 0,28.

c) L-Alanin-1(S)-benzyl-3-cyano-2(R,S)-hydroxy-propylamid wird durch Hydrierung von 350 mg Z-Ala-1(S)-benzyl-3-cyano-2(R,S)-hydroxy-propylamid in Gegenwart von 50 mg Palladium-Kohle (10 %) analog Beispiel 11a erhalten und durch Flash-Chromatographie mit System B7 gereinigt. $R_f$(B7) = 0,27.

d) Z-Ala-1(S)-benzyl-3-cyano-2(R,S)-hydroxy-propylamid wird analog Beispiel 1 ausgehend von 394 mg Z-Ala-OH, 305 mg 4(S)-Amino-3(R,S)-hydroxy-5-phenyl-valeriansäurenitril, 270 mg HOBt und 430 mg DCCI erhalten und durch Flash-Chromatographie (130 g Kieselgel, System N11) gereinigt. $R_f$(N11) = 0,42; $R_f$(N7) = 0,20.

e) 4(S)-Amino-3(R,S)-hydroxy-5-phenyl-valeriansäurenitril: 840 mg N-(1(S)-Benzyl-3-cyano-2(R,S)-hydroxy-propyl)-phthalimid werden in 20 ml Dichlormethan/Methanol 3:1 gelöst, mit 1 ml Hydrazin-Hydrat versetzt und 20 Std. bei Raumtemperatur gerührt. Es wird vom ausgefallenen Hydrazid befreit und die Titelverbindung durch Flash-Chromatographie mit System B7 gereinigt. $R_f$(B7) = 0,35.

f) N-(1(S)-Benzyl-3-cyano-2(R,S)-hydroxy-propyl)-phthalimid: 1,5 g N-(1(S)-Benzyl-3-cyano-2-keto-propyl)-phthalimid werden in 20 ml Dichlormethan und 2 ml Methanol gelöst, auf -14° gekühlt und innerhalb von 10 Min. mit 90 mg Natriumborhydrid versetzt. Nach 20 Min. wird das Reaktionsgemisch auf eiskalte 0,1N $H_2SO_4$ gegossen, die Titelverbindung mit Dichlormethan extrahiert und mittels Flash-Chromatographie (System N6) gereinigt. Die Titelverbindung besteht aus einem Diastereomerengemisch im Verhältnis 1:1. $R_f$(N6) = 0,41.

g) N-(1(S)-Benzyl-3-cyano-2-keto-propyl)-phthalimid: 2,2 g Phthaloyl-L-phenylalanin (hergestellt nach Houben-Weyl, Band 15/1, S. 252), 0,64 ml Cyanessigsäure-tert-butylester, 1,1 ml Diäthylphosphorcyanidat (J. Org. Chem. 43, 3631 (1978)) und 3,3 ml Aethyl-diisopropylamin werden in 40 ml DMF während 20 Std. bei 0°C intensiv gerührt. Die Reaktionslösung wird eingeengt, das resultierende gelbe Oel in 100 ml Methylenchlorid gelöst und zweimal mit 0,1 N $H_2SO_4$ gewaschen. Nach erneutem Einengen wird das Oel in 40 ml Trifluoressigsäure bei Raumtemperatur gelöst und nach 2 Std. am Rotationsverdampfer eingeengt. Das resultierende Rohprodukt wird in Methylenchlorid gelöst, einmal mit Eiswasser gewaschen und mittels Flash-Chromatographie im System Methylenchlorid-Aether 9:1 gereinigt. $R_f$ (Methylenchlorid-Aether 9:1) = 0,43.

Beispiel 21: H-Leu $\overset{c}{-}$ Val-Ile-2-(2-pyridyl)-äthylamid

a) H-Leu $\overset{c}{-}$ Val-Ile-2-(2-pyridyl)-äthylamid wird durch Hydrierung von 260 mg Z-Leu $\overset{cx}{-}$ Val-Ile-2-(2-pyridyl)-äthylamid in Gegenwart von 40 mg Palladium-Kohle (10 %) analog Beispiel 11a erhalten und durch Flash-Chromatographie mit System B7 gereinigt. $R_f$(B7) = 0,23.

b) Z-Leu $\overset{cx}{-}$ Val-Ile-2-(2-pyridyl)-äthylamid wird analog Beispiel 1 ausgehend von 189 mg Z-Leu $\overset{cx}{-}$ Val-OH, 180 mg L-Isoleucin-2-(2-pyridyl)-äthylamid, 93 mg HOBt und 144 mg DCCI erhalten und durch Flash-Chromatographie mit System N11 gereinigt. $R_f$(N11) = 0,45.

c) L-Isoleucin-2-(2-pyridyl)-äthylamid wird durch Hydrierung von 440 mg Z-Ile-2-(2-pyridyl)-äthylamid in Gegenwart von 50 mg Palladium-Kohle (10 %) analog Beispiel 11a erhalten und durch Flash-Chromatographie mit System B11 gereinigt. $R_f$(B11) = 0,65; $R_f$(N9) = 0,22.

d) Z-Ile-2-(2-pyridyl)-äthylamid wird analog Beispiel 1 ausgehend von 4,9 g Z-Ile-OH-Dicyclohexylaminsalz, 1,75 g 2-(2-Aminoäthyl)-pyridin, 1,69 g HOBt und 2,95 g DCCI erhalten und durch Flash-Chromatographie (250 g Kieselgel, System N11) gereinigt. $R_f$(N11) = 0,46.

Beispiel 22: H-Leu $\overset{c}{-}$ Val-Ile-2-pyridyl-methylamid

a) H-Leu $\overset{c}{-}$ Val-Ile-2-pyridyl-methylamid wird durch Hydrierung von 340 mg Z-Leu $\overset{cx}{-}$ Val-Ile-2-pyridyl-methylamid in Gegenwart von 50 mg Palladium-Kohle (10%) analog Beispiel 11a erhalten und durch Flash-Chromatographie mit System B9 gereinigt. $R_f$(B9) = 0,40.

b) Z-Leu $\overset{cx}{-}$ Val-Ile-2-pyridyl-methylamid wird analog Beispiel 1 ausgehend von 220 mg Z-Leu $\overset{cx}{-}$ Val-OH, 200 mg L-Isoleucin-2-pyridyl-methylamid, 91 mg HOBt und 146 mg DCCI erhalten und durch Flash-Chromatographie mit System N10 gereinigt. $R_f$(N10) = 0,23.

c) L-Isoleucin-2-pyridyl-methylamid wird durch Hydrierung von 500 mg Z-Ile-2-pyridyl-methylamid in Gegenwart von 50 mg Palladium-Kohle (10 %) analog Beispiel 11a erhalten und durch Flash-Chromatographie mit System B7 gereinigt. $R_f$(B7) = 0,26; $R_f$(B11) = 0,65.

d) Z-Ile-2-pyridyl-methylamid wird analog Beispiel 1 ausgehend von 894 mg Z-Ile-OH-Dicyclohexylaminsalz, 265 mg 2-Aminomethylpyridin, 337 mg HOBt und 536 mg DCCI erhalten und durch Flash-Chromatographie (100 g Kieselgel, System N7) gereinigt. $R_f$(N7) = 0,22; $R_f$(N11) = 0,48.

Beispiel 23: H-Leu $\overset{c}{-}$ Val-Ile-2-(3-indolyl)-äthylamid

a) H-Leu $\overset{c}{-}$ Val-Ile-2-(3-indolyl)-äthylamid wird durch Hydrierung von 210 mg Z-Leu $\overset{cx}{-}$ Val-Ile-2-(3-indolyl)-äthylamid in Gegenwart von 30 mg Palladium-Kohle (10 %) analog Beispiel 11a erhalten und durch Flash-Chromatographie mit System B7 gereinigt. $R_f$(B7) = 0,23.

b) Z-Leu $\overset{cx}{-}$ Val-Ile-2-(3-indolyl)-äthylamid wird analog Beispiel 1 ausgehend von 121 mg Z-Leu $\overset{cx}{-}$ Val-OH, 120 mg L-Isoleucin-2-(3-indolyl)-äthylamid, 68 mg HOBt und 92 mg DCCI erhalten und durch Flash-Chromatographie mit System N10 gereinigt. $R_f$(N10) = 0,22; $R_f$(B5) = 0,53.

c) L-Isoleucin-2-(3-indolyl)-äthylamid wird durch Hydrierung von 800 mg Z-Ile-2-(3-indolyl)-äthylamid in Gegenwart von 100 mg Palladium-Kohle (10 %) analog Beispiel 11a erhalten und durch Flash-Chromatographie mit System B7 gereinigt. $R_f$(B7) = 0,43.

d) Z-Ile-2-(3-indolyl)-äthylamid wird analog Beispiel 1 ausgehend von 894 mg Z-Ile-OH-Dicyclohexylaminsalz, 385 mg Tryptamin, 337 mg HOBt und 536 mg DCCI erhalten und durch Flash-Chromatographie (160 g Kieselgel, System N10) gereinigt. $R_f$(N10) = 0,30; $R_f$(N11) = 0,70.

Beispiel 24: H-Leu $\overset{c}{-}$ Val-Ile-2-(N-morpholino)-äthylamid

a) H-Leu $\overset{c}{-}$ Val-Ile-2-(N-morpholino)-äthylamid wird durch Hydrierung von 160 mg Z-Leu $\overset{cx}{-}$ Val-Ile-2-(N-morpholino)-äthylamid in Gegenwart von 30 mg Palladium-Kohle (10 %) analog Beispiel 11a erhalten und durch Flash-Chromatographie mit System B7 gereinigt. $R_f$(B7) = 0,14.

b) Z-Leu $\overset{cx}{-}$ Val-Ile-2-(N-morpholino)-äthylamid wird analog Beispiel 1 ausgehend von 133 mg Z-Leu $\overset{cx}{-}$ Val-OH, 120 mg L-Isoleucin-2-(N-morpholino)-äthylamid, 76 mg HOBt und 102 mg DCCI erhalten und durch Flash-Chromatographie mit System B5 gereinigt. $R_f$(B5) = 0,36.

c) L-Isoleucin-2-(N-morpholino)-äthylamid wird durch Hydrierung von 1,0 g Z-Ile-2-(N-morpholino)-äthylamid in Gegenwart von 150 mg Palladium-Kohle (10 %) analog Beispiel 11a erhalten und durch Flash-Chromatographie mit System B7 gereinigt. $R_f$(B7) = 0,22.

d) Z-Ile-2-(N-morpholino)-äthylamid wird analog Beispiel 1 ausgehend von 1,34 g Z-Ile-OH-Dicyclohexylaminsalz, 430 mg 4-(2-Aminoäthyl)-morpholin, 500 mg HOBt und 801 mg DCCI erhalten und durch Flash-Chromatographie (200 g Kieselgel, System B7) gereinigt. $R_f$(B7) = 0,50.

Beispiel 25: H-Leu $\overset{c}{-}$ Val-Ile-4-(carbamoylmethyl)-2-thiazolylamid

a) H-Leu — Val-Ile-4-(carbamoylmethyl)-2-thiazolylamid wird durch Hydrierung von 260 mg Z-Leu $\overset{cx}{-}$ Val-Ile-4-(carbamoylmethyl)-2-thiazolylamid in Gegenwart von 100 mg Palladium-Kohle (10 %) analog Beispiel 11a erhalten und durch Flash-Chromatographie mit System B9 gereinigt. $R_f$(B9) = 0,21.

b) Z-Leu $\overset{cx}{-}$ Val-Ile-4-(carbamoylmethyl)-2-thiazolylamid wird analog Beispiel 1 ausgehend von 181 mg Z-Leu $\overset{cx}{-}$ Val-OH, 181 mg L-Isoleucin-4-(carbamoylmethyl)-2-thiazolylamid, 103 mg HOBt und 138 mg DCCI erhalten und durch Flash-Chromatographie mit System B5 gereinigt. $R_f$(B5) = 0,20.

c) L-Isoleucin-4-(carbamoylmethyl)-2-thiazolylamid wird durch Hydrierung von 1,2 g Z-Ile-4-(carbamoylmethyl)-2-thiazolylamid in Gegenwart von 500 mg Palladium-Kohle (10 %) analog Beispiel 11a erhalten und durch Flash-Chromatographie mit System N9 gereinigt. $R_f$(N9) = 0,29.

d) Z-Ile-4-(carbamoylmethyl)-2-thiazolylamid: 300 mg Z-Ile-4-(äthoxycarbonylmethyl)-2-thiazolylamid werden in 20 ml einer 5N Lösung von Ammoniak in Methanol gelöst und 24 Std. bei Raumtemperatur stehen gelassen. Das Reaktionsgemisch wird am Rotationsverdampfer eingeengt und die Titelverbindung durch Flash-Chromatographie mit System N11 erhalten. $R_f$(N11) = 0,33.

e) Z-Ile-4-(äthoxycarbonylmethyl)-2-thiazolylamid wird analog Beispiel 1 ausgehend von 2,23 g Z-Ile-OH-Dicyclohexylaminsalz, 930 mg (2-Amino-4-thiazolyl)-essigsäureäthylester, 842 mg HOBt und 1,34 g DCCI erhalten und durch Flash-Chromatographie (250 g Kieselgel, System N10) gereinigt. $R_f$(N10) = 0,53.

Beispiel 26: H-Leu $\overset{c}{-}$ Val-Ile-(m-carbamoylphenyl)-methylamid

a) H-Leu $\overset{c}{-}$ Val-Ile-(m-carbamoylphenyl)-methylamid wird durch Hydrierung von 230 mg Z-Leu $\overset{cx}{-}$ Val-Ile-(m-carbamoylphenyl)-methylamid in Gegenwart von 40 mg Palladium-Kohle (10 %) analog Beispiel 11a erhalten und durch Flash-Chromatographie mit System B7 gereinigt. $R_f$(B7) = 0,11; $R_f$(B9) = 0,37.

b) Z-Leu $\overset{cx}{-}$ Val-Ile(m-carbamoylphenyl)-methylamid wird analog Beispiel 1 ausgehend von 164 mg Z-Leu $\overset{cx}{-}$ Val-OH, 160 mg L-Isoleucin-(m-carbamoylphenyl)-methylamid 93 mg HOBt und 126 mg DCCI erhalten und durch Flash-Chromatographie mit System N11 gereinigt. $R_f$(N11) = 0,39.

c) L-Isoleucin-(m-carbabmoylphenyl)-methylamid wird durch Hydrierung von 250 mg Z-Ile-(m-carbamoylphenyl)-methylamid in Gegenwart von 40 mg Palladium-Kohle (10 %) analog Beispiel 11a erhalten und durch Flash-Chromatographie mit System B7 gereinigt. $R_f$(B7) = 0,27; $R_f$(B11) = 0,56.

d) Z-Ile-(m-carbamoylphenyl)-methylamid wird analog Beispiel 1 ausgehend von 1.78 g Z-Ile-OH-Dicyclohexylaminsalz, 600 mg 3-Aminomethyl-benzoesäureamid, 673 mg HOBt und 1071 mg DCCI erhalten und durch Flash-Chromatographie (220 g Kieselgel, System N11) gereinigt. $R_f$(N11) = 0,37.

### Beispiel 27: H-Leu $\overset{c}{-}$ Val-Ile-dicarbamoyl-methylamid

a) H-Leu $\overset{c}{-}$ Val-Ile-dicarbamoyl-methylamid wird durch Hydrierung von 150 mg Z-Leu $\overset{cx}{-}$ Val-Ile-dicarbamoyl-methylamid in Gegenwart von 30 mg Palladium-Kohle (10 %) analog Beispiel 11a erhalten und durch Flash-Chromatographie mit System B11 gereinigt. $R_f$(B11) = 0,28.

b) Z-Leu $\overset{cx}{-}$ Val-Ile-dicarbamoyl-methylamid wird analog Beispiel 1 ausgehend von 150 mg Z-Leu $\overset{cx}{-}$ Val-OH, 130 mg L-Isoleucin-dicarbamoyl-methylamid, 85 mg HOBt und 115 mg DCCI erhalten und durch Flash-Chromatographie mit System N11 gereinigt. $R_f$(N11) = 0,25.

c) L-Isoleucin-dicarbamoyl-methylamid wird durch Hydrierung von 560 mg Z-Ile-dicarbamoyl-methylamid in Gegenwart von 100 mg Palladium-Kohle (10 %) analog Beispiel 11a erhalten und durch Flash-Chromatographie mit System B11 gereinigt. $R_f$(B11) = 0,22.

d) Z-Ile-dicarbamoyl-methylamid wird durch Reaktion von 1,00 g Z-Ile-bis(methoxycarbonyl)-methylamid mit 50 ml einer 5N Lösung von Ammoniak in Methanol bei 40° in 2 Std. erhalten und durch Abfiltrieren des Niederschlags und Trocknung am Hochvakuum isoliert. $R_f$(N9) = 0.48.

e) Z-Ile-bis(methoxycarbonyl)-methylamid wird analog Beispiel 1 ausgehend von 5.00 g Z-Ile-OH-Dicyclohexylaminsalz, 2,1 g Aminomalonsäuredimethylester-hydrochlorid, 1,72 g HOBt und 3,00 g DCCI erhalten und durch Flash-Chromatographie (250 g Kieselgel, System N6) gereinigt. $R_f$(N6) = 0.48; $R_f$(N10) = 0,66.

### Beispiel 28: H-Leu $\overset{c}{-}$ Val-Ile-Sta-$NH_2$

a) H-Leu $\overset{c}{-}$ Val-Ile-Sta-$NH_2$ wird durch Hydrierung von 250 mg Z-Leu $\overset{cx}{-}$ Val-Ile-Sta-$NH_2$ in Gegenwart von 50 mg Palladium-Kohle (10 %) analog Beispiel 11a erhalten und durch Flash-Chromatographie mit System B7 gereinigt. $R_f$(B7) = 0,29.

b) Z-Leu $\overset{cx}{-}$ Val-Ile-Sta-$NH_2$ wird analog Beispiel 1 ausgehend von 160 mg Z-Leu $\overset{cx}{-}$ Val-OH, 136 mg H-Ile-Sta-$NH_2$, 67 mg HOBt und 106 mg DCCI erhalten und durch Flash-Chromatographie mit System N11 gereinigt. $R_f$(N11) = 0.33.

c) H-Ile-Sta-$NH_2$ wird durch Hydrierung von 700 mg Z-Ile-Sta-$NH_2$ in Gegenwart von 100 mg Palladium-Kohle (10 %) analog Beispiel 11a erhalten und durch Flash-Chromatographie mit System B7 gereinigt. $R_f$(B7) = 0.20.

d) Z-Ile-Sta-$NH_2$ wird analog Beispiel 1 ausgehend von 983 mg Z-Ile-OH-Dicyclohexylaminsalz, 348 mg Statinamid, 367 mg HOBt und 536 mg DCCI erhalten und durch Flash-Chromatographie (160 g Kiesgel, System N11) gereinigt. $R_f$(N11) = 0.38.

e) Statinamid wird ausgehend von 900 mg Z-Sta-$NH_2$ durch Hydrierung in Gegenwart von 120 mg Palladium-Kohle (10 %) analog Beispiel 2c erhalten und durch Flash-Chromatographie mit System S9 gereinigt. $R_f$(S9) = 0.23.

f) Z-Sta-$NH_2$ wird durch Ammonolyse von 1,00 g Z-Sta-$OCH_3$ (hergestellt wie beschrieben in der Europäischen Patentanmeldung 111'266) in 20 ml 5N Ammoniak-Lösung in Methanol bei Raumtemperatur in 24 Std. erhalten. Die Reaktionslösung wird eingeengt und die Titelverbindung durch Flash-Chromatographie mit System S6 gereinigt. $R_f$(S6) = 0.15.

### Beispiel 29: H-Leu $\overset{c}{-}$ Val-Ile-His-Lys(BOC)-OMe

a) H-His-Leu $\overset{c}{-}$ Val-Ile-His-Lys(BOC)-OMe: 465 mg Z-Leu $\overset{cx}{-}$ Val-Ile-His-Lys(BOC)-OMe werden in 10 ml Methanol/Wasser (95:5) gelöst und nach Zugabe von 50 mg Palladium-Kohle (10 % Pd) unter $CO_2$-Absorption bis zur Sättigung hydriert. Der Katalysator wird abfiltriert, das Filtrat zur Tockene eingeengt, pulverisiert und im Hochvakuum bei 40° nachgetrocknet. Man erhält die Titelverbindung in amorpher, chromatographisch einheitlicher Form. $R_f$(B17) = 0,25; $R_f$(B21) = 0,6.

b) Z-Leu $\overset{cx}{-}$ Val-Ile-His-Lys(BOC)-OMe: 300 mg Z-Leu $\overset{cx}{-}$ Val-OH, 454 mg H-Ile-His-Lys(BOC)-OMe (hergestellt wie beschrieben in der Europäischen Patentanmeldung 111'266) und 136 mg HOBt werden in 5 ml DMF gelöst, auf 0° gekühlt und mit 198 mg DCCI versetzt. Nach kurzem Rühren lässt man

über Nacht bei 0° und weitere 24 Std. bei Raumtemperatur stehen und filtriert dann den auskristallisierten DCH ab. Das Filtrat wird im Hochvakuum bei 40° eingeengt und der ölige Rückstand in 12 ml Methanol-Eisessig-$H_2O$ (94:3:3) gelöst und während 1 Std. auf 60° erwärmt. Die Lösung wird wiederum zur Trockne eingeengt und der Rückstand durch Craig-Verteilung im System Methanol-Acetatpuffer-Aethylenchlorid-Chloroform 10:3:8:4 (Acetatpuffer: 14,3 ml Eisessig und 9,6 g Ammoniumacetat in 1000 ml Wasser) über 800 Stufen gereinigt. Die dünnschichtchromatographisch reinen Fraktionen (K = 0,26) werden vereinigt, zur Trockne eingeengt, pulverisiert und im Hochvakuum bei 40° nachgetrocknet. $R_f$(B17) = 0,70; $R_f$(S4) = 0,75.

Beispiel 30: H-Leu $\overset{c}{-}$ Val-tris(tert-butyldimethylsilyloxymethyl)-methylamid

a) H-Leu $\overset{c}{-}$ Val-tris-(tert-butyldimethylsilyloxymethyl)-methylamid wird erhalten analog Beispiel 2c durch Hydrierung von 139 mg Z-Leu $\overset{cx}{-}$ Val-tris-(tert-butyldimethylsilyloxymethyl)-methylamid in Gegenwart von 50 mg Palladium-Kohle (5 %) und Mitteldruckchromatographie (LOBAR®-Fertigsäule, Grösse B, 40-60 µm, System Methylenchlorid/Methanol/Ammoniak konz. 175:10:1) als farbloses Oel. $R_f$(B8) = 0,41.

b) Z-Leu $\overset{cx}{-}$ Val-tris-(tert-butyldimethylsilyloxymethyl)-methylamid wird hergestellt durch Kondensation von 250 mg Z-Leu $\overset{cx}{-}$ Val-OH mit 286 mg Tris-(tert-butyldimethylsilyloxymethyl)-methylamin (Beispiel 4c) und 106 µl Aethyldiisopropylamin, 94 mg HOBt und 165 mg DCCI analog Beispiel 1. Nach Mitteldruckchromatographie (LOBAR®-Fertigsäule, Grösse B, 40-60 µm, System N5) wird die Titelverbindung als farbloses Oel erhalten.

Beispiel 31: H-Leu $\overset{c}{-}$ Gly(phenyl)-Ile-His-$NH_2$ (Diastereomer I)

a) H-Leu $\overset{c}{-}$ Gly(phenyl)-Ile-His-$NH_2$ (Diastereomer I) wird erhalten analog Beispiel 2c durch Hydrierung von 160 mg Z-Leu $\overset{cx}{-}$ Gly(phenyl)-Ile-His-$NH_2$ (Diastereomer I) in Gegenwart von 50 mg Palladium-Kohle (10%) nach Mitteldruckchromatographie (LOBAR®-Fertigsäule, Grösse B, 40-60 µm, System B10) als farbloses Pulver. $R_f$(B11) = 0,35.

b) Z-Leu $\overset{cx}{-}$ Gly(phenyl)-Ile-His-$NH_2$ (Diastereomer I) wird analog Beispiel 1f ausgehend von 205 mg Z-Leu $\overset{cx}{-}$ Gly(phenyl)-OH (Diastereomer I), 124 mg H-Ile-His-$NH_2$, 71 mg HOBt und 125 mg DCCI hergestellt und nach Mitteldruckchromatographie (LOBAR®-Fertigsäule, Grösse B, 40-60 µm, System B8) als farbloses Pulver erhalten. $R_f$(B9) = 0,36.

c) Z-Leu $\overset{cx}{-}$ Gly(phenyl)-OH (Diastereomer I und II) wird durch Hydrolyse von 1,56 g Z-Leu $\overset{cx}{-}$ Gly(phenyl)-$OCH_3$ (Diastereomerengemisch) mit 124 mg Wasser und 966 mg Kalium-tert-butylat in Aether analog Beispiel 1e erhalten und durch Mitteldruckchromatographie (240 g Lichroprep® Si 60, 25-40 µm, System N3) die Diastereomeren getrennt. Diastereomer I: $R_f$(N3) = 0,24. Diastereomer II: $R_f$(N3) = 0,15.

d) Z-Leu $\overset{cx}{-}$ Gly(phenyl)-$OCH_3$ (Diastereomerengemisch) wird ausgehend von 0,723 ml Diisopropylamin, 3,3 ml Butyllithium, 767 mg Phenylessigsäuremethylester und 2,00 g der Verbindung XXV analog Beispiel 1d hergestellt und nach Mitteldruckchromatographie (LOBAR®-Fertigsäule, Grösse B, 40-60 µm, Fliessmittelsystem Essigester Hexan 1:5) als farbloses Oel erhalten. $R_f$(N4) = 0,35 und 0,33.

Beispiel 32: H-Leu $\overset{c}{-}$ Gly(phenyl)-Ile-His-$NH_2$ (Diastereomer II)

a) H-Leu $\overset{c}{-}$ Gly(phenyl)-Ile-His-$NH_2$ (Diastereomer II) wird erhalten analog Beispiel 31a durch Hydrierung von 167 mg Z-Leu $\overset{cx}{-}$ Gly(phenyl)-Ile-His-$NH_2$ (Diastereomer II) in Gegenwart von 50 mg Palladium-Kohle (10 %). $R_f$(B11) = 0,24.

b) Z-Leu $\overset{cx}{-}$ Gly(phenyl)-Ile-His-$NH_2$ (Diastereomer II) wird erhalten analog Beispiel 31b ausgehend von 289 mg Z-Leu $\overset{cx}{-}$ Gly(phenyl)-OH (Diastereomer II, Beispiel 64c), 175 mg H-Ile-His-$NH_2$, 100 mg HOBt und 175 mg DCCI. $R_f$(B9) = 0,41.

Beispiel 33: H-Leu $\overset{c}{-}$ Gly(cyclohexyl)-Ile-His-$NH_2$ (Diastereomer I)

a) H-Leu $\overset{c}{-}$ Gly(cyclohexyl)-Ile-His-$NH_2$ (Diastereomer I) wird erhalten analog Beispiel 2c durch Hydrierung von 200 mg Z-Leu $\overset{cx}{-}$ Gly(cyclohexyl)-Ile-His-$NH_2$ in Gegenwart von 50 mg Palladium-Kohle (10 %) nach Mitteldruckchromatographie (31 g Lichroprep® Si 60, 25-40 µm, System B8) als farbloses Pulver. $R_f$(B11) = 0,39.

b) Z-Leu $\overset{cx}{-}$ Gly(cyclohexyl)-Ile-His-$NH_2$ (Diastereomer I) wird analog Beispiel 1f ausgehend von 250 mg Z-Leu $\overset{cx}{-}$ Gly(cyclohexyl)-OH (Diastereomer I), 150 mg H-Ile-His-$NH_2$, 86 mg HOBt und 150 mg

DCCI hergestellt und nach Mitteldruckchromatographie (LOBAR®-Fertigsäule, Grösse B, 40-60 µm, System B7) als farbloses Pulver erhalten. $R_f$(B9) = 0,35.

c) Z-Leu $\overset{cx}{-}$ Gly(cyclohexyl)-OH (Diastereomer I und II) wird durch Hydrolyse von 1.67 g Z-Leu $\overset{cx}{-}$ Gly(cyclohexyl)-$OCH_3$ (Diastereomerengemisch) mit 131 mg Wasser und 898 mg Kalium-tert-butylat in Aether analog Beispiel 1e erhalten und durch Mitteldruckchromatographie (244 g Lichroprep® Si 60, 25-40 µm, System Essigester Hexan 1:4) die Diastereomeren getrennt. Diastereomer I: $R_f$(N4) = 0.14. Diastereomer II: $R_f$(N4) = 0,11.

d) Z-Leu $\overset{cx}{-}$ Gly(cyclohexyl)-$OCH_3$ (Diastereomerengemisch) wird ausgehend von 0.723 ml Diisopropylamin, 3.3 ml Butyllithium, 796 mg Cyclohexylessigsäuremethylester und 2.00 g der Verbindung XXV analog Beispiel 1d hergestellt und nach Mitteldruckchromatographie (244 g Lichroprep® Si 60, 25-40 µm, System N5) als farbloses Oel erhalten. $R_f$(N4) = 0.35 und 0.33.

Beispiel 34: H-Leu $\overset{c}{-}$ Gly(cyclohexyl)-Ile-His-$NH_2$ (Diastereomer II)

a) H-Leu $\overset{c}{-}$ Gly(cyclohexyl)-Ile-His-$NH_2$ (Diastereomer II) wird erhalten analog Beispiel 33a durch Hydrierung von 180 mg Z-Leu $\overset{cx}{-}$ Gly(cyclohexyl)-Ile-His-$NH_2$ (Diastereomer II) in Gegenwart von 50 mg Palladium-Kohle (10 %). $R_f$(B11) = 0.38.

b) Z-Leu $\overset{cx}{-}$ Gly(cyclohexyl)-Ile-His-$NH_2$ (Diastereomer II) wird erhalten analog Beispiel 33b ausgehend von 250 mg Z-Leu $\overset{cx}{-}$ Gly(cyclohexyl)-OH (Diastereomer II, Beispiel 33c), 150 mg H-Ile-His-$NH_2$, 86 mg HOBt und 150 mg DCCI. $R_f$(B9) = 0,47.

Beispiel 35: H-Leu $\overset{c}{-}$ Gly(n-octyl)-Ile-His-$NH_2$

a) H-Leu $\overset{c}{-}$ Gly(n-octyl)-Ile-His-$NH_2$ wird erhalten durch Hydrierung von 269 mg Z-Leu $\overset{cx}{-}$ Gly(n-octyl)-Ile-His-$NH_2$ in Gegenwart von 40 mg Palladium-Kohle analog Beispiel 2c nach Mitteldruckchromatographie (LOBAR®-Fertigsäule, Grösse B, 40-60 µm, System B9) als farbloses Harz. $R_f$(B11) = 0.35.

b) Z-Leu $\overset{cx}{-}$ Gly(n-octyl)-Ile-His-$NH_2$ wird analog Beispiel 1f ausgehend von 413 mg Z-Leu $\overset{cx}{-}$ Gly(n-octyl)-OH, 232 mg H-Ile-His-$NH_2$, 133 mg HOBt und 233 mg DCCI hergestellt und nach Mitteldruckchromatographie (LOBAR®-Fertigsäule, Grösse B, 40-60 µm, System B7) als farbloses Pulver erhalten. $R_f$(B11) = 0,64 und 0.58 (Diastereomerengemisch).

c) Z-Leu $\overset{cx}{-}$ Gly(n-octyl)-OH wird durch Hydrolyse von 827 mg Z-Leu $\overset{cx}{-}$ Gly(n-octyl)-$OCH_3$ mit 85 mg Wasser und 569 mg Kalium-tert-butylat in Aether analog Beispiel 1c nach Mitteldruckchromatographie (LOBAR®-Fertigsäule, Grösse B, 40-60 µm, System N5) als farbloses Oel erhalten. $R_f$(N4, zweimal entwickelt) = 0.20 und 0.15 (Diastereomerengemisch).

d) Z-Leu $\overset{cx}{-}$ Gly(n-octyl)-$OCH_3$ wird ausgehend von 1,21 ml Diisopropylamin, 5.5 ml Butyllithium, 1595 mg Decansäuremethylester und 738 mg der Verbindung XXV analog Beispiel 1d hergestellt und nach Mitteldruckchromatographie (LOBAR®-Fertigsäule, Grösse B, 40-60 µm, System: Hexan 100 %) als farbloses Oel erhalten.

Beispiel 36:

Analog der vorstehenden Beispiele können hergestellt werden: H-Leu $\overset{c}{-}$ Val-Ile-tert-butylester, H-Leu $\overset{c}{-}$ Val-Ile-(N-äthoxycarbonyl-4-piperidyl)-amid, H-Leu $\overset{c}{-}$ Val-Ala-bis-(2-hydroxyäthylaminocarbamoyl)-methylamid, H-Leu $\overset{c}{-}$ Val-Ala-1-hydroxymethyl-2-(4-imidazolyl)-äthylamid, H-Leu $\overset{c}{-}$ Val-carbamoyl-(3-methoxycarbonyl-2-hydroxy-1-isobutyl-propylaminocarbonyl)-methylamid und H-Leu $\overset{c}{-}$ Val-Gly-Gly-tris-(hydroxymethyl)-methylamid.

## Ansprüche

1. 5-Amino-4-hydroxyvalerylderivate der Formel

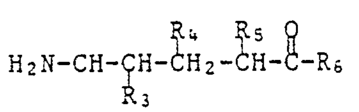

(I),

worin $R_3$ Wasserstoff, Alkyl, Cycloalkyl, Arylniederalkyl oder Aryl, $R_4$ Hydroxy oder veräthertes oder

verestertes Hydroxy, $R_5$ Alkyl, Cycloalkyl, Arylniederalkyl oder Aryl und $R_6$ substituiertes oder freies Amino oder substituiertes Hydroxy darstellen, und Salze dieser Verbindungen.

2. Verbindungen gemäss Anspruch 1 der Formel I, worin $R_3$ Wasserstoff, Niederalkyl, Cycloalkyl, Arylniederalkyl oder Aryl, $R_4$ Hydroxy, $R_5$ Alkyl, Cycloalkyl, Arylniederalkyl oder Aryl, und $R_6$ Amino oder eine Amino- oder Hydroxygruppe, welche durch einen oder gegebenenfalls zwei unsubstituierte oder substituierte, gesättigte oder ungesättigte, aliphatische Kohlenwasserstoffreste bis einschliesslich 18 C-Atomen oder einen aromatischen, heteroaromatischen, aromatisch-aliphatischen oder heteroaromatisch-aliphatischen Kohlenwasserstoffrest bis einschliesslich 18 C-Atomen substituiert ist, oder einen N-terminal mit der Gruppe -CO-verbundenen und C-terminal gegebenenfalls amidierten oder veresterten Rest einer natürlichen α-Aminosäure oder einen N-terminal mit der Gruppe -CO- verbundenen und C-terminal gegebenenfalls amidierten oder veresterten Di- oder Tripeptidrest, der aus natürlichen α-Aminosäuren und gewünschtenfalls aus Statin besteht, darstellen, ferner Salze dieser Verbindungen.

3. Verbindungen gemäss Anspruch 1 der Formel I, worin $R_3$ Wasserstoff, Niederalkyl, Cycloalkyl, Phenylniederalkyl oder Phenyl, $R_4$ Hydroxy, $R_5$ Alkyl mit 1-10 C-Atomen, Cycloalkyl, Phenylniederalkyl oder Phenyl
und $R_6$ Amino, Alkylamino mit 1 bis 10 C-Atomen, Diniederalkylamino, Hydroxyniederalkylamino, Di-(hydroxyniederalkyl)-amino, Niederalkoxyniederalkylamino, Niederalkanoyloxyniederalkylamino, Halogenniederalkyl amino, Hydroxysulfonyloxyniederalkylamino, Phenyloxyniederalkylamino oder Phenyloxy-hydroxyniederalkylamino, worin Phenyl gegebenenfalls durch Niederalkyl, Niederalkoxy, Hydroxy, Carboxy, Niederalkoxycarbonyl oder Carbamoyl substituiert ist, Carboxyalkylamino, Niederalkoxycarbonylalkylamino, physiologisch spaltbares verestertes Carboxyalkylamino, Carbamoylniederalkylamino, Niederalkylcarbamoylniederalkylamino, Hydroxyniederalkylcarbamoylniederalkylamino, Triniederalkylsilyloxyniederalkylcarbamoylniederalkylamino, Niederalkoxycarbonyl-hydroxy-niederalkylcarbamoylniederalkylamino, Diniederalkylcarbamoylniederalkylamino, Sulfoniederalkylamino, Aminoalkylamino, Niederalkylaminoniederalkylamino, Hydroxyniederalkylaminoniederalkylamino, Diniederalkylaminoniederalkylamino, 1-Piperidinylniederalkylamino, 4-Morpholinylniederalkylamino, 1-Pyrrolidinylniederalkylamino, worin Pyrrolidin gegebenenfalls durch Hydroxy und/oder Oxo substituiert ist, Niederalkanoylaminoalkylamino, Niederalkoxycarbonylaminoalkylamino, Niederalkenylamino, Niederalkinylamino, Phenylamino oder Phenylniederalkylamino, worin Phenyl gegebenenfalls durch Niederalkyl, Hydroxy, Niederalkoxy, Niederalkanoyloxy, Halogen, Carboxy, Niederalkoxycarbonyl, Carbamoyl, Amino, Niederalkylamino, Diniederalkylamino, Acylamino, und/oder durch Nitro mono-oder mehrfach substituiert ist, Phenylniederalkylamino, worin Niederalkyl durch Hydroxy, Cyano, Carboxy, Niederalkoxy und/oder Carbamoyl substituiert ist, Oxazolylamino, Thiazolylamino, das gegebenenfalls durch Carboxyniederalkyl, Niederalkoxycarbonylniederalkyl oder Carbamoylniederalkyl substituiert ist, Pyridylniederalkylamino, Imidazolylniederalkylamino, Indolylniederalkylamino, 1-Benzylpiperidinylamino, 1-Niederalkoxycarbonylpiperidinylamino, ferner Niederalkoxy, Phenyloxy, Phenylniederalkoxy, oder physiologisch spaltbares Alkoxy,
ferner -His-, -Phe-, -Ala-, -Ile-, C-terminal substituiert durch Amino, Alkylamino, Diniederalkylamino, Hydroxyniederalkylamino, Carboxyalkylamino, Niederalkoxycarbonylalkylamino, physiologisch spaltbares, verestertes Carboxyalkylamino, Carbamoylniederalkylamino, Hydroxyniederalkylcarbamoyl-niederalkylamino, Aminoalkylamino, Hydroxyniederalkylaminoniederalkylamino, Diniederalkylaminoniederalkylamino, über ein Stickstoff-Atom gebundenes fünf oder sechsgliedriges Heterocyclylniederalkylamino, Acylaminoniederalkylamino, Phenylniederalkylamino, worin Phenyl durch Carboxy, Niederalkoxycarbonyl oder Carbamoyl und Niederalkyl durch Hydroxy, Cyano oder Carboxy substituiert sein kann, unsubstituiertes oder substituiertes Thiazolylamino, Pyridylniederalkylamino, Imidazolylniederalkylamino, Indolylniederalkylamino, Piperidinylamino, 1-Niederalkoxycarbonylpiperidinylamino, ausserdem durch Niederalkoxy oder physiologisch spaltbares substituiertes Alkoxy,
ferner -Ile-His-, -Ile-Phe-, -Ala-His-, -Gly-Gly-, -Gly-His-, -Gly-Ala-, -Gly-Ser-, -Ala-Sta-, -Ile-Sta-, -Gly-Gly-Gly-, -Gly-His-Lys-, -Gly-Ala-Gly- oder -Ile-His-Lys-, worin die Aminofunktion in der Seitenkette von -Lys-gegebenenfalls durch Niederalkanoyl, Niederalkoxycarbonyl oder Phenylniederalkoxycarbonyl acyliert ist, C-terminal substituiert durch Amino, Niederalkylamino, Diniederalkylamino, Hydroxyniederalkylamino, ferner Niederalkoxy oder physiologisch spaltbares substituiertes Alkoxy darstellt, sowie Salze dieser Verbindungen.

4. Verbindungen gemäss Anspruch 1 der Formel I, worin $R_3$ Niederalkyl, $R_4$ Hydroxy, $R_5$ Alkyl, Cyclohexyl oder Phenyl und $R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkylamino, Niederalkoxycarbonylalkylamino, physiologisch spaltbares verestertes Carboxyalkylamino, Carbamoylniederalkylamino, Hydroxyniederalkylcarbamoylniederalkylamino, Niederalkoxycarbonyl-hydroxy-niederalkoxycarbamoyl-niederalkylamino, Aminoalkylamino, Hydroxyniederalkylaminoniederalkylamino, Diniederalkylaminoniederalkylamino, über ein Stickstoffatom gebundenes fünf- oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, sowie Niederalkoxy, oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, Diniederalkylaminoniederalkylamino, 4-Morpholinylniederalkylamino, Benzylamino, Carboxybenzylamino, Niederalkoxycarbonylbenzylamino, Carbamoylbenzylamino, 2-Phenyläthylamino, 1-Phenylprop-2-yl-amino, 3-Phenylpropylamino, 1-Hydroxymethyl-2-phenyläthylamino, 2-Hydroxy-1- oder 2-phenyläthylamino oder 1-(2-Cyano- oder 2-Carboxy-1- hydroxyäthyl)-2-phenyläthylamino, 4- oder 5-Carbamoylmethyl-2-thiazolylamino, Pyridylniederalkylamino, Imidazolylniederalkylamino, Indolylniederalkylamino, 1-Niederalkoxycarbonylpiperidinylamino, Niederalkoxy oder physiologisch spaltbares substituiertes Alkoxy, ferner -Ile-His-, -Ile-Phe, -Ile-Sta-, -Ala-His-, -Ala-Sta- oder -Ile-His-Lys-, worin die Aminofunktion in der Seitenkette von -Lys- gegebenenfalls durch Niederalkanoyl, Niederalkoxcarbonyl oder Phenylniederalkoxycarbonyl acyliert ist, C-terminal substituiert durch Amino, Niederalkylamino, Diniederalkylamino, Hydroxyniederalkylamino, Niederalkoxy oder physiologisch spaltbares substituiertes Alkoxy, darstellt, sowie Salze dieser Verbindungen.

5. Verbindungen gemäss Anspruch 1 der Formel I, worin $R_3$ Isopropyl oder Isobutyl, $R_4$ Hydroxy, $R_5$ Isopropyl, n-Octyl, Cyclohexyl oder Phenyl und $R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkylamino, Niederalkoxycarbonylalkylamino, physiologisch spaltbares verestertes Carboxyalkylamino, Carbamoylniederalkylamino, Hydroxyniederalkylcarbamoylniederalkylamino, α-Carbamoyl-α-(2-hydroxy-1-isobutyl-3-methoxycarbonylpropyl)-carbamoyl-methylamino, Aminoalkylamino, Hydroxyniederalkylaminoniederalkylamino, Diniederalkylaminoniederalkylamino, über ein Stickstoff-Atom gebundenes fünf- oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, Niederalkoxy oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, 4-Morpholinylniederalkylamino, Carboxybenzylamino, Carbamoylbenzylamino, 4- oder 5-Carbamoylmethyl-2-thiazolylamino, Pyridylniederalkylamino, Imidazolylniederalkylamino, Indolylniederalkylamino, Niederalkoxy oder physiologisch spaltbares substituiertes Alkoxy, ferner-Ile-His-, -Ile-Sta- oder -Ala-Sta-, C-terminal substituiert durch Amino, Nieder alkylamino, Diniederalkylamino, Hydroxyniederalkylamino, Niederalkoxy oder physiologisch spaltbares substituiertes Alkoxy darstellt, sowie Salze dieser Verbindungen.

6. Verbindungen gemäss Anspruch 1 der Formel I, worin $R_3$ Isobutyl, $R_4$ Hydroxy, $R_5$ Isopropyl, n-Octyl, Cyclohexyl oder Phenyl und $R_6$ Amino, Niederalkylamino, Diniederalkylamino, Hydroxyniederalkylamino, Carboxyalkylamino, Niederalkoxycarbonylalkylamino, physiologisch spaltbares verestertes Carboxyalkylamino, Aminoalkylamino, Hydroxyniederalkylaminoniederalkylamino, Diniederalkylaminoniederalkylamino, 4-Morpholinylniederalkylamino, 2-Oxo-1-pyrrolidinylniederalkylamino, ferner -Ala-oder -Ile-, C-terminal substituiert durch Amino, Niederalkylamino, Diniederalkylamino, Hydroxyniederalkylamino, Dicarbamoylmethylamino, 2-(2-Hydroxyäthylamino)-äthylamino, 2-(4-Morpholinyl)-äthylamino, 3-Carbamoylbenzylamino, 4-Carbamoylmethyl-2-thiazolylamino, 2-Pyridylmethylamino, 2-(2-Pyridyl)-äthylamino, 3-(2-Pyridyl)-propylamino, 2-(4-Imidazolyl)-äthylamino oder 2-(3-Indolyl)-äthylamino, ferner -Ile-His- oder -Ile-Sta-, C-terminal substituiert durch Amino, oder -Ala-Sta-, C-terminal substituiert durch Niederalkoxy, darstellt, sowie Salze dieser Verbindungen.

7. Verbindungen gemäss Anspruch 1 der Formel I, worin $R_3$ Isobutyl, $R_4$ Hydroxy, $R_5$ Isopropyl und $R_6$ Amino, Niederalkylamino, Dimethylamino, 4-Carboxy-n-butylamino, 7-Carboxy-n-heptylamino, 4-tert-Butoxycarbonyl-n-butylamino, 7-tert-Butoxycarbonyl-n-heptylamino, 7-Pivaloyloxymethoxycarbonyl-n-heptylamino, 3-(2-Oxo-1-pyrrolidinyl)-propylamino, -Ala-, C-terminal substituiert durch 2-(4-Imidazolyl)-äthylamino oder 2-(2-Pyridyl)-äthylamino, -Ile-, C-terminal substituiert durch Dicarbamoylmethylamino, 3-Carbamoylbenzylamino, 4-Carbamoylmethyl-2-thiazolylamino, 2-(2-Pyridyl)-äthylamino, 2-Pyridylmethylamino oder 2-(3-Indolyl)-äthylamino, -Ile-His- oder -Ile-Sta-, C-terminal substituiert durch Amino, oder -Ala-Sta-, C-terminal substituiert durch Methoxy, darstellt, sowie Salze dieser Verbindungen.

8. [5(S)-Amino-4(S)-hydroxy-2(S)-isopropyl-7-methyloctanoyl]-Ile-His-$NH_2$ gemäss Anspruch 1.

9. [5(S)-Amino-4(S)-hydroxy-2(S)-isopropyl-7-methyloctanoyl]-Ala-Sta-$OCH_3$ gemäss Anspruch 1

10. [5(S)-Amino-4(S)-hydroxy-(2S)-isopropyl-7-methyloctanoyl]-Ile-dicarbamoylmethylamid gemäss Anspruch 1.

11. [5(S)-Amino-4(S)-hydroxy-2(S)-isopropyl-7-methyloctanoyl]-2-(3-carbamoyl-4-hydroxy-phenyloxy)-äthylamid gemäss Anspruch 1.

12. [5(S)-Amino-4(S)-hydroxy-2(S)-isopropyl-7-methyloctanoyl]-3-(2-pyrrolidinon-1-yl)-propylamid gemäss Anspruch 1.

13. [5(S)-Amino-4(S)-hydroxy-2(S)-isopropyl-7-methyloctanoyl]-methylamid gemäss Anspruch 1.

14. [5(S)-Amino-4(S)-hydroxy-2(S)-isopropyl-7-methyloctanoyl]-4-[tris-(hydroxymethyl)-methylaminocarbonyl]-butylamid gemäss Anspruch 1.

15. [5(S)-Amino-4(S)-hydroxy-2(S)-isopropyl-7-methyloctanoyl]-carbamoyl-(3-methoxycarbonyl-2-hydroxy-1-isobutyl-propylaminocarbonyl)-methylamid gemäss Anspruch 1.

16. Verwendung von Verbindungen der Formel I gemäss Anspruch 1 oder deren Salze zur Herstellung von Renininhibitoren.

17. Verfahren zur Herstellung von Verbindungen der Formel I, worin die Substituenten die in Anspruch 1 genannten Bedeutungen haben, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, eine Verbindung der Formel

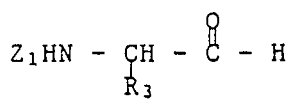 (IX),

worin $R_3$ die genannte Bedeutung hat und $Z_1$ eine Aminoschutzgruppe ist, mit einer Schwefel-Ylid-Verbindung in ein Epoxid der Formel

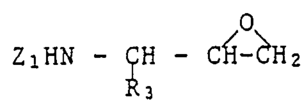 (X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

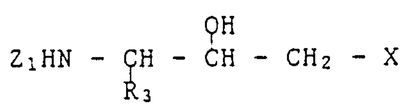 (XI),

worin $R_3$ und $Z_1$ die genannten Bedeutungen haben und X eine nukleofuge Abgangsgruppe ist, mit einer Carbonylverbindung der Formel

$R_a$ - (C = O) - $R_b$ (XII),

worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

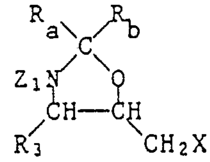 (XIII),

worin die Substituenten die genannten Bedeutungen haben, mit einem Carbonsäureestersalz der Formel

$[R_5 - \overset{\ominus}{C}H - \overset{O}{\overset{\|}{C}} - OZ_2]\,Y^{\oplus}$ (XIV),

worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium-oder bevorzugt das Lithiumion, ist, umsetzt, und in einer erhältlichen Verbindung der Formel

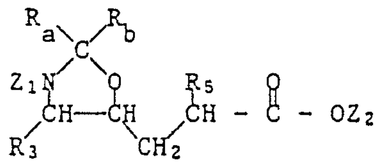

(XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2$ = H) je nach Bedeutung des einzuführenden Rests $R_6$ amidiert, verestert oder mit einem C-terminal gegebenenfalls amidierten oder veresterten Rest einer Aminosäure, eines Di-oder Tripeptidrest substituiert und in einer erhältlichen Verbindung der Formel

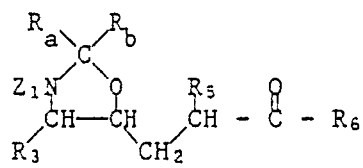

(XVI),

worin die Substituenten die genannten Bedeutungen haben, mit einem geeigneten Solvolysereagens den Ring öffnet und gegebenenfalls die Schutzgruppe $Z_1$ abspaltet,
und gewünschtenfalls in einer erhältlichen Verbindung vorhandene Schutzgruppen abspaltet und/oder gewünschtenfalls nach Ausführung eines der vorstehend genannten Verfahren a) oder b) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

Patentansprüche für folgenden Vertragsstaat: AT

1. Verfahren zur Herstellung von 5-Amino-4-hydroxyvalerylderivaten der Formel

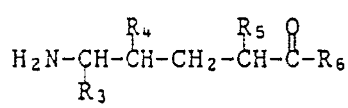

(I),

worin $R_3$ Wasserstoff, Alkyl, Cycloalkyl, Arylniederalkyl oder Aryl, $R_4$ Hydroxy oder veräthertes oder verestertes Hydroxy, $R_5$ Alkyl, Cycloalkyl, Arylniederalkyl oder Aryl und $R_6$ substituiertes oder freies Amino oder substituiertes Hydroxy darstellen, und von Salzen dieser Verbindungen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, eine Verbindung der Formel

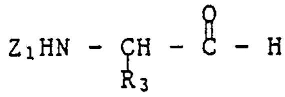
(IX),

worin $R_3$ die genannte Bedeutung hat und $Z_1$ eine Aminoschutzgruppe ist, mit einer Schwefel-Ylid-Verbindung in ein Epoxid der Formel

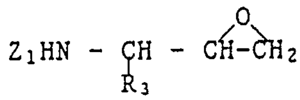
(X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

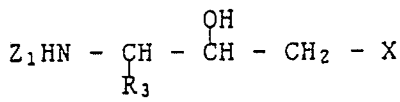
(XI),

worin $R_3$ und $Z_1$ die genannten Bedeutungen haben und X eine nukleofuge Abgangsgruppe ist, mit einer Carbonylverbindung der Formel
$R_a$ - (C = O) - $R_b$ (XII),
worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

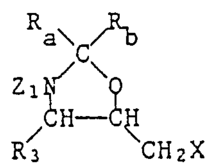
(XIII),

worin die Substituenten die genannten Bedeutungen haben, mit einem Carbonsäureestersalz der Formel

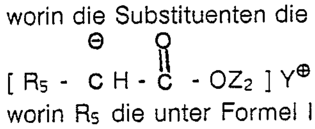
(XIV),
worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium-oder bevorzugt das Lithiumion, ist, umsetzt, und in einer erhältlichen Verbindung der Formel

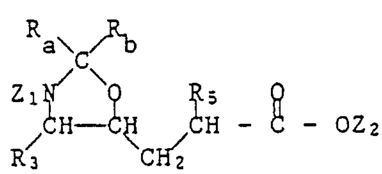
(XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2 = H$) je nach Bedeutung des einzuführenden Rests $R_6$ amidiert, verestert oder mit einem C-terminal gegebenenfalls amidierten oder veresterten Rest einer Aminosäure, eines Di- oder Tripeptidrest substituiert und in einer erhältlichen Verbindung der Formel

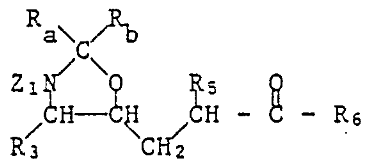

(XVI),

worin die Substituenten die genannten Bedeutungen haben, mit einem geeigneten Solvolysereagens den Ring öffnet und gegebenenfalls die Schutzgruppe Z· abspaltet, und gewünschtenfalls in einer erhältlichen Verbindung vorhandene Schutzgruppen abspaltet und/oder gewünschtenfalls nach Ausführung eines der vorstehend genannten Verfahren a) oder b) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

2. Verfahren gemäss Anspruch 1, zur Herstellung von Verbindungen der Formel I, worin $R_3$ Wasserstoff, Niederalkyl, Cycloalkyl, Arylniederalkyl oder Aryl, $R_4$ Hydroxy, $R_5$ Alkyl, Cycloalkyl, Arylniederalkyl oder Aryl und $R_6$ Amino oder eine Amino- oder Hydroxygruppe, welche durch einen oder gegebenenfalls zwei unsubstituierte oder substituierte, gesättigte oder ungesättigte, aliphatische Kohlenwasserstoffreste bis einschliesslich 18 C-Atomen oder einen aromatischen, heteroaromatischen, aromatisch-aliphatischen oder heteroaromatisch-aliphatischen Kohlenwasserstoffrest bis einschliesslich 18 C-Atomen substituiert ist, oder einen N-terminal mit der Gruppe -CO-verbundenen und C-terminal gegebenenfalls amidierten oder veresterten Rest einer natürlichen α-Aminosäure oder einen N-terminal mit der Gruppe -CO- verbundenen und C-terminal gegebenenfalls amidierten oder veresterten Di- oder Tripeptidrest, der aus natürlichen α-Aminosäuren und gewünschtenfalls aus Statin besteht, darstellen, und von Salzen dieser Verbindungen.

3. Verfahren gemäss Anspruch 1, zur Herstellung von Verbindungen der Formel I, worin $R_3$ Wasserstoff, Niederalkyl, Cycloalkyl, Phenylniederalkyl oder Phenyl, $R_4$ Hydroxy, $R_5$ Alkyl mit 1-10 C-Atomen, Cycloalkyl, Phenylniederalkyl oder Phenyl

und $R_6$ Amino, Alkylamino mit 1 bis 10 C-Atomen, Diniederalkylamino, Hydroxyniederalkylamino, Di-(hydroxyniederalkyl)-amino, Niederalkoxyniederalkylamino, Niederalkanoyloxyniederalkylamino, Halogenniederalkylamino, Hydroxysulfonyloxyniederalkylamino, Phenyloxyniederalkylamino oder Phenyloxy-hydroxyniederalkylamino, worin Phenyl gegebenenfalls durch Niederalkyl, Niederalkoxy, Hydroxy, Carboxy, Niederalkoxycarbonyl oder Carbamoyl substituiert ist, Carboxyalkylamino, Niederalkoxycarbonylalkylamino, physiologisch spaltbares verestertes Carboxyalkylamino, Carbamoylniederalkylamino, Niederalkylcarbamoylniederalkylamino, Hydroxyniederalkylcarbamoylniederalkylamino, Triniederalkylsilyloxyniederalkylcarbamoylniederalkylamino, Niederalkoxycarbonyl-hydroxy-niederalkylcarbamoylniederalkylamino, Diniederalkylcarbamoylniederalkylamino, Sulfoniederalkylamino, Aminoalkylamino, Niederalkylaminoniederalkylamino, Hydroxyniederalkylaminoniederalkylamino, Diniederalkylaminoniederalkylamino, 1-Piperidinylniederalkylamino, 4-Morpholinylniederalkylamino, 1-Pyrrolidinylniederalkylamino, worin Pyrrolidin gegebenenfalls durch Hydroxy und/oder Oxo substituiert ist, Niederalkanoylaminoalkylamino, Niederalkoxycarbonylaminoalkylamino, Niederalkenylamino, Niederalkinylamino, Phenylamino oder Phenylniederalkylamino, worin Phenyl gegebenenfalls durch Niederalkyl, Hydroxy, Niederalkoxy, Niederalkanoyloxy, Halogen, Carboxy, Niederalkoxycarbonyl, Carbamoyl, Amino, Niederalkylamino, Diniederalkylamino, Acylamino, und/oder durch Nitro mono- oder mehrfach substituiert ist, Phenylniederalkylamino, worin Niederalkyl durch Hydroxy, Cyano, Carboxy, Niederalkoxy und/oder Carbamoyl substituiert ist, Oxazolylamino, Thiazolylamino, das gegebenenfalls durch Carboxyniederalkyl, Niederalkoxycarbonylniederalkyl oder Carbamoylniederalkyl substituiert ist, Pyridylniederalkylamino, Imidazolylniederalkylamino, Indolylniederalkylamino, 1-Benzylpiperidinylamino, 1-Niederalkoxycarbonylpiperidinylamino, ferner Niederalkoxy, Phenyloxy, Phenylniederalkoxy, oder physiologisch spaltbares Alkoxy,

ferner -His-, -Phe-, -Ala-, -Ile-, C-terminal substituiert durch Amino, Alkylamino, Diniederalkylamino, Hydroxyniederalkylamino, Carboxyalkylamino, Niederalkoxycarbonylalkylamino, physiologisch spaltbares, verestertes Carboxyalkylamino, Carbamoylniederalkylamino, Hydroxyniederalkylcarbamoyl-niederalkylamino, Aminoalkylamino, Hydroxyniederalkylaminoniederalkylamino, Diniederalkylaminoniederalkylamino, über ein Stickstoff-Atom gebundenes fünf oder sechsgliedriges Heterocyclylniederalkylamino, Acylaminoniederalkylamino, Phenylniederalkylamino, worin Phenyl durch Carboxy, Niederalkoxycarbonyl oder Carbamoyl und Niederalkyl durch Hydroxy, Cyano oder Carboxy substituiert sein kann, unsubstituiertes oder substituiertes

Thiazolylamino, Pyridylniederalkylamino, Imidazolylniederalkylamino, Indolylniederalkylamino, Piperidinylamino, 1-Niederalkoxycarbonylpiperidinylamino, ausserdem durch Niederalkoxy oder physiologisch spaltbares substituiertes Alkoxy, ferner -Ile-His-, -Ile-Phe-, -Ala-His-, -Gly-Gly-, -Gly-His-, -Gly-Ala-, -Gly-Ser-, -Ala-Sta-, -Ile-Sta-, -Gly-Gly-Gly-, -Gly-His-Lys-, -Gly-Ala-Gly- oder -Ile-His-Lys-, worin die Aminofunktion in der Seitenkette von -Lys- gegebenenfalls durch Niederalkanoyl, Niederalkoxycarbonyl oder Phenylniederalkoxycarbonyl acyliert ist, C-terminal substituiert durch Amino, Niederalkylamino, Diniederalkylamino, Hydroxyniederalkylamino, ferner Niederalkoxy oder physiologisch spaltbares substituiertes Alkoxy darstellt, sowie von Salzen dieser Verbindungen.

4. Verfahren gemäss Anspruch 1 zur Herstellung von Verbindungen der Formel I, worin $R_3$ Niederalkyl, $R_4$ Hydroxy, $R_5$ Alkyl, Cyclohexyl oder Phenyl und $R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkyl amino, Niederalkoxycarbonylalkylamino, physiologisch spaltbares verestertes Carboxyalkylamino, Carbamoylniederalkylamino, Hydroxyniederalkylcarbamoylniederalkylamino, Niederalkoxycarbonyl-hydroxy-niederalkoxycarbamoyl-niederalkylamino, Aminoalkylamino, Hydroxyniederalkylaminoniederalkylamino, Diniederalkylaminoniederalkylamino, über ein Stickstoffatom gebundenes fünf-oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, sowie Niederalkoxy, oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, Diniederalkylaminoniederalkylamino, 4-Morpholinylniederalkylamino, Benzylamino, Carboxybenzylamino, Niederalkoxycarbonylbenzylamino, Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1- oder 2-phenyläthylamino oder 1-(2-Cyano-oder 2-Carboxy-1-hydroxyäthyl)-2-phenyläthylamino, 4- oder 5-Carbamoylmethyl-2-thiazolylamino, Pyridylniederalkylamino, Imidazolylniederalkylamino, Indolylniederalkylamino, 1-Niederalkoxycarbonylpiperidinylamino, Niederalkoxy oder physiologisch spaltbares substituiertes Alkoxy, ferner -Ile-His-, -Ile-Phe, -Ile-Sta-, -Ala-His-, -Ala-Sta- oder -Ile-His-Lys-, worin die Aminofunktion in der Seitenkette von -Lys- gegebenenfalls durch Niederalkanoyl, Niederalkoxcarbonyl oder Phenylniederalkoxycarbonyl acyliert ist, C-terminal substituiert durch Amino, Niederalkylamino, Diniederalkylamino, Hydroxyniederalkylamino, Niederalkoxy oder physiologisch spaltbares substituiertes Alkoxy, darstellt, sowie von Salzen dieser Verbindungen.

5. Verfahren gemäss Anspruch 1 zur Herstellung von Verbindungen der Formel I, worin $R_3$ Isopropyl oder Isobutyl, $R_4$ Hydroxy, $R_5$ Isopropyl, n-Octyl, Cyclohexyl oder Phenyl und $R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkylamino, Niederalkoxycarbonylalkylamino, physiologisch spaltbares verestertes Carboxyalkylamino, Carbamoylniederalkyl amino, Hydroxyniederalkylcarbamoylniederalkylamino, α-Carbamoyl-α-(2-hydroxy-1-isobutyl-3-methoxycarbonylpropyl)-carbamoyl-methylamino, Aminoalkylamino, Hydroxyniederalkylaminoniederalkylamino, Diniederalkylaminoniederalkylamino, über ein Stickstoff-Atom gebundenes fünf- oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, Niederalkoxy oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, 4-Morpholinylniederalkylamino, Carboxybenzylamino, Carbamoylbenzylamino, 4- oder 5-Carbamoylmethyl-2-thiazolylamino, Pyridylniederalkylamino, Imidazolylniederalkylamino, Indolylniederalkylamino, Niederalkoxy oder physiologisch spaltbares substituiertes Alkoxy, ferner -Ile-His-, -Ile-Sta- oder -Ala-Sta-, C-terminal substituiert durch Amino, Niederalkylamino, Diniederalkylamino, Hydroxyniederalkylamino, Niederalkoxy oder physiologisch spaltbares substituiertes Alkoxy, darstellt, sowie von Salzen dieser Verbindungen.

6. Verfahren gemäss Anspruch 1 zur Herstellung von Verbindungen der Formel I, worin $R_3$ Isobutyl, $R_4$ Hydroxy, $R_5$ Isopropyl, n-Octyl, Cyclohexyl oder Phenyl und $R_6$ Amino, Niederalkylamino, Diniederalkylamino, Hydroxyniederalkylamino, Carboxyalkylamino, Niederalkoxycarbonylalkylamino, physiologisch spaltbares verestertes Carboxyalkylamino, Aminoalkylamino, Hydroxyniederalkylaminoniederalkylamino, Diniederalkylaminoniederalkylamino, 4-Morpholinylniederalkylamino, 2-Oxo-1-pyrrolidinylniederalkylamino, ferner -Ala- oder -Ile-, C-terminal substituiert durch Amino, Niederalkylamino, Diniederalkylamino, Hydroxyniederalkylamino, Dicarbamoylmethylamino, 2-(2-Hydroxyäthylamino)-äthylamino, 2-(4-Morpholinyl)-äthylamino, 3-Carbamoylbenzylamino, 4-Carbamoylmethyl-2-thiazolylamino, 2-Pyridylmethylamino, 2-(2-Pyridyl)-äthylamino,

3-(2-Pyridyl)-propylamino, 2-(4-Imidazolyl)-äthylamino oder 2-(3-Indolyl)-äthylamino, ferner -Ile-His- oder -Ile-Sta-, C-terminal substituiert durch Amino, oder -Ala-Sta-, C-terminal substituiert durch Niederalkoxy, darstellt, sowie von Salzen dieser Verbindungen.

7. Verfahren gemäss Anspruch 1 zur Herstellung von Verbindungen der Formel I, worin $R_3$ Isobutyl, $R_4$ Hydroxy, $R_5$ Isopropyl und $R_6$ Amino, Niederalkylamino, Dimethylamino, 4-Carboxy-n-butylamino, 7-Carboxy-n-heptylamino, 4-tert-Butoxycarbonyl-n-butylamino, 7-tert-Butoxycarbonyl-n-heptylamino, 7-Pivaloyloxymethoxycarbonyl-n-heptylamino, 3-(2-Oxo-1-pyrrolidinyl)-propylamino, -Ala-, C-terminal substituiert durch 2-(4-Imidazolyl)-äthylamino oder 2-(2-Pyridyl)-äthylamino, -Ile-, C-terminal substituiert durch Dicarbamoylmethylamino, 3-Carbamoylbenzylamino, 4-Carbamoylmethyl-2-thiazolylamino, 2-(2-Pyridyl)-äthylamino, 2-Pyridylmethylamino oder 2-(3-Indolyl)-äthylamino, -Ile-His- oder -Ile-Sta-, C-terminal substituiert durch Amino, oder -Ala-Sta-, C-terminal substituiert durch Methoxy, darstellt, sowie von Salzen dieser Verbindungen.

8. Verfahren gemäss Anspruch 1 zur Herstellung von [5(S)-Amino-4(S)-hydroxy-2(S)-isopropyl-7-methyloctanoyl]-Ile-His-$NH_2$.

9. Verfahren gemäss Anspruch 1 zur Herstellung von [5(S)-Amino-4(S)-hydroxy-2(S)-isopropyl-7-methyloctanoyl]-Ala-Sta-$OCH_3$.

10. Verfahren gemäss Anspruch 1 zur Herstellung von [5(S)-Amino-4(S)-hydroxy-(2S)-isopropyl-7-methyloctanoyl]-Ile-dicarbamoylmethylamid.

11. Verfahren gemäss Anspruch 1 zur Herstellung von [5(S)-Amino-4(S)-hydroxy-2(S)-isopropyl-7-methyloctanoyl]-2-(3-carbamoyl-4-hydroxy-phenyloxy)-äthylamid.

12. Verfahren gemäss Anspruch 1 zur Herstellung von [5(S)-Amino-4(S)-hydroxy-2(S)-isopropyl-7-methyloctanoyl]-3-(2-pyrrolidinon-1-yl)-propylamid.

13. Verfahren gemäss Anspruch 1 zur Herstellung von [5(S)-Amino-4(S)-hydroxy-2(S)-isopropyl-7-methyloctanoyl]-methylamid.

14. Verfahren gemäss Anspruch 1 zur Herstellung von [5(S)-Amino-4(S)-hydroxy-2(S)-isopropyl-7-methyloctanoyl]-4-[tris-(hydroxymethyl)-methylaminocarbonyl]-butylamid.

15. Verfahren gemäss Anspruch 1 zur Herstellung von [5(S)-Amino-4(S)-hydroxy-2(S)-isopropyl-7-methyloctanoyl]-carbamoyl-(3-methoxycarbonyl-2-hydroxy-1-isobuyl-propylaminocarbonyl)-methylamid.

Europäisches Patentamt

# EUROPÄISCHER RECHERCHENBERICHT

Nummer der Anmeldung

EP 90 10 5494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br><br>A | EP-A-0 045 665 (SZELKE et al.)<br>* Das ganze Dokument, insbesondere Seite 10, letzte Formel *<br>--- | 1<br><br>2-17 | C 07 K 5/02<br>C 07 K 5/06<br>C 07 C 229/22<br>C 07 C 237/06 |
| X | CHEMICAL ABSTRACTS, Band 101, nr. 11, 10. September 1984, Seite 262, Zusammenfassung Nr. 86219s, Columbus, Ohio, US; M. SZELKE et al.: "Novel transition-state analog inhibitors of renin", & PEPT.: STRUCT. FUNCT., PROC. AM. PEPT. SYMP., 8TH 1983, 579-82<br>* Zusammenfassung *<br>--- | 1 | |
| X | JOURNAL OF MEDICINAL CHEMISTRY, Band 26, Nr. 10, Oktober 1983, Seiten 1457-1462, American Chemical Society; R.L. JOHNSON et al.: "Inhibition of renin by angiotensinogen peptide fragments containing the hydroxy amino acid residue 5-amino-3-hydroxy-7-methyloctanoic acid"<br>* Das ganze Dokument *<br>--- | 1 | RECHERCHIERTE SACHGEBIETE (Int. Cl.5)<br>C 07 K<br>C 07 C |
| X | TETRAHEDRON LETTERS, Band 24, Nr. 41, 1983, Seiten 4401-4404, Pergamon Press Ltd, GB; M.W. HOLLLADAY et al.: "Synthesis of hydroxyethylene and kemothylene dipeptide isosteres"<br>* Das ganzes Dokument *<br>--- | 1 | |
| P,X | WO-A-8 403 044 (FERRING)<br>* Das ganze Dokument *<br>----- | 1 | |

Der vorliegende Recherchenbericht wurde für alle Patentansprüche erstellt

| Recherchenort | Abschlußdatum der Recherche | Prüfer |
|---|---|---|
| DEN HAAG | 18-07-1990 | MASTURZO P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument